# EUROPEAN PATENT APPLICATION

(11) **EP 2 295 553 A1**
(43) Date of publication of application: **16.03.2011**
(21) Application number: 10182235.1
(22) Date of filing: 04.02.2003
(51) Int. Cl.: C12N 9/16, C12N 15/82, A23K 1/165

(54) **Phytase variants**

(30) Priority: 08.02.2002 DK 200200193; 30.09.2002 DK 200201449
(62) Divisional of application: 03701481.8
(71) Applicant: Novozymes A/S, 2880 Bagsvaerd (DK)
(72) Inventor: Matsui, Tomoko, Ichikawa Chiba 272-0026 (JP); Fuglsang, Claus Crone, 3670, Veksoe (DK); Svendsen, Allan, 2970, Hoersholm (DK); Fukuyama, Shiro, Narashino-shi Chiba 275-0017 (JP)

(57) **Abstract**

The present invention relates to phytase variants derived from a parent phytase which is homologous to a *Peniophora lycii* phytase and comprises at least one substitution in at least one of a number of positions corresponding to a position in the *Peniophora lycii* phytase. The variants are of improved properties, in particular of a higher specific activity and/or more thermostable than the parent phytase, e.g. of a Tm of up to 82°C at pH 5.5, as determined by DSC, and/or of a doubled specific activity. The invention also relates to DNA encoding the phytase variants, methods of their production, as well as the use thereof, e.g. in animal feed and animal feed additives.

SEQUENCE LISTING

<110>  Novozymes A/S

<120>  Phytase Variants

<130>  10261

<160>  14

<170>  PatentIn version 3.2

<210>  1
<211>  1320
<212>  DNA
<213>  Peniophora lycii


<220>
<221>  sig_peptide
<222>  (1)..(48)

<220>
<221>  CDS
<222>  (49)..(1320)

<400>  1
atggtttctt cggcattcgc accttccatc ctacttagct tgatgtcg agt ctt gct      57
                                                     Ser Leu Ala
                                                     1

ttg agc acg cag ttc agc ttt gtt gcg gcg cag cta cct atc ccc gca      105
Leu Ser Thr Gln Phe Ser Phe Val Ala Ala Gln Leu Pro Ile Pro Ala
    5                   10                  15

caa aac aca agt aat tgg ggg cct tac gat ccc ttc ttt ccc gtc gaa      153
Gln Asn Thr Ser Asn Trp Gly Pro Tyr Asp Pro Phe Phe Pro Val Glu
20                  25                  30                  35

ccg tat gca gct ccg ccg gaa ggg tgc aca gtg aca cag gtc aac ctg      201
Pro Tyr Ala Ala Pro Pro Glu Gly Cys Thr Val Thr Gln Val Asn Leu
                40                  45                  50

att cag agg cac ggc gcg cgt tgg ccc aca tcc ggc gcg cgg tcg cgg      249
Ile Gln Arg His Gly Ala Arg Trp Pro Thr Ser Gly Ala Arg Ser Arg
            55                  60                  65

cag gtc gcc gcc gta gcg aag ata caa atg gcg cga cca ttc acg gat      297
Gln Val Ala Ala Val Ala Lys Ile Gln Met Ala Arg Pro Phe Thr Asp
        70                  75                  80

ccc aag tat gag ttc ctc aac gac ttc gtg tac aag ttc ggc gtc gcc      345
Pro Lys Tyr Glu Phe Leu Asn Asp Phe Val Tyr Lys Phe Gly Val Ala
    85                  90                  95

gat ctg cta ccg ttc ggg gct aac caa tcg cac caa acc ggc acc gat      393
Asp Leu Leu Pro Phe Gly Ala Asn Gln Ser His Gln Thr Gly Thr Asp
100                 105                 110                 115

atg tat acg cgc tac agt aca cta ttt gag ggc ggg gat gta ccc ttt      441
Met Tyr Thr Arg Tyr Ser Thr Leu Phe Glu Gly Gly Asp Val Pro Phe
                120                 125                 130

gtg cgc gcg gct ggt gac caa cgc gtc gtt gac tcc tcg acg aac tgg      489
Val Arg Ala Ala Gly Asp Gln Arg Val Val Asp Ser Ser Thr Asn Trp
            135                 140                 145

acg gca ggc ttt ggc gat gct tct ggc gag act gtt ctc ccg acg ctc      537
Thr Ala Gly Phe Gly Asp Ala Ser Gly Glu Thr Val Leu Pro Thr Leu

150                 155                 160

cag gtt gtg ctt caa gaa gag ggg aac tgc acg ctc tgc aat aat atg       585
Gln Val Val Leu Gln Glu Glu Gly Asn Cys Thr Leu Cys Asn Asn Met
    165                 170                 175

tgc ccg aat gaa gtg gat ggt gac gaa tcc aca acg tgg ctg ggg gtc       633
Cys Pro Asn Glu Val Asp Gly Asp Glu Ser Thr Thr Trp Leu Gly Val
180                 185                 190                 195

ttt gcg ccg aac atc acc gcg cga ttg aac gct gct gcg ccg agt gcc       681
Phe Ala Pro Asn Ile Thr Ala Arg Leu Asn Ala Ala Ala Pro Ser Ala
                200                 205                 210

aac ctc tca gac agc gac gcg ctc act ctc atg gat atg tgc ccg ttc       729
Asn Leu Ser Asp Ser Asp Ala Leu Thr Leu Met Asp Met Cys Pro Phe
            215                 220                 225

gac act ctc agc tcc ggg aac gcc agc ccc ttc tgt gac cta ttt acc       777
Asp Thr Leu Ser Ser Gly Asn Ala Ser Pro Phe Cys Asp Leu Phe Thr
        230                 235                 240

gcg gag gag tat gtg tcg tac gag tac tac tat gac ctc gac aag tac       825
Ala Glu Glu Tyr Val Ser Tyr Glu Tyr Tyr Tyr Asp Leu Asp Lys Tyr
    245                 250                 255

tat ggc acg ggc ccc ggg aac gct ctc ggt cct gtc cag ggc gtc gga       873
Tyr Gly Thr Gly Pro Gly Asn Ala Leu Gly Pro Val Gln Gly Val Gly
260                 265                 270                 275

tac gtc aat gag ctg ctt gca cgc ttg acc ggc caa gcc gtt cga gac       921
Tyr Val Asn Glu Leu Leu Ala Arg Leu Thr Gly Gln Ala Val Arg Asp
                280                 285                 290

gag acg cag acg aac cgc acg ctc gac agc gac cct gca aca ttc ccg       969
Glu Thr Gln Thr Asn Arg Thr Leu Asp Ser Asp Pro Ala Thr Phe Pro
            295                 300                 305

ctg aac cgt acg ttc tac gcc gac ttc tcg cat gat aac acc atg gtg       1017
Leu Asn Arg Thr Phe Tyr Ala Asp Phe Ser His Asp Asn Thr Met Val
        310                 315                 320

ccc atc ttt gcg gcg ctc ggg ctc ttc aac gcc acc gcc ctc gac ccg       1065
Pro Ile Phe Ala Ala Leu Gly Leu Phe Asn Ala Thr Ala Leu Asp Pro
    325                 330                 335

ctg aag ccc gac gag aac agg ttg tgg gtg gac tct aag ctg gta ccg       1113
Leu Lys Pro Asp Glu Asn Arg Leu Trp Val Asp Ser Lys Leu Val Pro
340                 345                 350                 355

ttc tct gga cat atg acg gtc gag aag ctg gca tgt tct ggg aag gag       1161
Phe Ser Gly His Met Thr Val Glu Lys Leu Ala Cys Ser Gly Lys Glu
                360                 365                 370

gcg gtc agg gtg ctc gtg aac gac gcg gtg cag ccg ctg gag ttc tgc       1209
Ala Val Arg Val Leu Val Asn Asp Ala Val Gln Pro Leu Glu Phe Cys
            375                 380                 385

gga ggt gtt gat ggg gtg tgc gag ctt tcg gct ttc gta gag agc cag       1257
Gly Gly Val Asp Gly Val Cys Glu Leu Ser Ala Phe Val Glu Ser Gln
        390                 395                 400

acg tat gcg cgg gag aat ggg caa ggc gac ttc gcc aag tgc ggc ttt       1305
Thr Tyr Ala Arg Glu Asn Gly Gln Gly Asp Phe Ala Lys Cys Gly Phe
    405                 410                 415

gtt ccg tcg gaa tag                                                   1320
Val Pro Ser Glu
420

<210>  2
<211>  423
<212>  PRT
<213>  Peniophora lycii

<400>  2

Ser Leu Ala Leu Ser Thr Gln Phe Ser Phe Val Ala Ala Gln Leu Pro
1               5                   10                  15

Ile Pro Ala Gln Asn Thr Ser Asn Trp Gly Pro Tyr Asp Pro Phe Phe
            20                  25                  30

Pro Val Glu Pro Tyr Ala Ala Pro Pro Glu Gly Cys Thr Val Thr Gln
        35                  40                  45

Val Asn Leu Ile Gln Arg His Gly Ala Arg Trp Pro Thr Ser Gly Ala
    50                  55                  60

Arg Ser Arg Gln Val Ala Ala Val Ala Lys Ile Gln Met Ala Arg Pro
65                  70                  75                  80

Phe Thr Asp Pro Lys Tyr Glu Phe Leu Asn Asp Phe Val Tyr Lys Phe
                85                  90                  95

Gly Val Ala Asp Leu Leu Pro Phe Gly Ala Asn Gln Ser His Gln Thr
            100                 105                 110

Gly Thr Asp Met Tyr Thr Arg Tyr Ser Thr Leu Phe Glu Gly Gly Asp
        115                 120                 125

Val Pro Phe Val Arg Ala Ala Gly Asp Gln Arg Val Val Asp Ser Ser
    130                 135                 140

Thr Asn Trp Thr Ala Gly Phe Gly Asp Ala Ser Gly Glu Thr Val Leu
145                 150                 155                 160

Pro Thr Leu Gln Val Val Leu Gln Glu Glu Gly Asn Cys Thr Leu Cys
                165                 170                 175

Asn Asn Met Cys Pro Asn Glu Val Asp Gly Asp Glu Ser Thr Thr Trp
            180                 185                 190

Leu Gly Val Phe Ala Pro Asn Ile Thr Ala Arg Leu Asn Ala Ala Ala
        195                 200                 205

Pro Ser Ala Asn Leu Ser Asp Ser Asp Ala Leu Thr Leu Met Asp Met
    210                 215                 220

Cys Pro Phe Asp Thr Leu Ser Ser Gly Asn Ala Ser Pro Phe Cys Asp
225                 230                 235                 240

Leu Phe Thr Ala Glu Glu Tyr Val Ser Tyr Glu Tyr Tyr Tyr Asp Leu
                245                 250                 255

Asp Lys Tyr Tyr Gly Thr Gly Pro Gly Asn Ala Leu Gly Pro Val Gln
            260                 265                 270

Gly Val Gly Tyr Val Asn Glu Leu Leu Ala Arg Leu Thr Gly Gln Ala
        275                 280                 285

Val Arg Asp Glu Thr Gln Thr Asn Arg Thr Leu Asp Ser Asp Pro Ala
    290                 295                 300

Thr Phe Pro Leu Asn Arg Thr Phe Tyr Ala Asp Phe Ser His Asp Asn
305                 310                 315                 320

Thr Met Val Pro Ile Phe Ala Ala Leu Gly Leu Phe Asn Ala Thr Ala
                325                 330                 335

Leu Asp Pro Leu Lys Pro Asp Glu Asn Arg Leu Trp Val Asp Ser Lys
            340                 345                 350

Leu Val Pro Phe Ser Gly His Met Thr Val Glu Lys Leu Ala Cys Ser
        355                 360                 365

Gly Lys Glu Ala Val Arg Val Leu Val Asn Asp Ala Val Gln Pro Leu
    370                 375                 380

Glu Phe Cys Gly Gly Val Asp Gly Val Cys Glu Leu Ser Ala Phe Val
385                 390                 395                 400

Glu Ser Gln Thr Tyr Ala Arg Glu Asn Gly Gln Gly Asp Phe Ala Lys
                405                 410                 415

Cys Gly Phe Val Pro Ser Glu
            420

<210>  3
<211>  42
<212>  DNA
<213>  Artificial Sequence

<220>
<223>  primer 620AM34

<220>
<221>  misc_feature
<223>  primer

<400>  3
gagtactatc ttgcatttgt actaggagtt tagtgaactt gc                          42

<210>  4
<211>  41
<212>  DNA
<213>  Artificial Sequence

<220>
<223>  primer 680AM35


<220>
<221>  misc_feature
<223>  primer

<400>  4
atggttatgg atttcgggga ttcttcgagc gtcccaaaac c                          41


<210>  5
<211>  20
<212>  DNA
<213>  Artificial Sequence

<220>
<223>  primer AM34


<220>
<221>  misc_feature
<223>  primer

<400>  5
taggagttta gtgaacttgc                                                  20


<210>  6
<211>  18
<212>  DNA
<213>  Artificial Sequence

<220>
<223>  primer AM35


<220>
<221>  misc_feature
<223>  primer

<400>  6
ttcgagcgtc ccaaaacc                                                    18


<210>  7
<211>  22
<212>  DNA
<213>  Artificial Sequence

<220>
<223>  primer 620


<220>
<221>  misc_feature
<223>  primer

<400>  7
gagtactatc ttgcatttgt ac                                               22


<210>  8
<211>  21
<212>  DNA
<213>  Artificial Sequence

<220>
<223>  primer 680


<220>
<221>  misc_feature
<223>  primer

<400>  8
atggttatgg atttcgggga t                                                21


<210>  9
<211>  62
<212>  DNA
<213>  Artificial Sequence

<220>
<223>  primer Dope1


<220>
<221>  misc_feature
<222>  (19)..(19)
<223>  N in position 19 is 91%A, 3%G, 3%T, 3%C

<220>
<221>  misc_feature
<222>  (20)..(20)
<223>  N in position 20 is 91%C, 3%G, 3%A, 3%T

<220>
<221>  misc_feature
<222>  (21)..(21)
<223>  N in position 21 is G or T

<220>
<221>  misc_feature
<222>  (22)..(22)
<223>  N in position is 88%C, 12%A

<220>
<221>  misc_feature
<222>  (23)..(23)
<223>  N in position 23 is 90%T, 3%G, 7%A

<220>
<221>  misc_feature
<222>  (24)..(24)
<223>  N in position 24 is 47%G, 8%A, 45%T

<220>
<221>  misc_feature
<222>  (25)..(25)
<223>  N in position 25 is 9%G, 91%A

<220>
<221>  misc_feature
<222>  (26)..(26)
<223>  N in position 26 is 83%G, 4%A, 9%T, 4%C

<220>
<221>  misc_feature
<222>  (27)..(27)
<223>  N in position 27 is T

<220>
<221>  misc_feature
<222>  (28)..(28)

<223>  N in position 28 is 91%T, 3%G, 3%A, 3%C

<220>
<221>  misc_feature
<222>  (29)..(29)
<223>  N in position 29 is 91%C, 3%G, 3%A, 3%T

<220>
<221>  misc_feature
<222>  (30)..(30)
<223>  N in position 30 is G or T

<220>
<221>  misc_feature
<222>  (31)..(31)
<223>  N in position 31 is 91%G, 3%C, 3%A, 3%T

<220>
<221>  misc_feature
<222>  (32)..(32)
<223>  N in position 32 is 91%G, 3%C, 3%A, 3%T-

<220>
<221>  misc_feature
<222>  (33)..(33)
<223>  N in position 33 is G or T

<220>
<221>  misc_feature
<222>  (34)..(34)
<223>  N in position 34 is 91%A, 3%G, 3%T, 3%C

<220>
<221>  misc_feature
<222>  (35)..(35)
<223>  N in position 35 is 91%A, 3%G, 3%T, 3%C

<220>
<221>  misc_feature
<222>  (36)..(36)
<223>  N in position 36 is 95%T, 5%G

<220>
<221>  misc_feature
<222>  (37)..(37)
<223>  N in position 37 is 91%G, 3%C, 3%A, 3%T

<220>
<221>  misc_feature
<222>  (38)..(38)
<223>  N in position 38 is 91%C, 3%G, 3%A, 3%T

<220>
<221>  misc_feature
<222>  (39)..(39)
<223>  N in position 39 is G or T

<220>
<221>  misc_feature
<222>  (40)..(40)
<223>  N in position 40 is 95%T, 5%G

<400>  9
   gatatgtgcc cgttcgacnn nnnnnntnnn nnnnnnnnnn cccccttctg tgacctattt      60
   ac                                                                    62


<210>  10

<211>  18
<212>  DNA
<213>  Artificial Sequence

<220>
<223>  primer Dope1R


<220>
<221>  misc_feature
<223>  primer

<400>  10
gtcgaacggg cacatatc                                                    18


<210>  11
<211>  43
<212>  DNA
<213>  Artificial Sequence

<220>
<223>  primer L102TI


<220>
<221>  misc_feature
<222>  (22)..(22)
<223>  N in position 22 is C or T

<400>  11
agttcggcgt cgccgatctg ancccgttcg gggctaacca atc                        43


<210>  12
<211>  53
<212>  DNA
<213>  Artificial Sequence

<220>
<223>  primer VE248+251X


<220>
<221>  misc_feature
<222>  (21)..(21)
<223>  N in position 21 is A or C or G

<220>
<221>  misc_feature
<222>  (22)..(22)
<223>  N in position 22 is A or C or G or T

<220>
<221>  misc_feature
<222>  (23)..(23)
<223>  N in position 23 is A or C or G or T

<220>
<221>  misc_feature
<222>  (30)..(30)
<223>  N in position 30 is A or C or G

<220>
<221>  misc_feature
<222>  (31)..(31)
<223>  N in position 31 is A or C or G or T

<220>

<221>  misc_feature
<222>  (32)..(32)
<223>  N in position 32 is A or C or G or T

<400>  12
tatttaccgc ggaggagtat nnntcgtacn nntactacta tgacctcgac aag          53


<210>  13
<211>  45
<212>  DNA
<213>  Artificial Sequence

<220>
<223>  primer V271AI


<220>
<221>  misc_feature
<222>  (21)..(21)
<223>  N in position 21 is A or G

<220>
<221>  misc_feature
<222>  (22)..(22)
<223>  N in position 22 is C or T

<400>  13
ccgggaacgc tctcggtcct nnccagggcg tcggatacgt caatg                   45


<210>  14
<211>  42
<212>  DNA
<213>  Artificial Sequence

<220>
<223>  primer G286X


<220>
<221>  misc_feature
<222>  (24)..(24)
<223>  N in position 24 is A or C or G

<220>
<221>  misc_feature
<222>  (25)..(26)
<223>  N in positions 25 and 26 are independently A or C or G or T

<400>  14
atgagctgct tgcacgcttg accnnncaag ccgttcgaga cg                      42

## Description

### Field of the Invention

The present invention relates to variants of a parent phytase, in particular variants of improved thermostability and/or specific activity. The invention also relates to DNA sequences encoding such variants, their production in a recombinant host cell, as well as methods of using the variants, in particular within the field of animal feed. A preferred parent phytase is the *Peniophora* phytase comprising SEQ ID NO: 2.

### Background of the Invention

EP 897010 entitled "Modified phytases" discloses, i.a., certain variants of an *Aspergillus fumigatus* phytase. EP 897985 entitled "Consensus phytases" discloses, i.a., a fungal consensus phytase which may be designed on the basis of, i.a., a multiple alignment of several ascomycete phytases. WO 99/48380 entitled "Thermostable phytases in feed preparation and plant expression" relates to certain aspects of using thermostable phytases. Examples of thermostable phytases are the various consensus phytases listed at p. 30 below the bold line. WO 00/43503 entitled "Improved phytases" relates i.a. to certain phytase variants of increased thermostability, which may be designed by a process similar to the one described in EP 897985. Examples of thermostable phytases are shown at p. 54-55 below the bold line. These phytases all have a percentage of identity to SEQ ID NO: 2 of below 75%.

It is an object of the present invention to provide novel and improved phytase variants, in particular of improved thermostability and/or increased specific activity.

### Background art

A phytase derived from *Peniophora lycii* is disclosed in WO 98/28408, and certain variants thereof in WO 99/49022 (see e.g. claim 31 at p. 76-77), which variants, however, for the present purposes have been disclaimed.

### Summary of the Invention

The present invention relates to a variant of a parent phytase, comprising a substitution in at least one position of at least one region selected from the group of regions consisting of: 20; 26; 28-31; 37-48; 55-69; 73-83; 91-119; 123-126; 135-142; 152-163; 169-199; 204-222; 229-238; 248-266; 284-289; 300-308; 321-335; 343-350; 384-398; 407-412; and 419-430; wherein
(a) the variant has phytase activity; and
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of: G26S; Y28N; D29S; F31Y; E42D,K,A; T45R; V461; W59F; S62D; A64K; R65Q,E,G,A; S66T; R67A,I,Q,K; Q68Y,V,I; K74R,A; A99N; D100S; L102V; P103E; F104L; N107T,Q; S109M; H110S,V; Q111E,N; T112A,S; M116F,I,L; A135S; D137Y; Q138D,S,G; D142A; ()154fH; S155N; G156P; E1570,S; E169S; E170A,P; G171A; ()17laS; T174P,A; N177S; N178S,T; M179T,V,L; N182A,V; V184G; D185E; ()185eT; G186S,A; D187R,K,S; ()187aV,T; E1880,S,A,V; S189E; V195L; N199A,P; L204N; A207D,H; S216T; D217E; L219Y,T; T220Y,N; L221 F; M222L; L230V; ()235eE,Q; V248R,I; S249Q,A; E251D; Y254A,L,G; D255N; D257K; Y259F; T262H; P264A; G286R,H; Q287K,S; A288P; T3210,S,G; M3221; V3231; P324A; A327S,F,I,L; F332Y; N333P; E344R; R346P; W348F; V349L,A,S; D350S,V; G388A; V393R; E395K,T; and L396R.

The present invention also relates to isolated nucleic acid sequences encoding the phytase variant and to nucleic acid constructs, vectors, and host cells comprising the nucleic acid sequences as well as methods for producing and using the phytase variants.

### Brief Description of the Figures

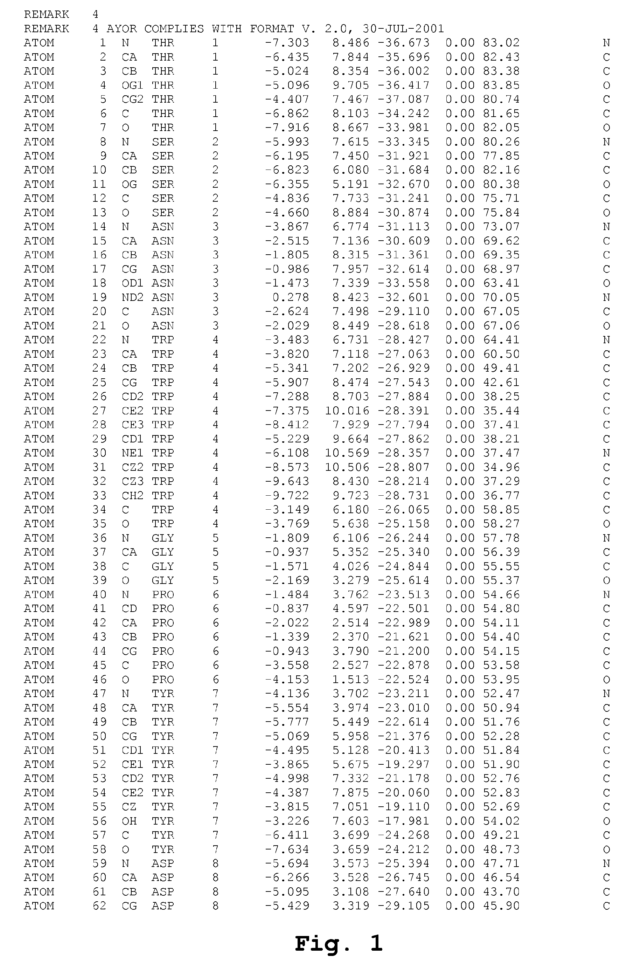

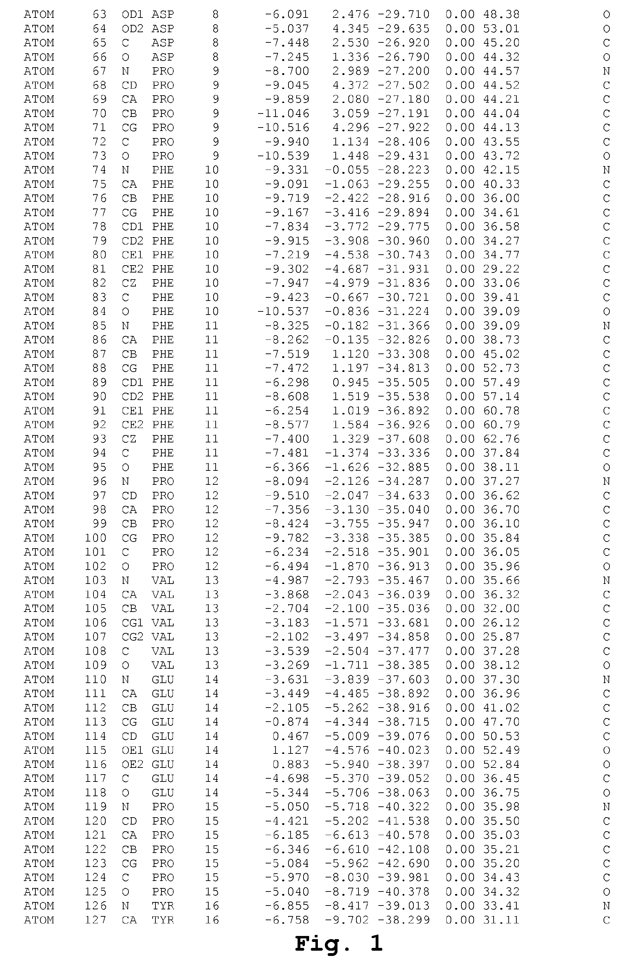

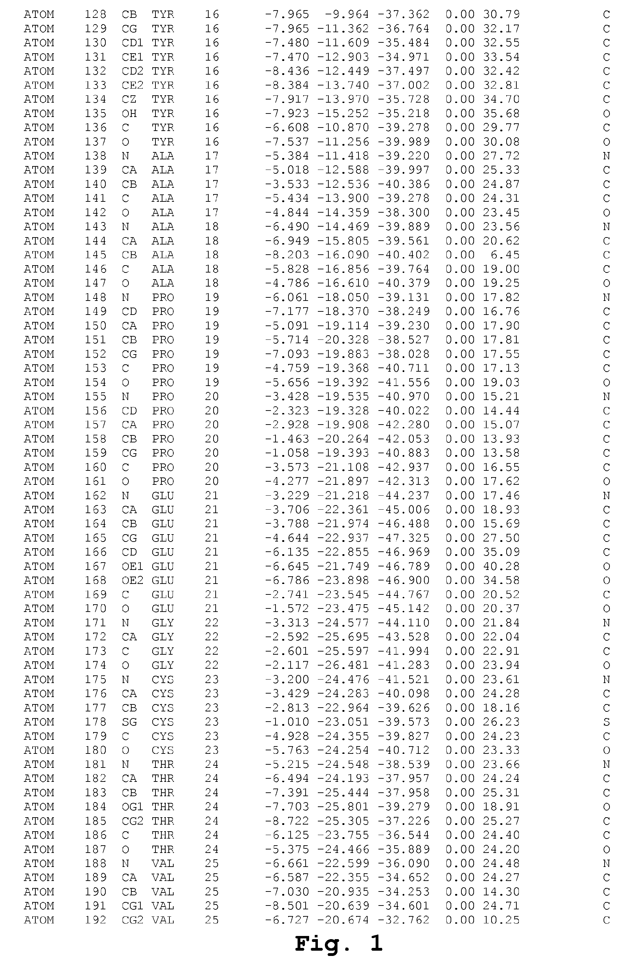

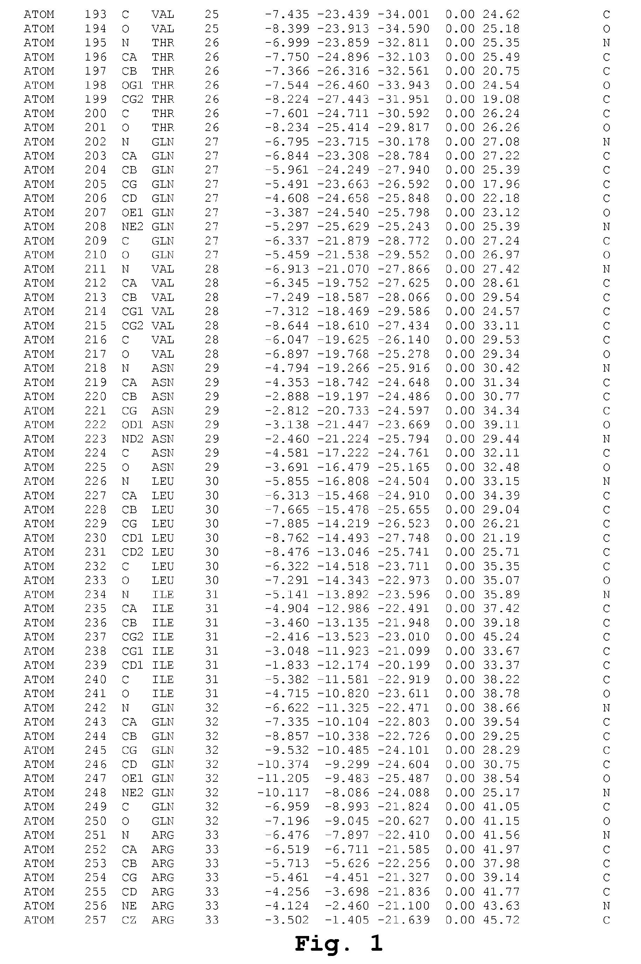

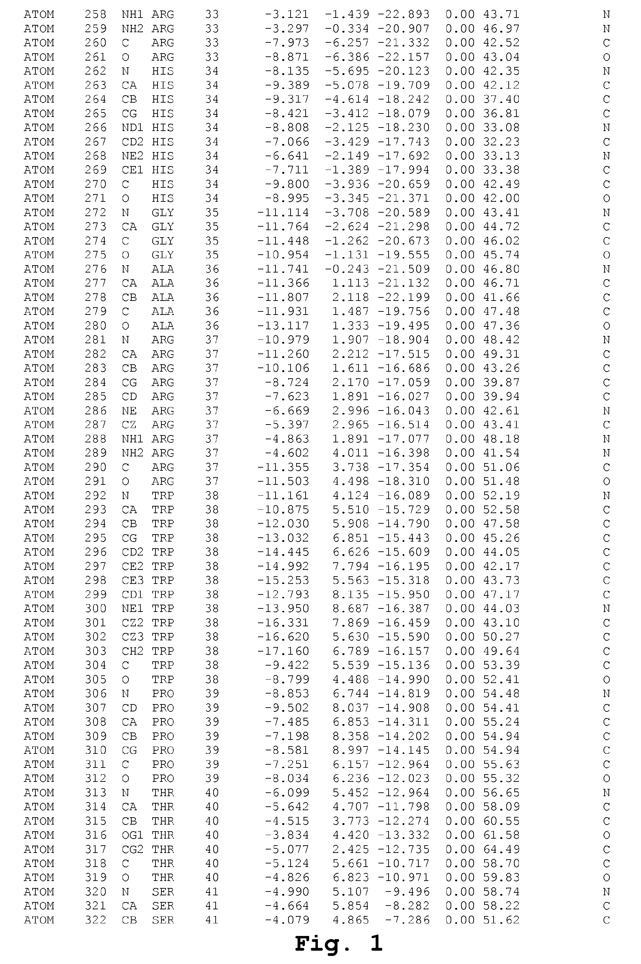

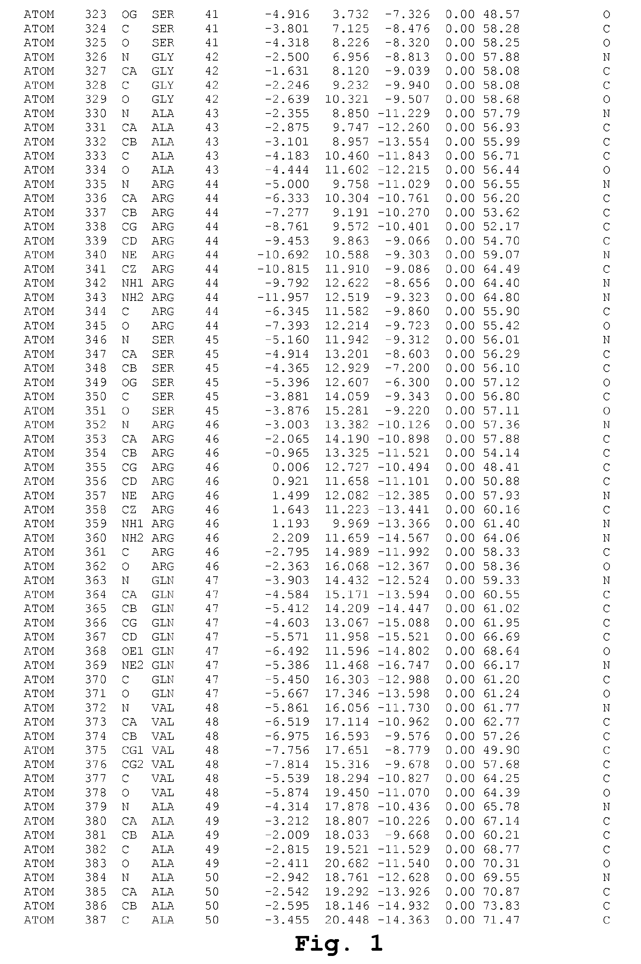

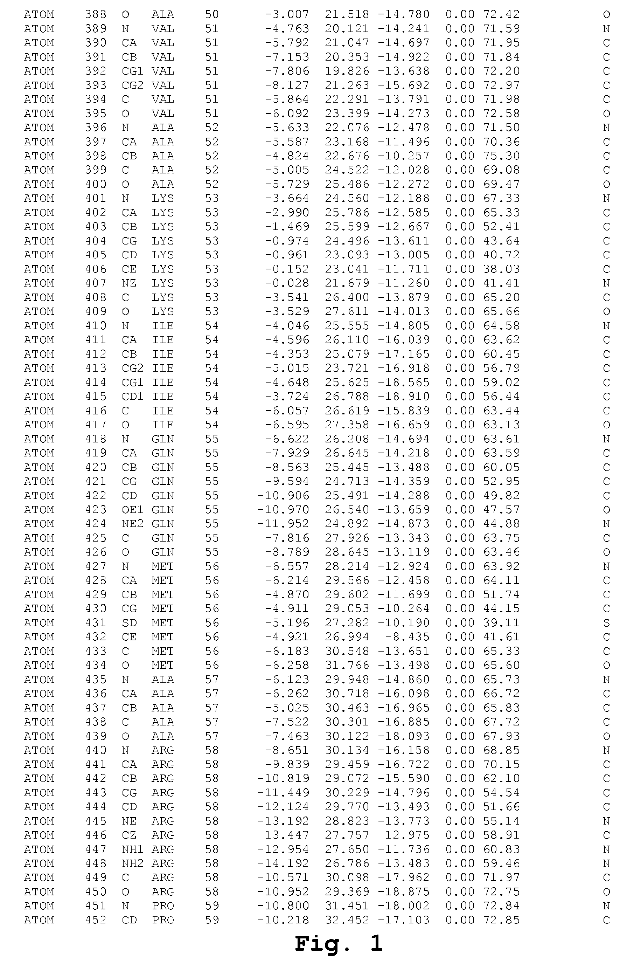

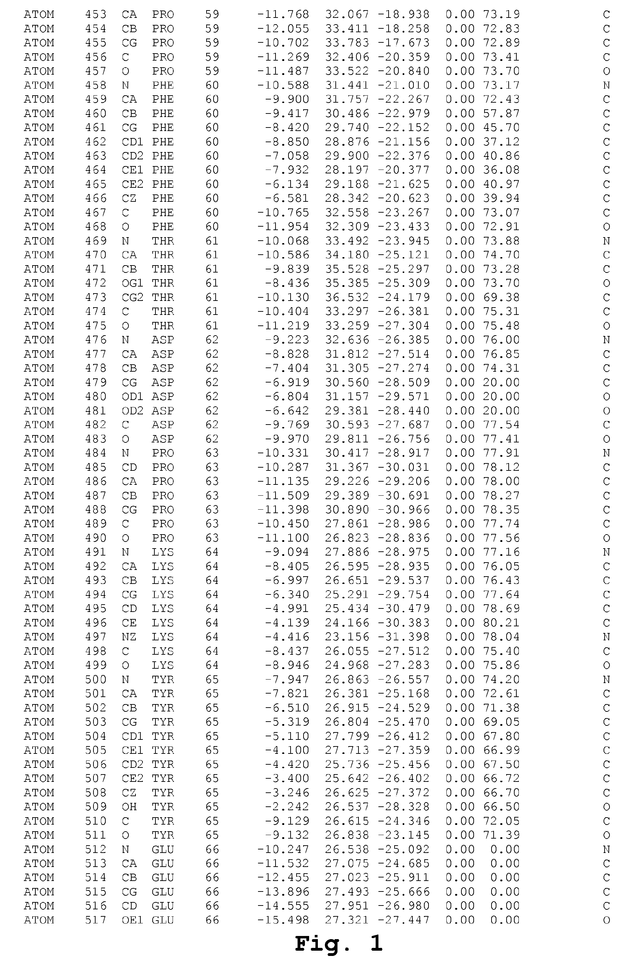

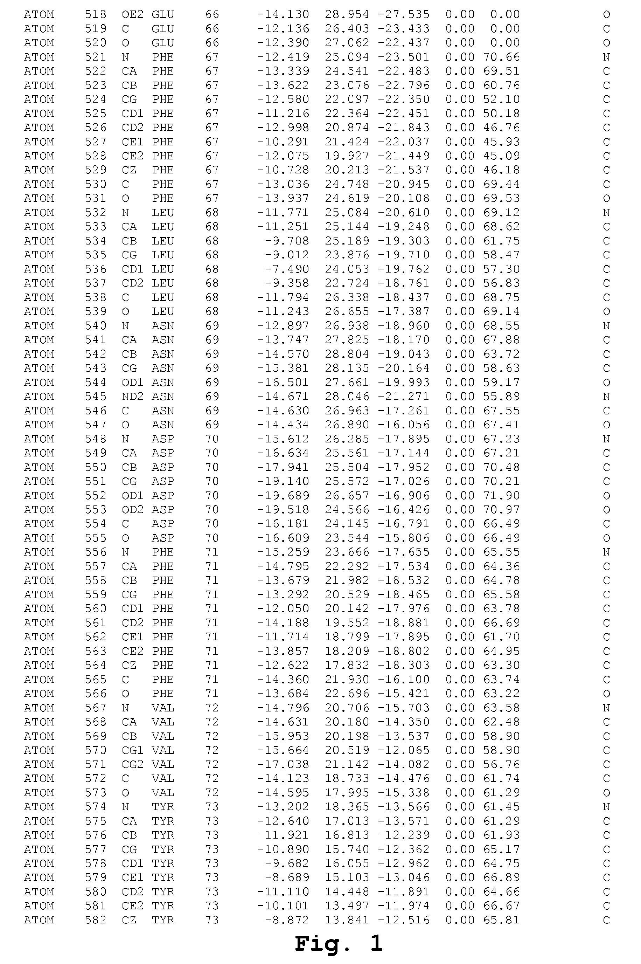

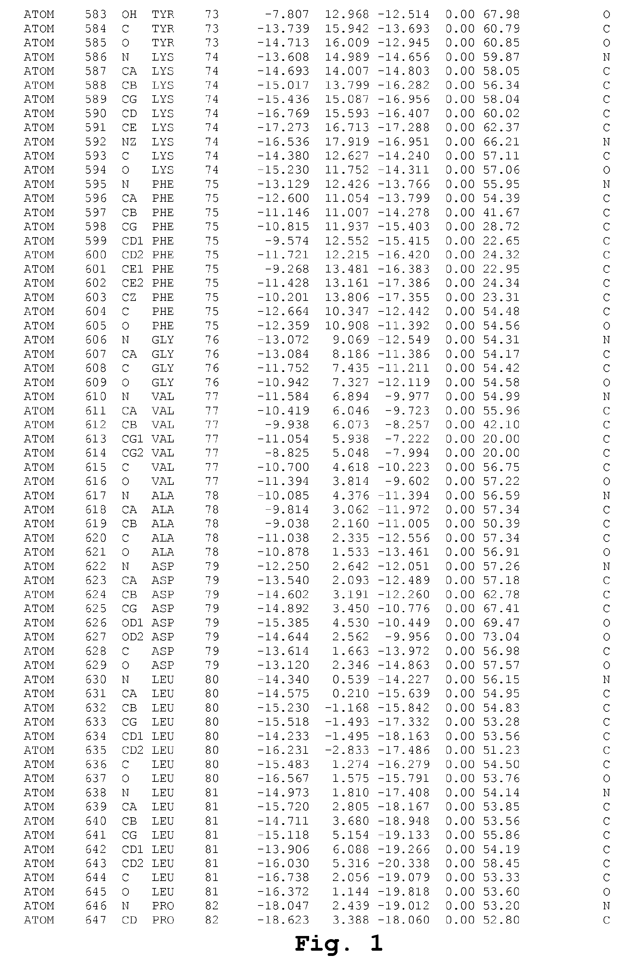

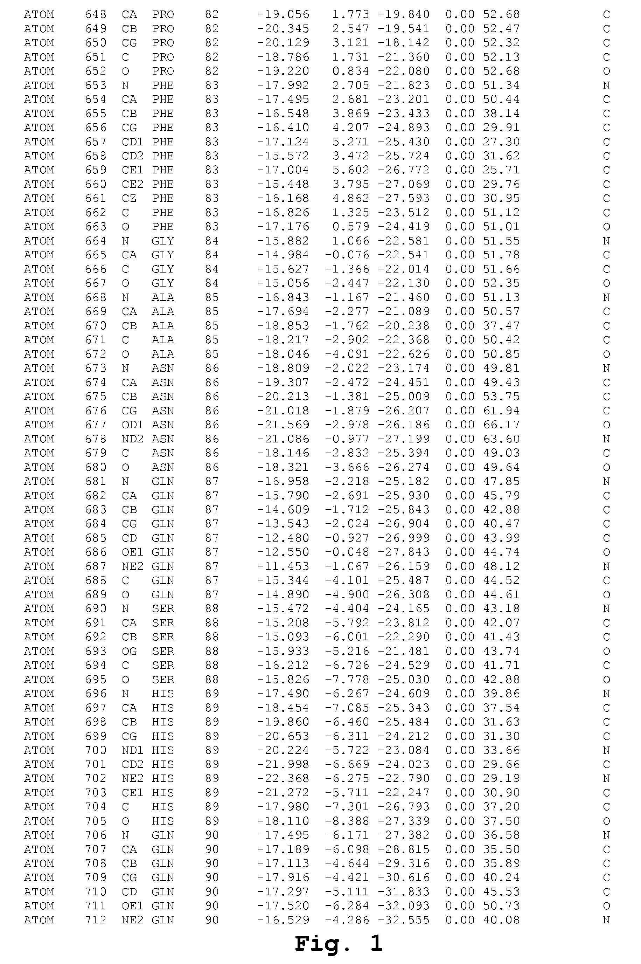

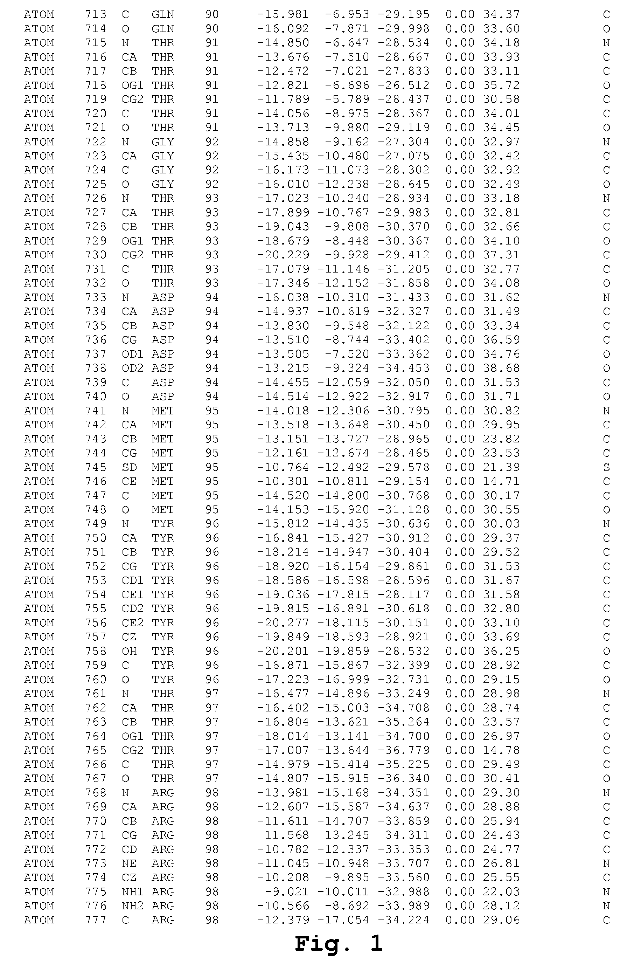

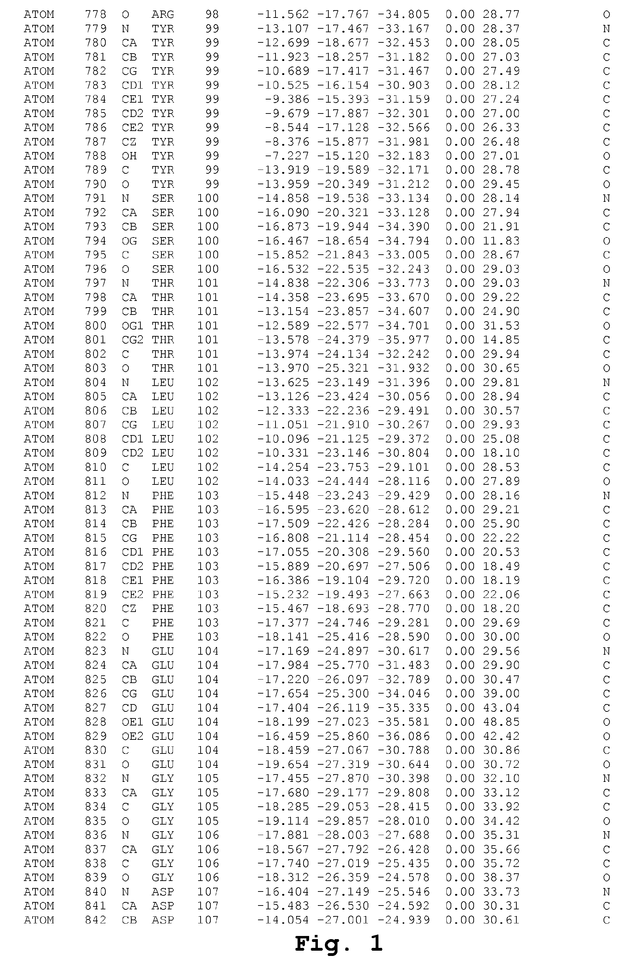

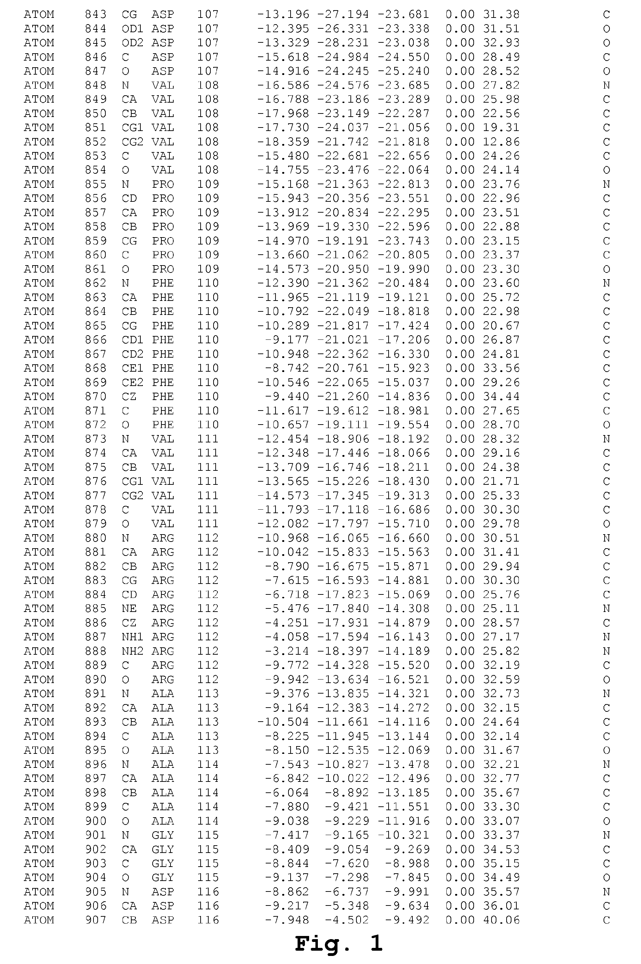

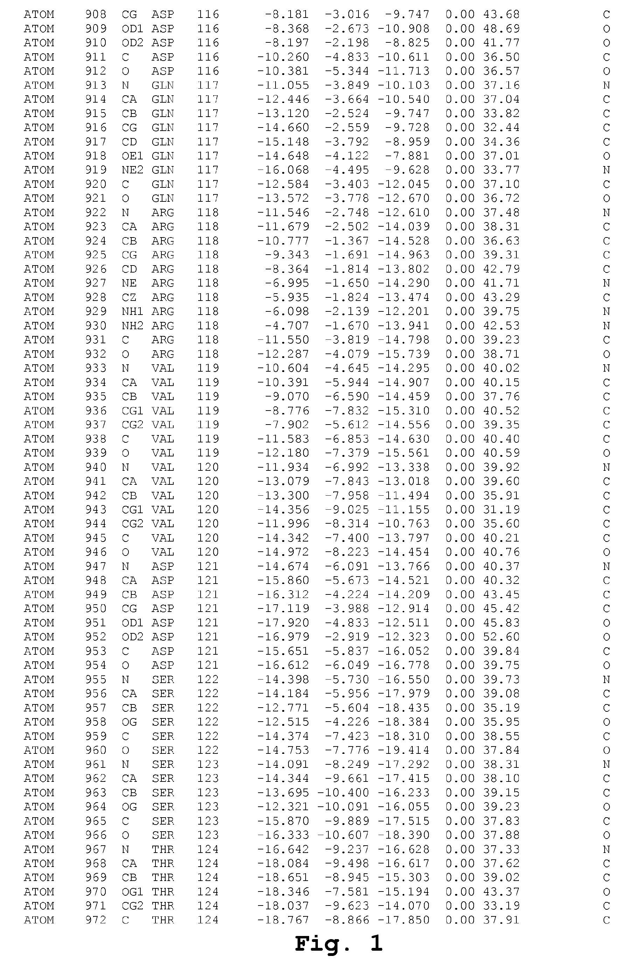

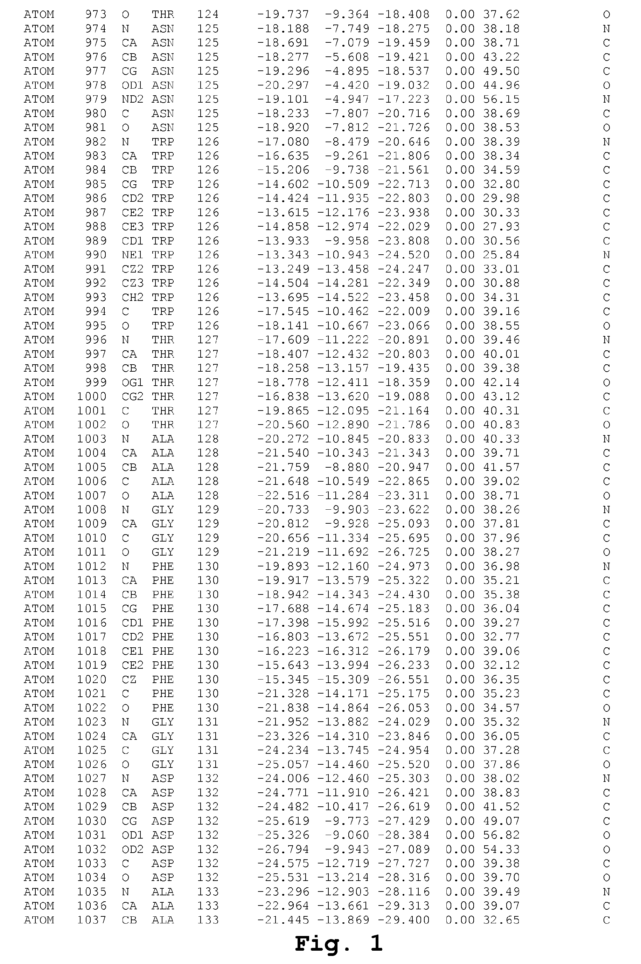

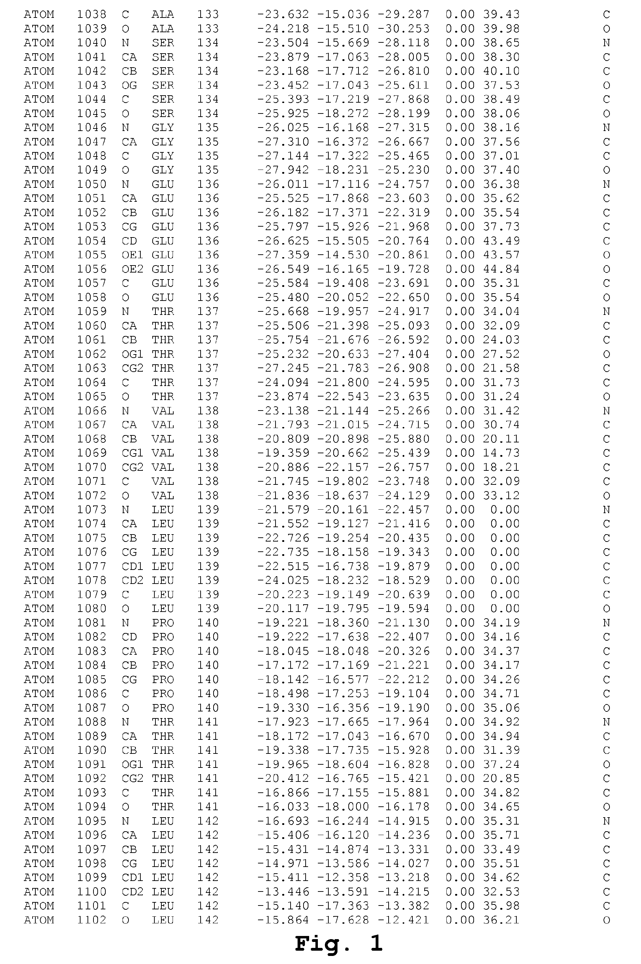

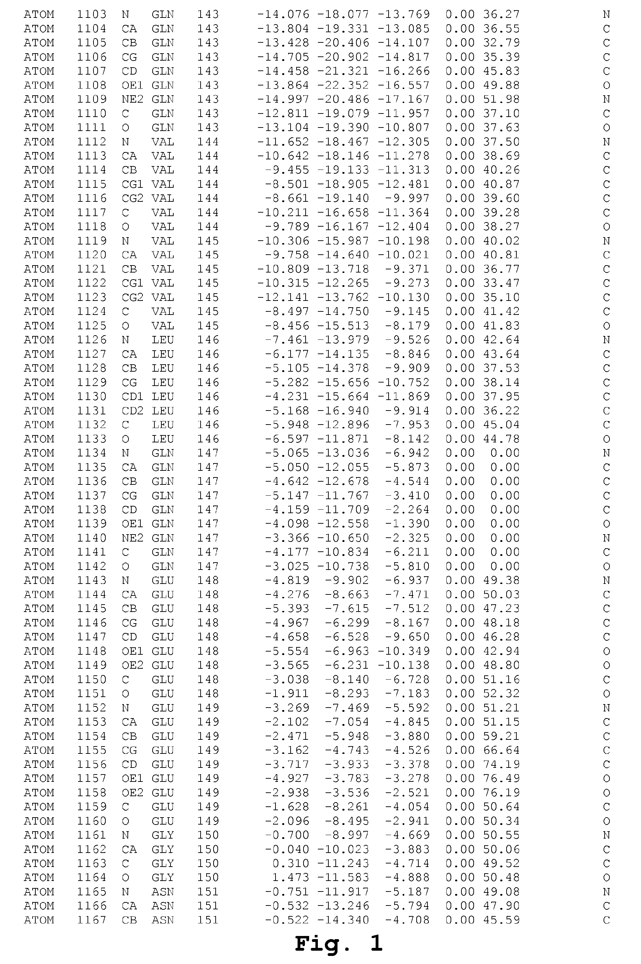

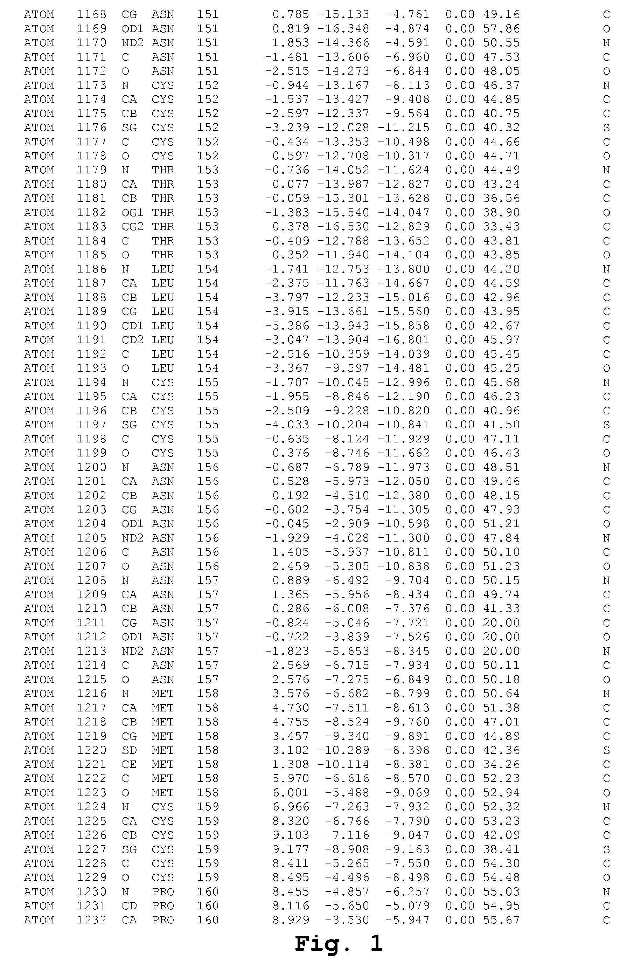

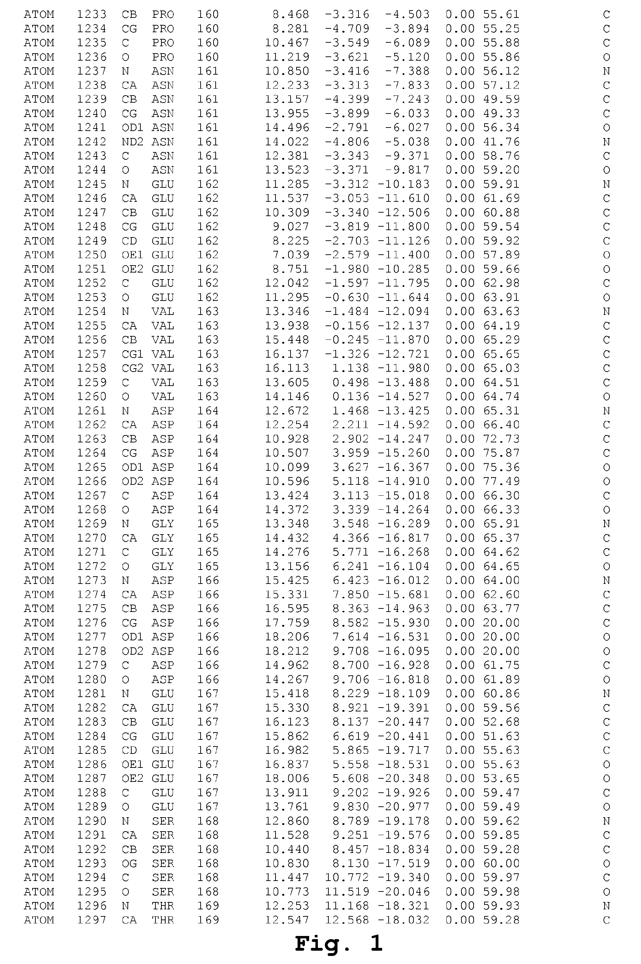

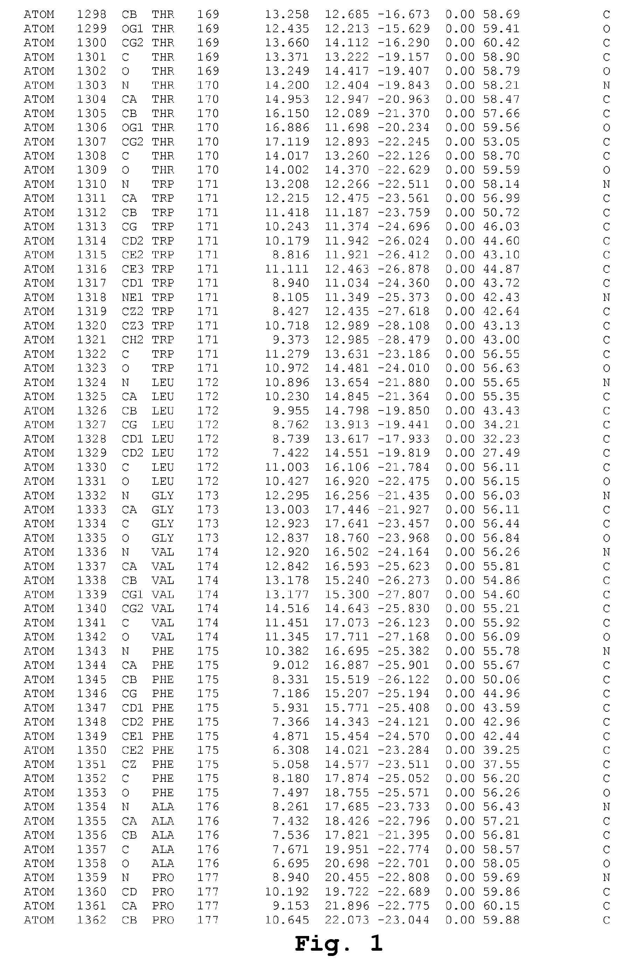

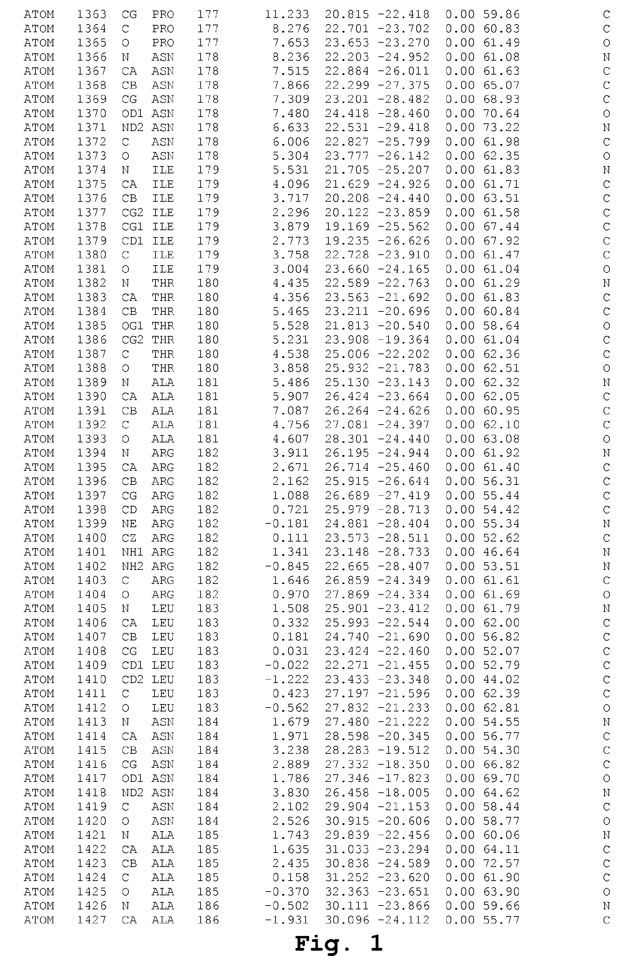

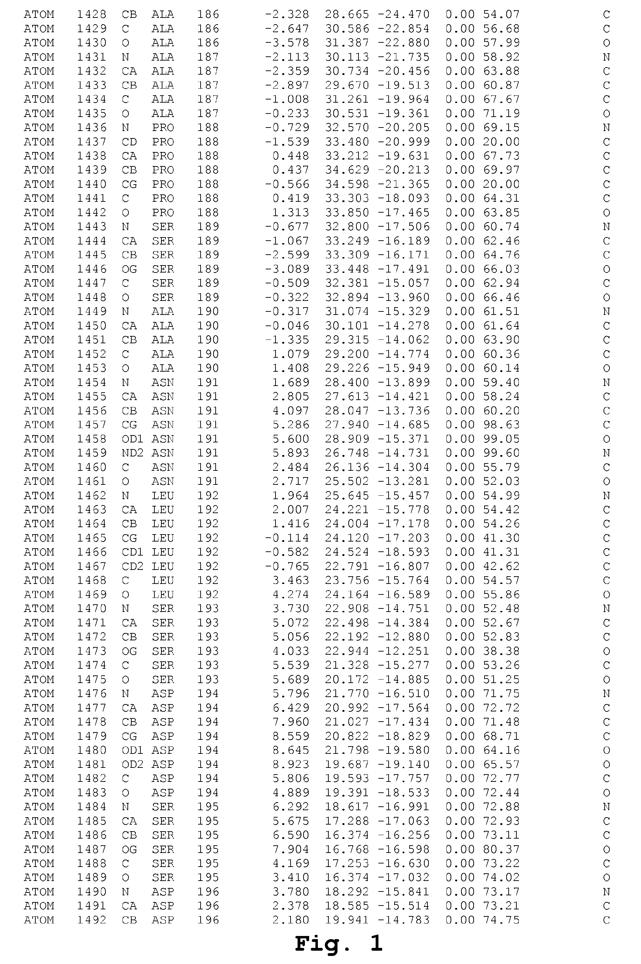

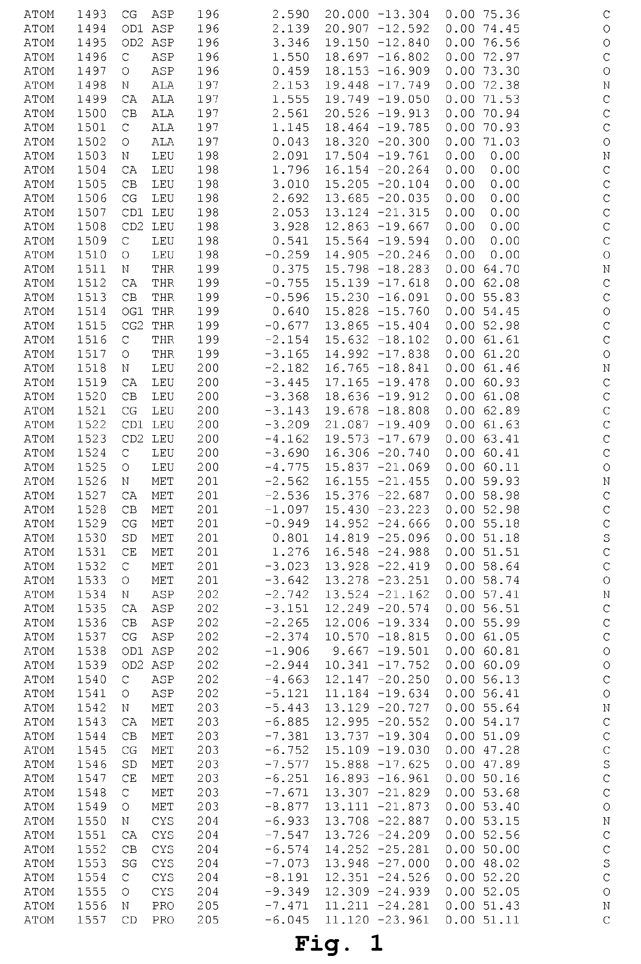

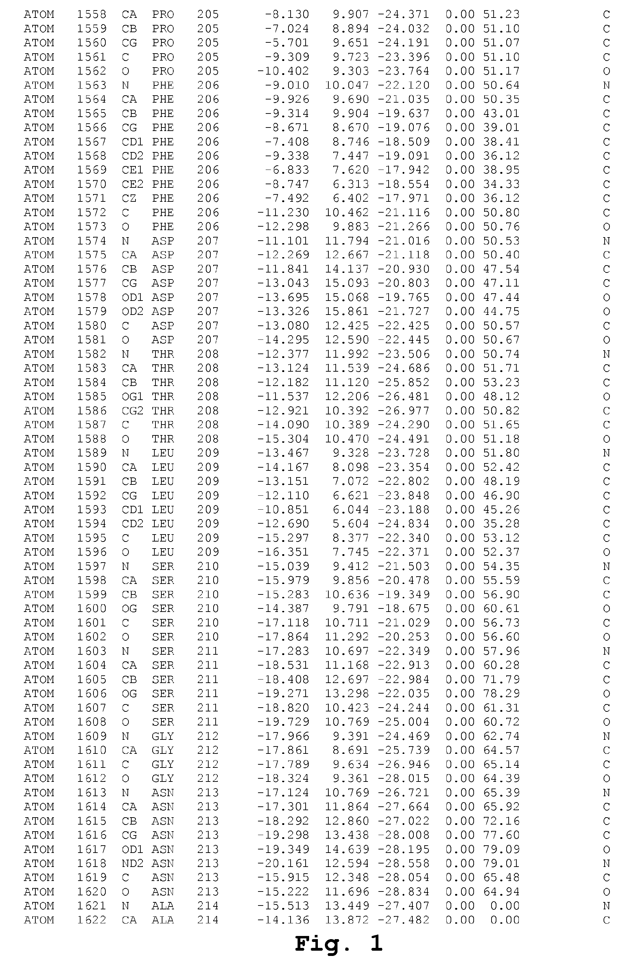

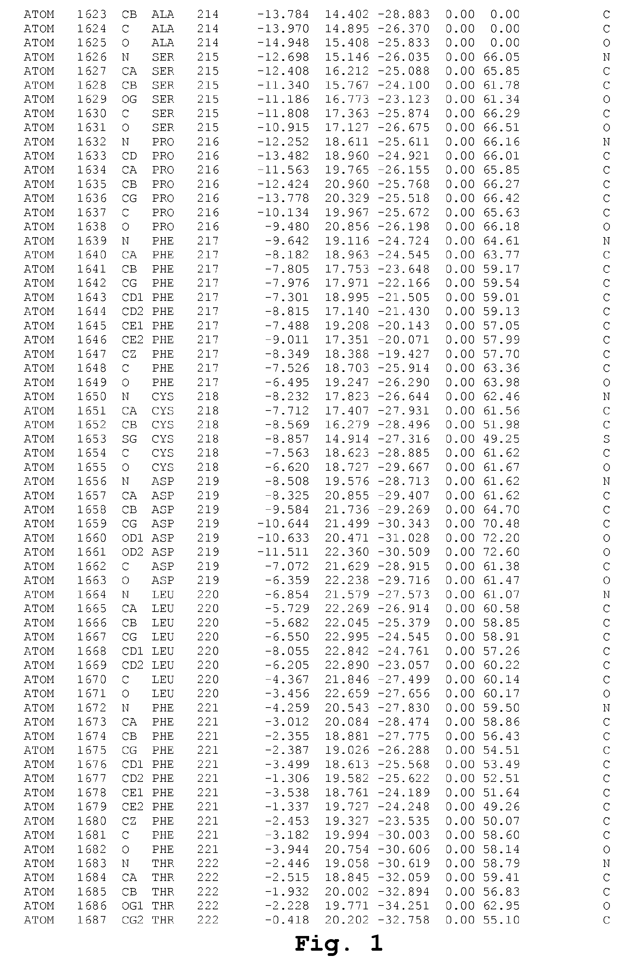

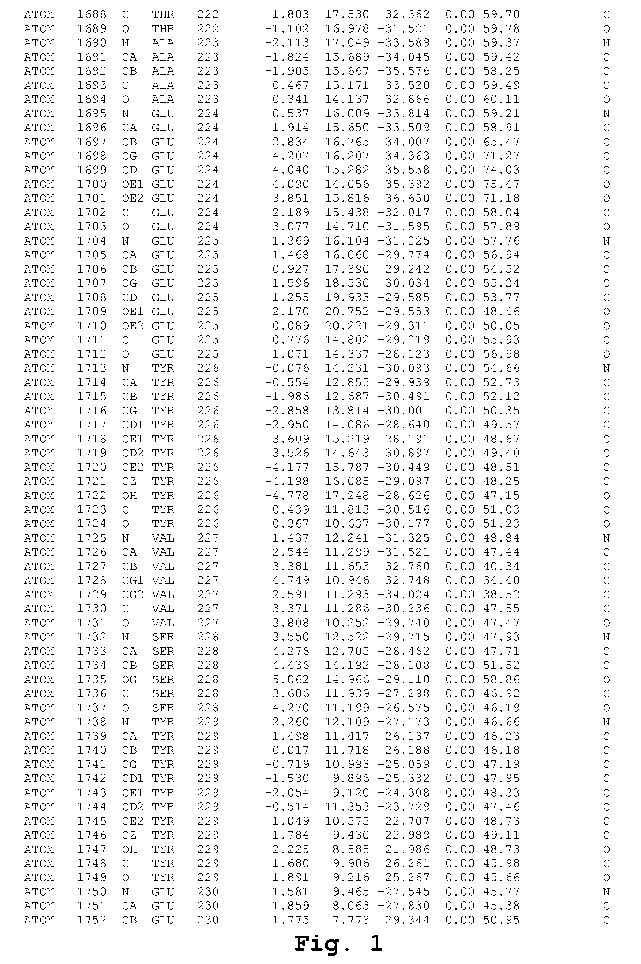

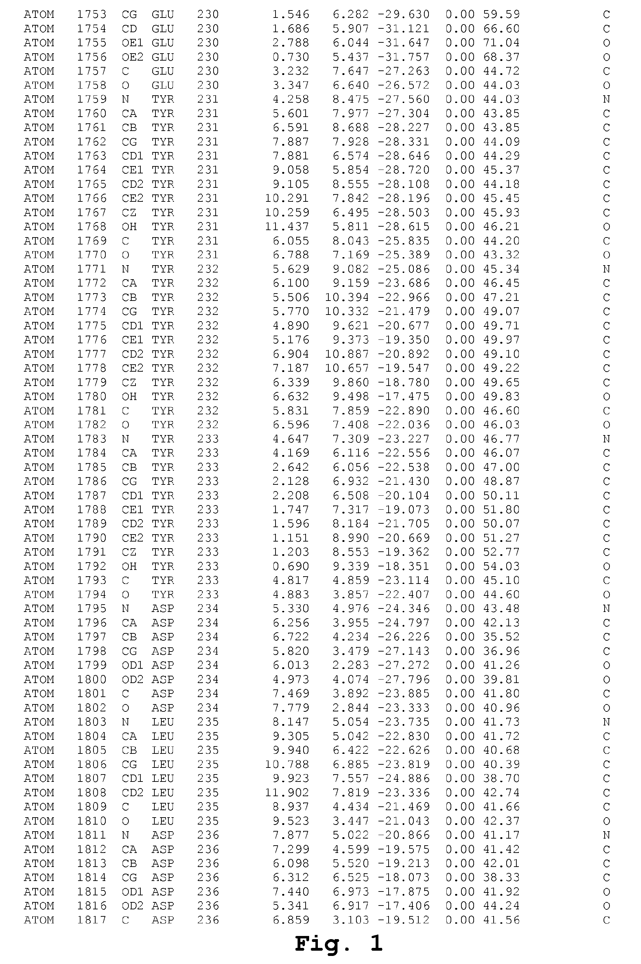

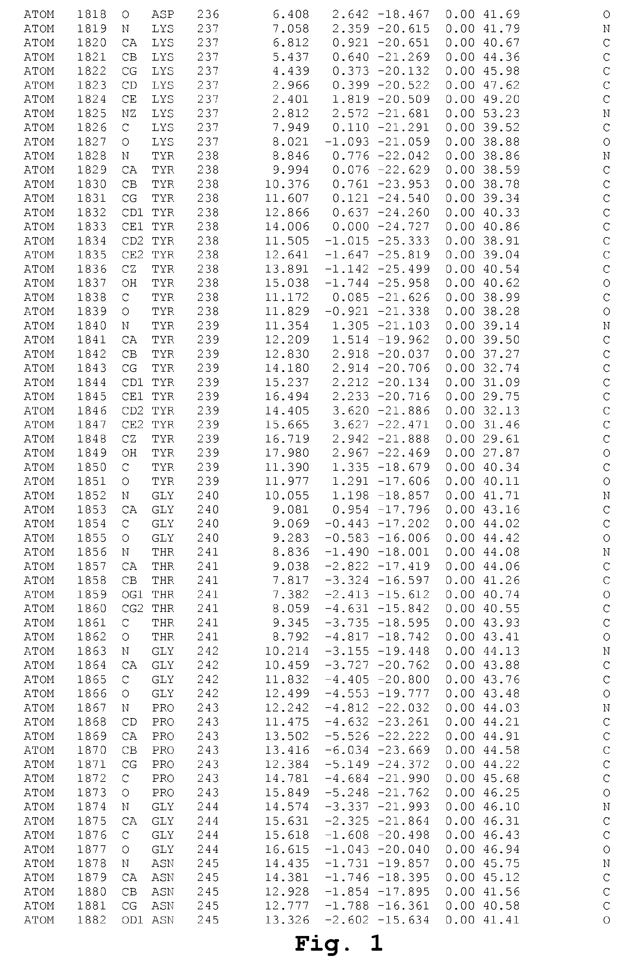

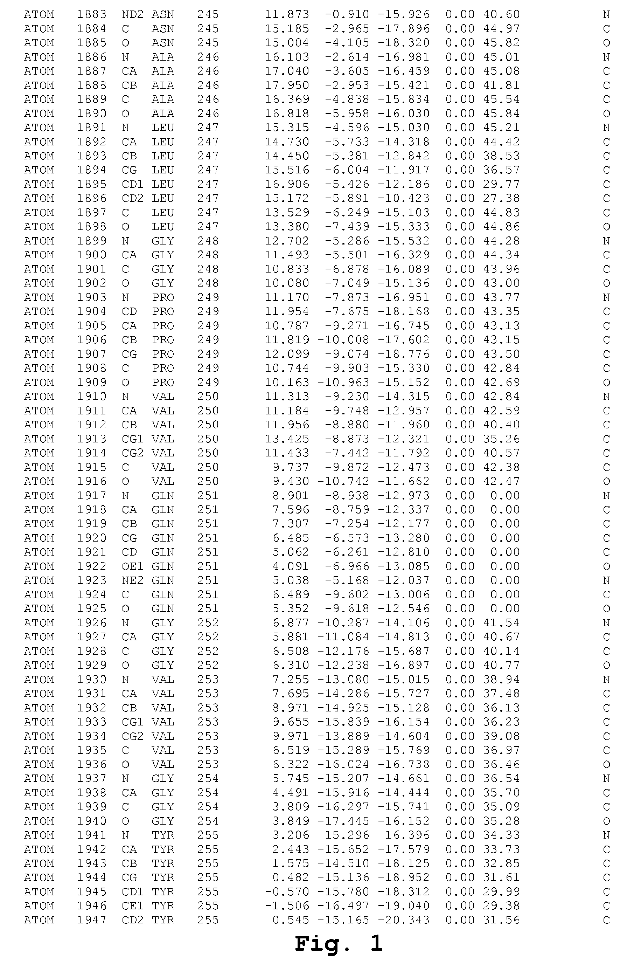

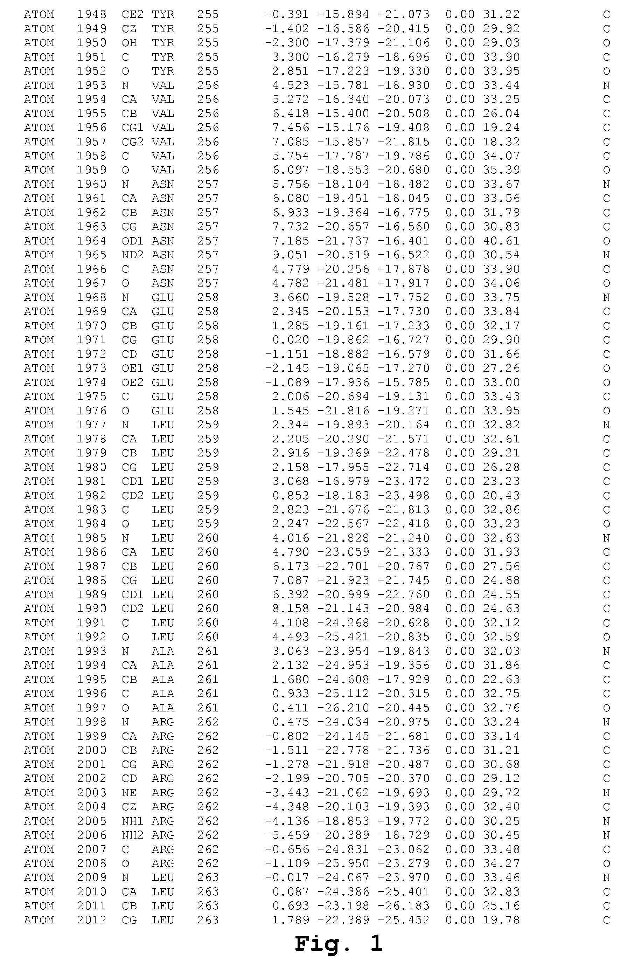

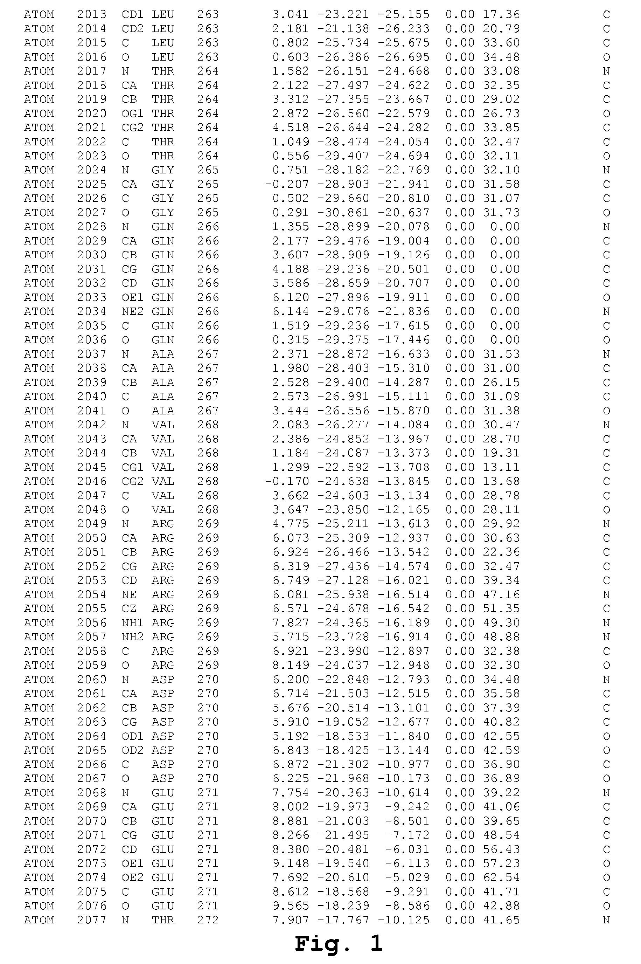

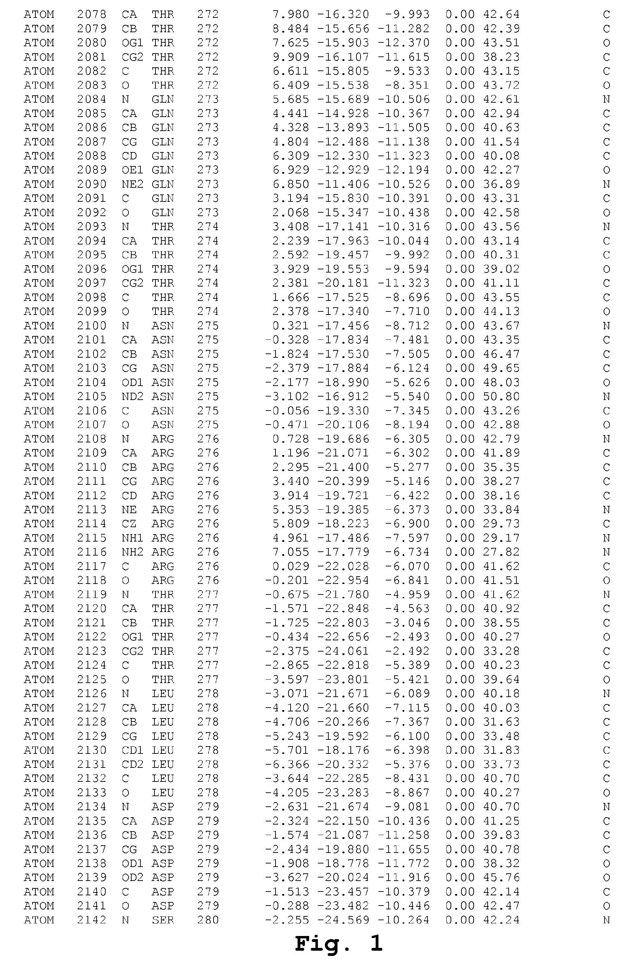

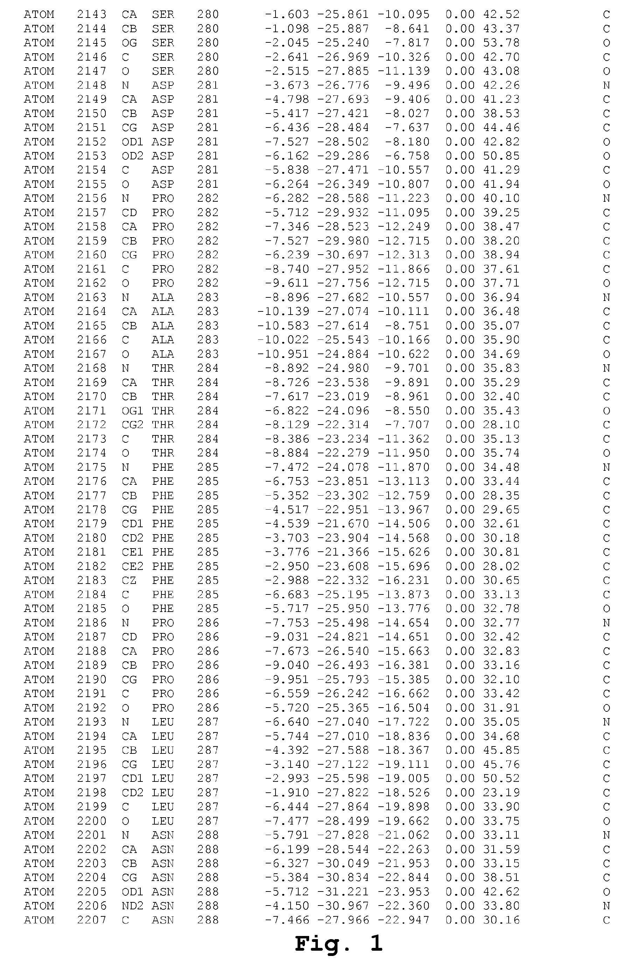

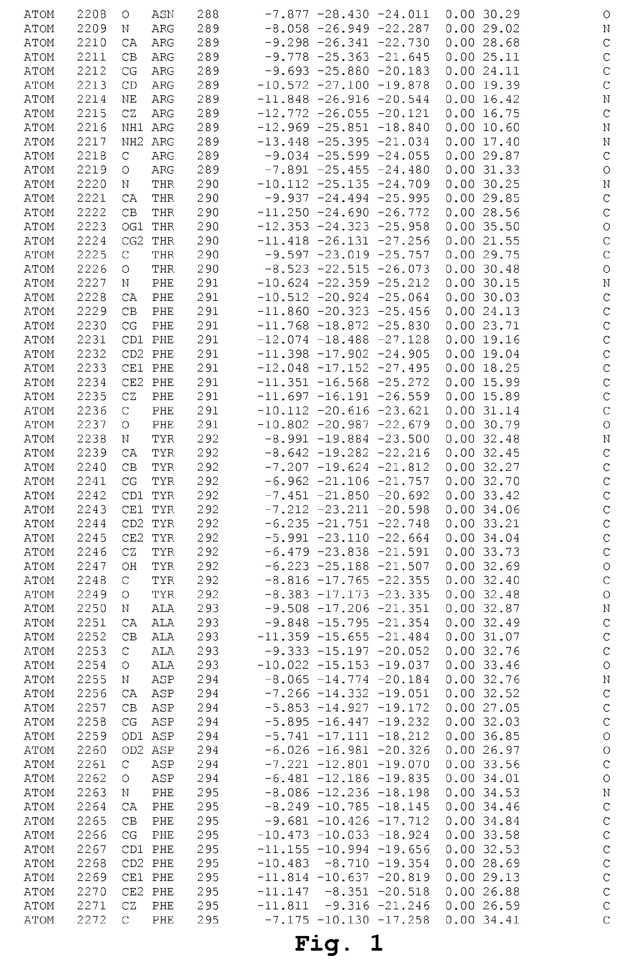

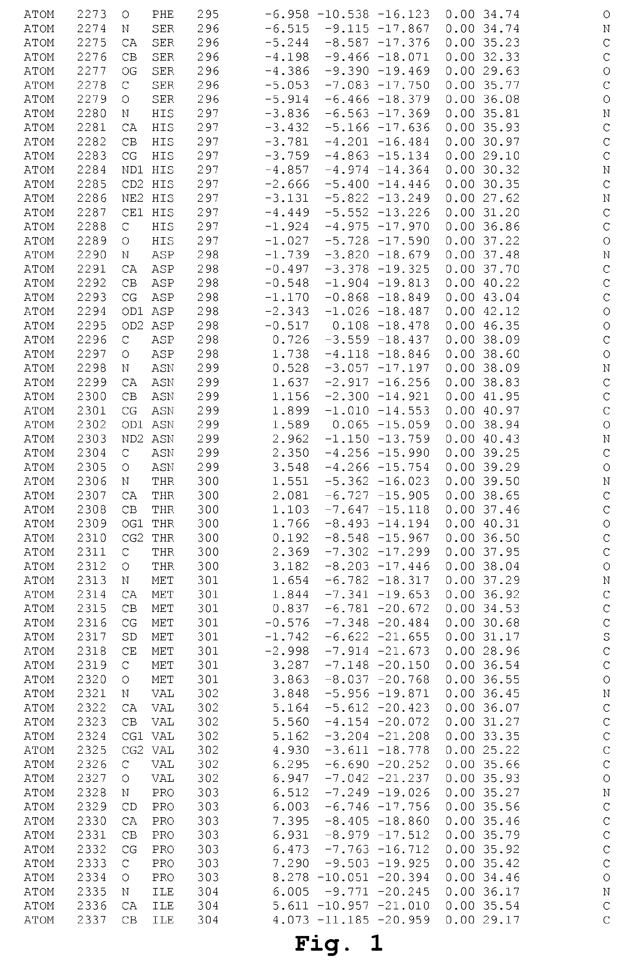

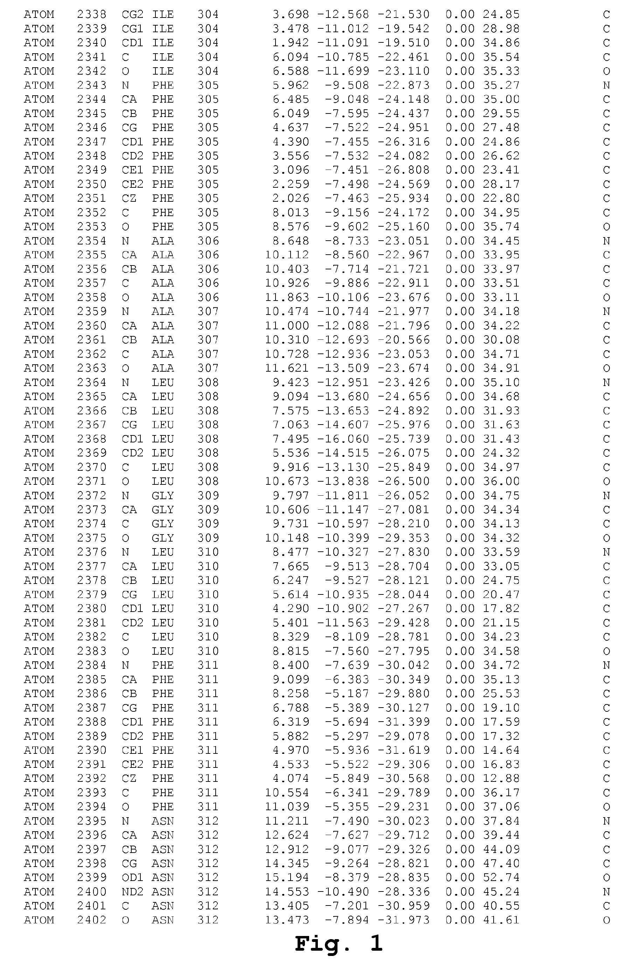

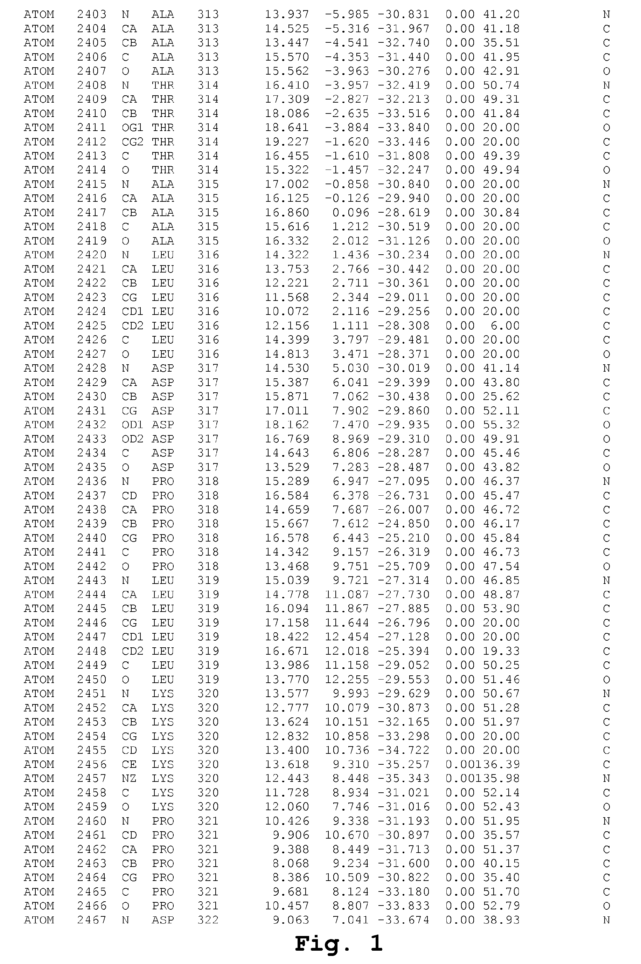

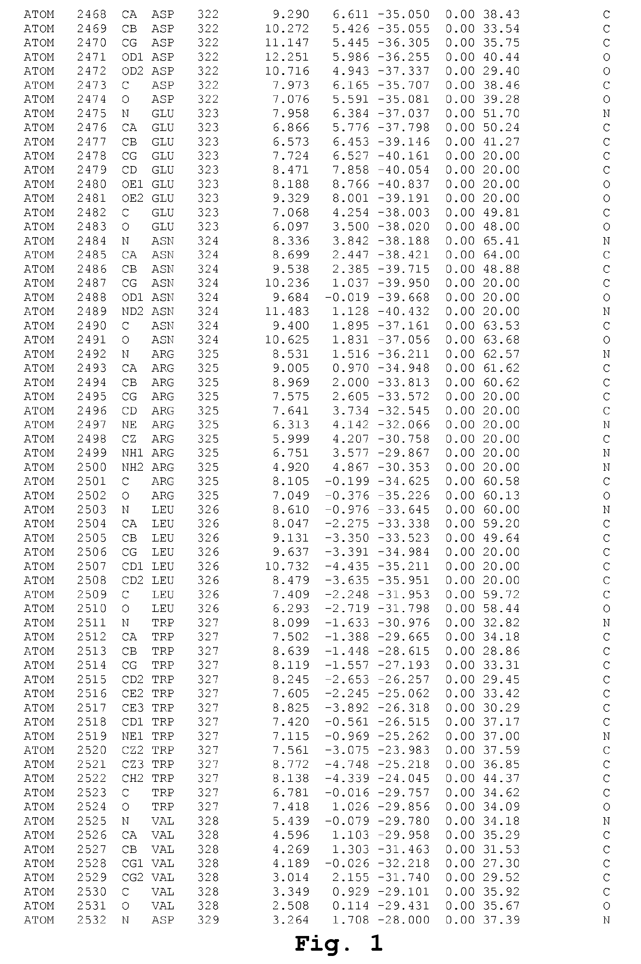

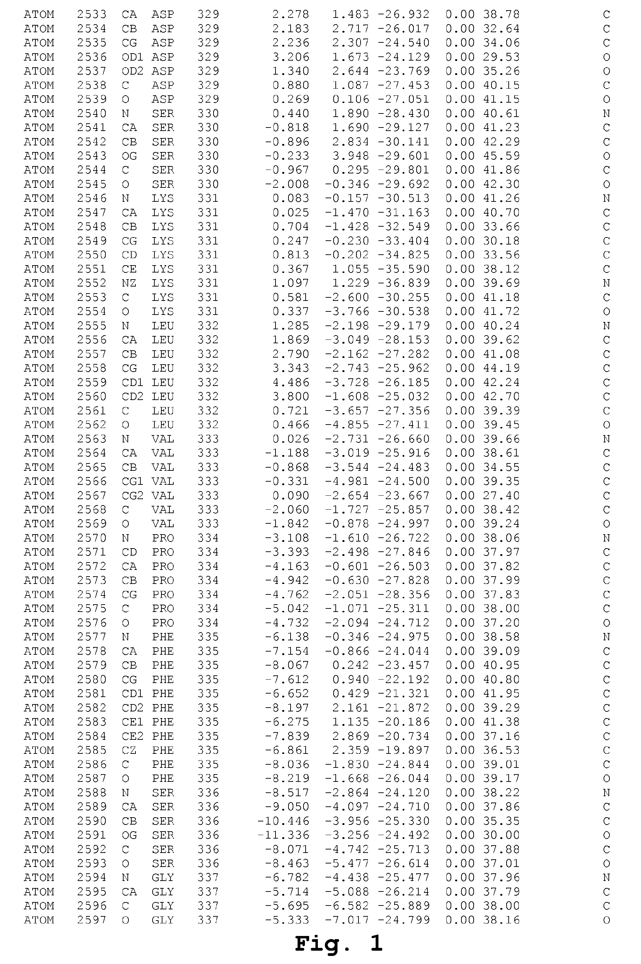

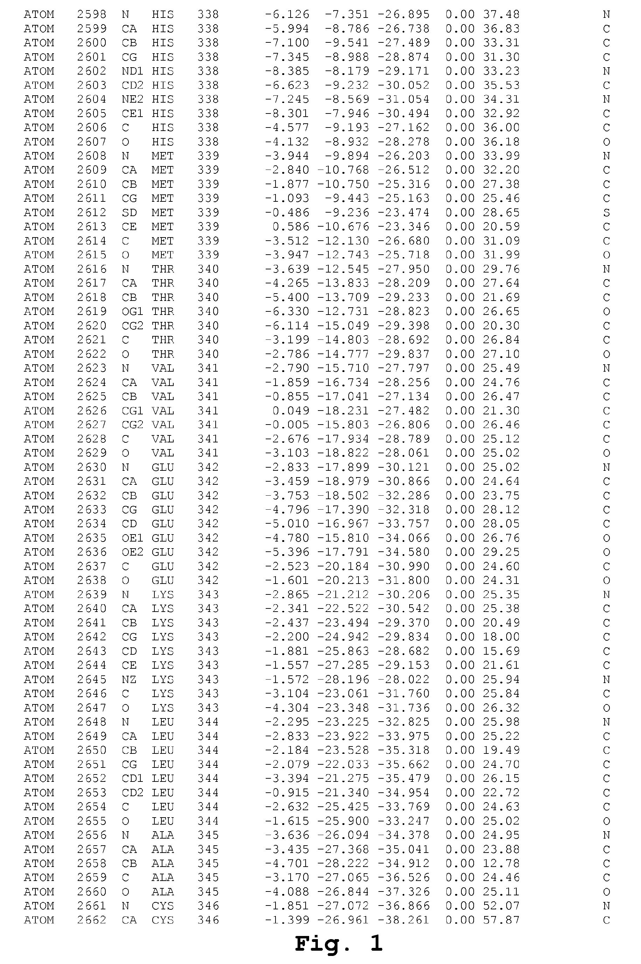

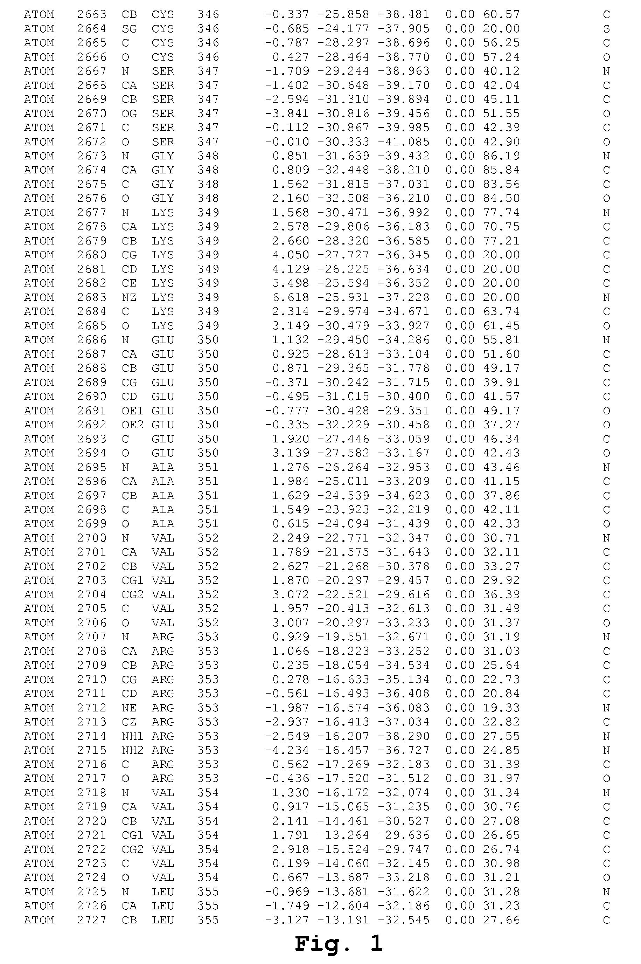

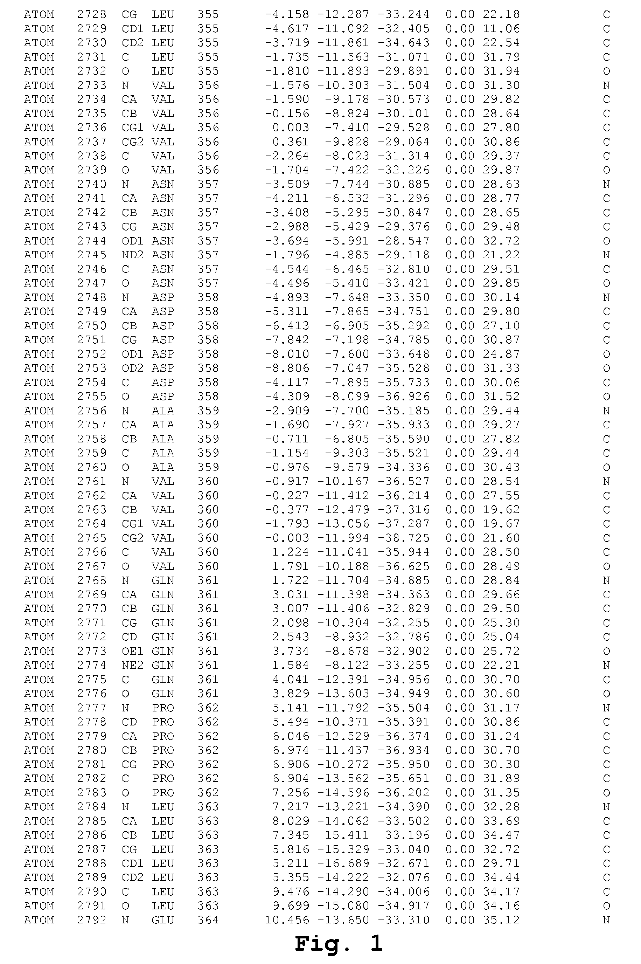

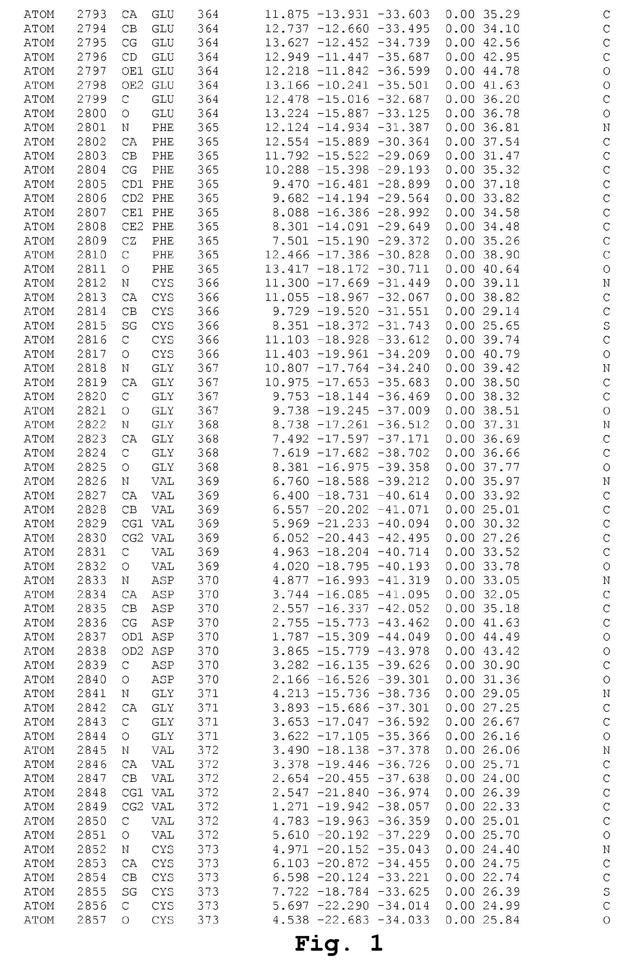

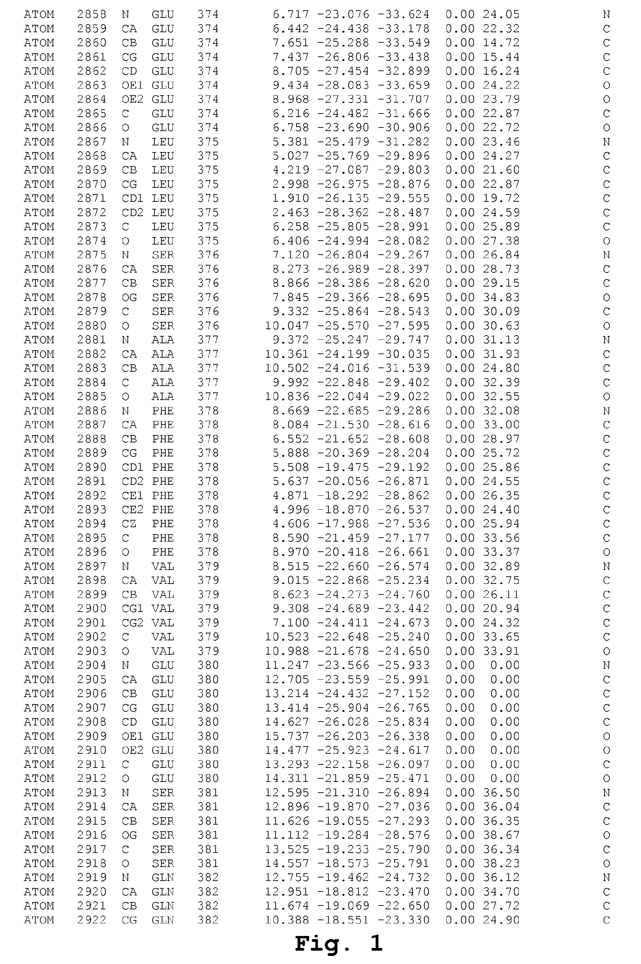

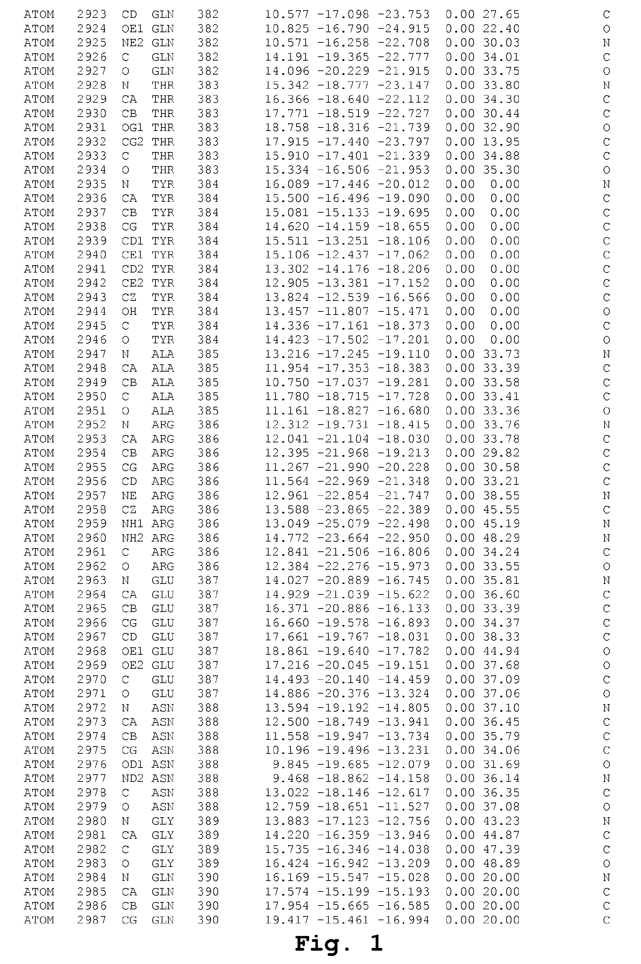

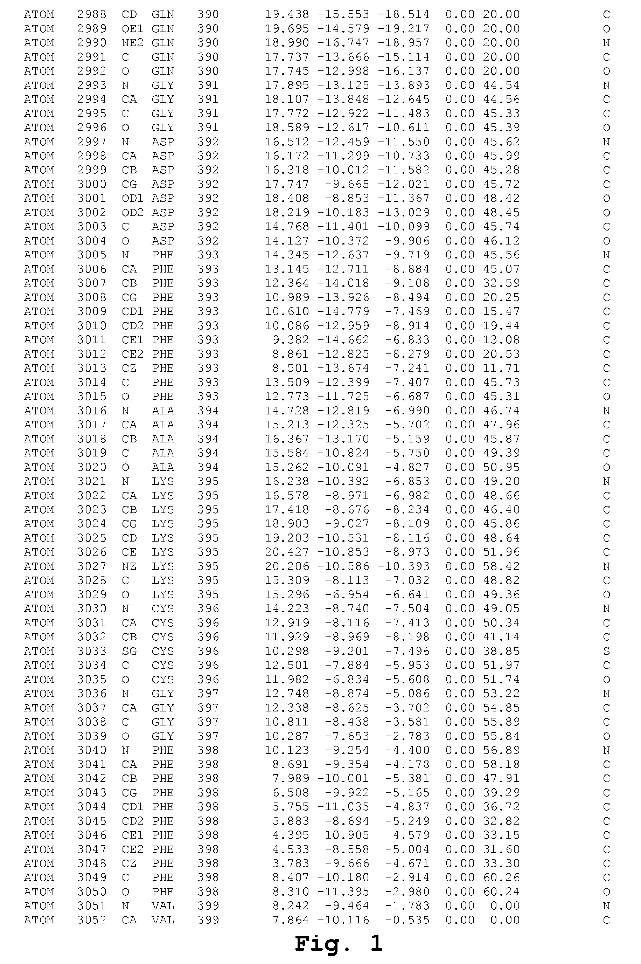

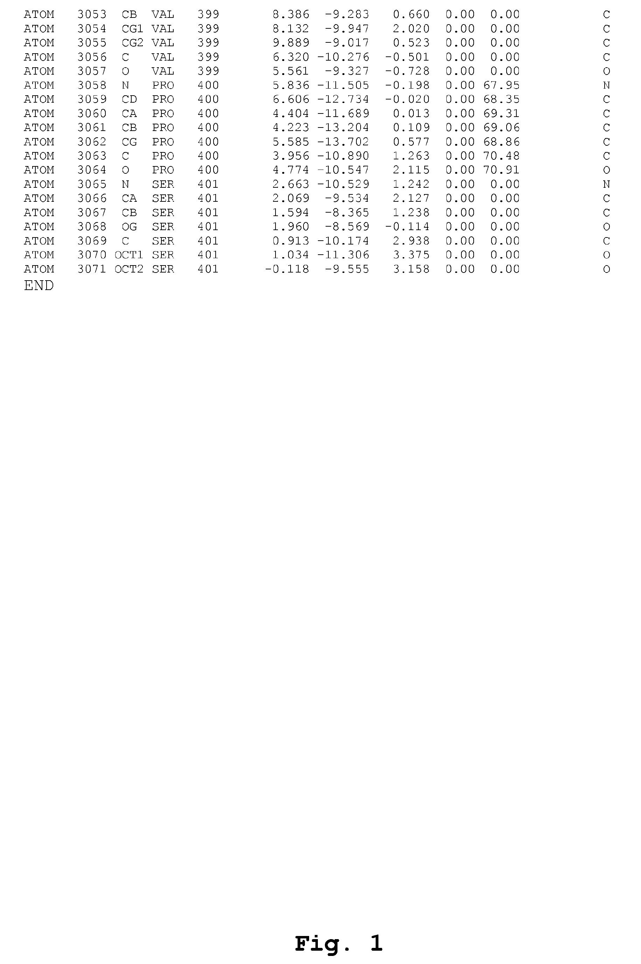

Figure 1 provides the coordinates for the 3D structure of a phytase from *Peniophora lycii* that comprises the amino acid sequence of SEQ ID NO: 2.

### Detailed Description of the Invention

### Three-dimensional Structure of the Peniophora Phytase

The structure of the phytase of *Peniophora lycii* was solved in accordance with the principles for X-ray crystallographic methods as given, for example, in X-Ray Structure Determination, Stout, G.K. and Jensen, L.H., John Wiley & Sons, Inc. NY, 1989. The structural coordinates for the crystal structure at 2.2 A resolution using the isomorphous replacement method are given in Fig. 1 in standard PDB format (Protein Data Bank, Brookhaven National Laboratory, Brookhaven, CT). The PDB file of Fig. 1 relates to a part only of the sequence of this phytase, viz. the part corresponding to residues 22-422 of SEQ ID NO: 2.

### Molecular Dynamics (MD)

Molecular Dynamics (MD) simulations are indicative of the mobility of the amino acids in a protein structure (see McCammon, JA and Harvey, SC., (1987), "Dynamics of proteins and nucleic acids", Cambridge University Press). Such protein dynamics are often compared to the crystallographic B-factors (see Stout, GH and Jensen, LH, (1989), "X-ray structure determination", Wiley). By running the MD simulation at, e.g., different temperatures, the temperature related mobility of residues is simulated. Regions having the highest mobility or flexibility (here isotropic fluctuations) may be suggested for random mutagenesis. It is here understood that the high mobility found in certain areas of the protein, may be thermally improved by substituting these residues.

Using the program CHARMM (Molecular Simulations (MSI)), the *Peniophora* phytase structure described above was subjected to MD at 300, 350, 400, 450, and 500K for 300 and 600ps and then minimized using 300 steps Steepest Descent, 600 steps Conjugated Gradients, and 3000 steps ABNR with a tolerance grade of 0.001 kcal/mole.

The following suggested regions for mutagenesis result from MD simulations:
MD at 300 and 400K without water: 37-48, 91-119, 232-238, 300-308, 325-335, 343-348;
MD at 300 and 400K with water: 407-412;
MD at 300K without water: 229-236;
MD in a cubic water box with period boundaries: 123-126, 154-157, 204-216, 284-289, 419-423.

The following suggested regions for mutagenesis result from evaluations of the B-factors in the X-structure: 73-83, 207-217.

The above regions may be combined as follows: 37-48; 73-83; 91-119; 123-126; 154-157; 204-217; 229-238; 284-289; 300-308; 325-335; 343-348; 407-412; 419-423.

Other regions resulting from MD simulations are: 154-163; 204-218; 323-335; 342-350.

Other regions resulting from evaluation of B-factors are: 1-29, preferably 1-28; 59-106; 169-171; 178-247; 339-344; 367-371; 417 onwards. More preferred regions are: 1-24; 69-94; 182-188; 199-220; 229-241; 368-370; 420-423.

### Active Site and Substrate Binding Shells

Programs known in the art, such as INSIGHTII from Molecular Simulations MSI, San Diego, California can be used to define active site shells comprising those amino acid residues which are in close proximity to the catalytic residues (H55, D319, R54). Substrate binding shells can also be defined, comprising those amino acid residues which are in close proximity to the substrate binding site (H55, R54, R139, N320) and which can therefore be expected to be in contact with the substrate. Close proximity may be defined as, e.g., 0Å, 15Å, 16Å, or 20Å, from the relevant residues. Information about substrate binding can be deduced by docking phytic acid or a derivative thereof such as inositol-1,4,5-triphosphate (Brookhaven database file 1djx. Inositol-1,4,5-triphosphate) into the active site cleft (the residues making up the surface of the active site.

### Strategy for Preparing Variants

Based on the 3D-structure of Fig. 1 and SEQ ID NO: 2, and following study of active site and substrate binding shells (16Å, preferably 15Å, more preferably 10Å), as well as molecular dynamics, the following regions were suggested for mutagenesis, mainly with a view to improving thermostability and/or increasing specific activity: Amino acids 26-31; 55-69; 99-104; 135-142; 169-193; 253-266; or amino acids 321-323 of SEQ ID NO: 2.

The following positions of the above regions of SEQ ID NO: 2 are preferably subjected to mutagenesis: G26, P27, Y28, D29, P30, W59, P60, T61, S62, G63, A64, R65, S66, R67, Q68, V69, A99, D100, L102, P103, F104, A135, G136, D137, Q138, V141, D142, E169, E170, N177, N178, M179, P181, N182, D189, T191, W192, L193, Y253, Y254, D257, K258, Y259, Y260, T262, G265, N266, T321, and/or V323.

The following variants are contemplated: G26S,A; P27X,M; Y28F,N,Q; D29S,T,A; F30X; W59X,F,Y; P60S; T61S; S62A; G63A,S; A64G,K,R,S; R65A; S66K,T; R67X,K,A,L,V,S; Q68E,D,N,M; V69X; A99X,D,S,E,N; D100X,S,E,N; L102V; P103X,E; F104X,L; A135S,D; G136X,S,D; D137S; Q138X,D,N; V141X; D142X; E169A; E170X,A,T; N177D; N178A,G,L; M179V,T; P181X,T,V; N182X,A; D189X; T191X; W192X; L193T,I; Y253N,G; Y254A,G; D257S; K258L; Y259X,F,P,W; Y260X,P,W; T262Y,V; G265X; N266Q; T3210,L,N; and/or V3231.

Amino acid residues or positions mainly pointing towards the substrate are the following: P27, Y28, R65, R67, Q68, A99, D100, L102, A135, G136, D137, E169, N177, T191, W192, Y253, Y254, T262, and T321. Variants being altered in one or more of these positions are specifically contemplated, e.g. variants comprising an alteration in at least one, at least two, at least three, at least four, at least five, at least six, at least seven, at least eight, at least nine, or at least ten of these positions. In a particular embodiment, the variant comprises alterations in at least eleven, at least twelve, at least thirteen, at least fourteen, at least fifteen, at least sixteen, at least seventeen, at least eighteen, or in all neighteen positions. Examples of such variants are those comprising at least one, two, three, four, five, six, seven, eight, nine, or at least ten mutations selected from the following: P27X,M; Y28F,N,Q; R65A; R67X,K,A,L,V,S; Q68E,D,N,M; A99X,D,S,E,N; D100X,S,E,N; L102V; A135S,D; G136X,S,D; D137S; E169A; N177D; T191X; W192X; Y253N,G; Y254A,G; T262Y,V; and/or T321Q,L,N. In a particular embodiment, the variant comprises at least eleven, twelve, thirteen, fourteen, fifteen, sixteen, seventeen, at least eighteen, or nineteen of these mutations.

### Polypeptides Having Phytase Activity

In the present context a polypeptide having phytase activity (a phytase) is an enzyme which catalyzes the hydrolysis of phytate (myo-inositol hexakisphosphate) to (1) myo-inositol and/or (2) mono-, di-, tri-, tetra- and/or penta-phosphates thereof and (3) inorganic phosphate.

In the present context the term a phytase substrate encompasses, i.a., phytic acid and any phytate (salt of phytic acid), as well as the phosphates listed under (2) above.

The ENZYME site at the internet (http://www,expasy,ch/enzyme/) is a repository of information relative to the nomenclature of enzymes. It is primarily based on the recommendations of the Nomenclature Committee of the International Union of Biochemistry and Molecular Biology (IUB-MB) and it describes each type of characterized enzyme for which an EC (Enzyme Commission) number has been provided (Bairoch A. The ENZYME database, 2000, Nucleic Acids Res 28:304-305). See also the handbook Enzyme Nomenclature from NC-IUBMB, 1992).

According to the ENZYME site, two different types of phytases are known: A so-called 3-phytase (myo-inositol hexaphosphate 3-phosphohydrolase, EC 3.1.3.8) and a so-called 6-phytase (myo-inositol hexaphosphate 6-phosphohydrolase, EC 3.1.3.26). For the purposes of the present invention, both types are included in the definition of phytase.

For the purposes of the present invention the phytase activity is determined in the unit of FYT, one FYT being the amount of enzyme that liberates 1 micro-mol inorganic ortho-phosphate per min. under the following conditions: pH 5.5; temperature 37°C; substrate: sodium phytate (C₆H₆0₂₄P₆Na₁₂) in a concentration of 0.0050 mol/I. Suitable phytase assays are the FYT and FTU assays described in Example 1 of WO 00/20569. FTU is for determining phytase activity in feed and premix. The FYT assay is also described in Example 2 herein.

### Parent Phytase

A parent phytase is a phytase which is homologous to the phytase derived from *Peniophora lycii* CBS 686.96. In the present context, homologous means having an identity of at least 60% to SEQ ID NO: 2 herein, corresponding to amino acids 17-439 of the complete amino acid sequence, including the signal peptide part, of a phytase derived from *Peniophora lycii* CBS 686.96 (SEQ ID NO: 2 of WO 98/28408). Homology is determined as generally described below in the section entitled Amino Acid Homology.

The parent phytase may be a wild-type or naturally occurring polypeptide, or an allelic variant thereof, or a fragment thereof that has phytase acticity, in particular a mature part thereof. It may also be a variant thereof and/or a genetically engineered or synthetic polypeptide.

In a particular embodiment the wild-type parent phytase is a fungal phytase, such as a filamentous fungal phytase, or a yeast phytase. An example of a yeast phytase is the *Schwanniomyces occidentalis* phytase described in US 5830732.

In another particular embodiment the wild-type fungal parent phytase is derived from a filamentous fungus, e.g. of the phylum *Ascomycota* or the phylum *Basidiomycota* (an ascomycete phytase, or a basidiomycete phytase, respectively).

Examples of ascomycete phytases are those derived from a strain of *Aspergillus,* for example *Aspergillus awamori* PHYA (SWISSPROT P34753, Gene 133:55-62 (1993)), *Aspergillus niger (ficuum)* PHYA (SWISSPROT P34752, EP 420358, Gene 127:87-94 (1993)), *Aspergillus awamori* PHYB (SWISSPROT P34755, Gene 133:55-62 (1993)), *Aspergillus niger* PHYB (SWISSPROT P34754, Biochem. Biophys. Res. Commun. 195:53-57(1993)); or a strain of *Emericella,* for example *Emericella nidulans* PHYB (SWISSPROT 000093, Biochim. Biophys. Acta 1353:217-223 (1997)); or a strain of *Thermomyces (Humicola),* for example the *Thermomyces lanuginosus* phytase described in WO 97/35017. Other examples of ascomycete phytases are disclosed in EP 684313 (for example derived from strains of *Aspergillus fumigatus, Aspergillus terreus,* and *Myceliophthora thermophila);* JP 11000164 (a phytase derived from a strain of *Penicillium.);* US 6139902 (a phytase derived from a strain of *Aspergillus),* and WO 98/13480 *(Monascus anka* phytase).

Examples of basidiomycete phytases are the phytases derived from *Paxillus involutus, Trametes pubescens, Agrocybe pediades* and *Peniophora lycii* (see WO 98/28409).

A preferred parent phytase is derived from a species of *Peniophora,* preferably from a strain of *Peniophora lycii,* most preferably from the strain *Peniophora lycii* CBS 686.96.

It will be understood that the definition of the aforementioned species includes both the perfect and imperfect states, and other taxonomic equivalents e.g., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

Furthermore, such polypeptides may be identified and obtained from other sources including microorganisms isolated from nature (e.g., soil, composts, water, etc.) using suitable probes. Techniques for isolating microorganisms from natural habitats are well known in the art. The nucleic acid sequence may then be derived by similarly screening a genomic or cDNA library of another microorganism. Once a nucleic acid sequence encoding a polypeptide has been detected with the probe(s), the sequence may be isolated or cloned by utilizing techniques which are known to those of ordinary skill in the art (see, e.g., Sambrook *et al.,* 1989, *supra).*

In still another particular embodiment the parent phytase is a synthetic phytase. A synthetic phytase is designed by man, and expectedly does not occur in nature. EP 897985 and WO 00/43503 disclose examples of such synthetic, fungal phytases generally designated consensus phytases. Shuffled phytases are other examples of a synthetic or genetically engineered parent phytase, which can be prepared as is generally known in the art, eg by Site-directed Mutagenesis, by PCR (using a PCR fragment containing the desired mutation as one of the primers in the PCR reactions), or by Random Mutagenesis. Included in the concept of a synthetic phytase is also any hybrid or chimeric phytase, i.e. a phytase which comprises a combination of partial amino acid sequences derived from at least two phytases.

The parent phytase may comprise the amino acid sequence specified, or it may be an allelic variant thereof; or a fragment thereof that has phytase activity. In one embodiment, the parent phytase comprises the amino acid sequence specified or an allelic variant thereof; or a fragment thereof that has phytase activity. In another embodiment, the parent phytase consists of the amino acid sequence specified, or an allelic variant thereof; or a fragment thereof that has the phytase activity.

A fragment of a specified amino acid sequence is a polypeptide having one or more amino acids deleted from the amino and/or carboxyl terminus of this amino acid sequence. In one embodiment, a fragment contains at least 150, or at least 200, or at least 250, or at least 260, or at least 280, or at least 300, or at least 320, or at least 340, or at least 360, or at least 380, or at least 390, or at least 400, or at least 410, or at least 420, or at least 430, or at least 440, or at least 450 amino acid residues.

An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

The parent phytase may also be a mature part of any of the amino acid sequences referred to above. A mature part means a mature amino acid sequence and refers to that part of an amino acid sequence which remains after a potential signal peptide part has been cleaved off.

In still another embodiment, the parent phytase may be a variant of the phytases referred to above comprising a substitution, deletion, and/or insertion of one or more amino acids. The amino acid sequence of the variant phytase may differ from the amino acid sequence specified by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter the specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

### Microorganism Taxonomy

Questions relating to taxonomy may be solved by consulting a taxonomy data base, such as the NCBI Taxonomy Browser which is available at the following internet site: http://wvw.ncbi.nlm.nih.gov/Taxonomy/taxonomyhom.html/, and/or by consulting Taxonomy handbooks. For the present purposes, a preferred handbook in relation to fungal taxonomy is Dictionary of the Fungi, 9th edition, edited by Kirk, P.M., P.F. Cannon, J.C. David & J.A. Stalpers, CAB Publishing, 2001.

A synthetic phytase, such as a consensus phytase, a shuffled phytase, or a hybrid phytase, may comprise a combination of partial amino acid sequences deriving from at least two ascomycete phytases, at least two basidiomycete phytases or from at least one ascomycete and at least one basidiomycete phytase. These ascomycete and basidiomycete phytases from which a partial amino acid sequence derives may, e.g., be any of those specific phytases referred to herein.

In the present context, a synthethic phytase derived from at least one fungus is a fungal phytase. Furthermore, a synthethic phytase derived from ascomycete phytases only is an ascomycete phytase; and a synthethic phytase derived from basidiomycete phytases only is a basidiomycete phytase. Any synthethic phytase derived from at least one ascomycete phytase as well as at least one basidiomycete phytase may be designated a mixed ascomycete/basidiomycete phytase.

The designation *Peniophora* phytase means a wild-type phytase derived from a species of the genus *Peniophora,* including allelic variants, fragments or variants thereof, or a synthethic phytase prepared on the basis of at least one, preferably at least two, *Peniophora* phytase(s), more preferably on the basis only of *Peniophora* phytases. Examples of *Peniophora* species are: *Peniophora aurantiaca, P. cinerea, P. decorticans, P. duplex, P. ericsonii, P. incarnate, P. lycii, P. meridionalis, P. nuda, P. piceae, P. pini, P. pithya, P. polygonia, P. proxima, P. pseudo-pini, P. rufa, P. versicolor,* and species simply classified as *Peniophora sp.* A preferred species is *Peniophora lycii.* A preferred strain is *Peniophora lycii* CBS 686.96.

### Amino Acid Homology

The present invention refers to phytases having an amino acid sequence which has a certain degree of identity to SEQ ID NO: 2 (hereinafter "homologous phytases").

For purposes of the present invention the degree of identity between two amino acid sequences, as well as the degree of identity between two nucleotide sequences, is determined by the program "align" which is a Needleman-Wunsch alignment (i.e. a global alignment). The program is used for alignment of polypeptide, as well as nucleotide sequences. The default scoring matrix BLOSUM50 is used for polypeptide alignments, and the default identity matrix is used for nucleotide alignments. The penalty for the first residue of a gap is -12 for polypeptides and -16 for nucleotides. The penalties for further residues of a gap are -2 for polypeptides, and -4 for nucleotides.

"Align" is part of the FASTA package version v20u6 (see W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA," Methods in Enzymology 183:63-98). FASTA protein alignments use the Smith-Waterman algorithm with no limitation on gap size (see "Smith-Waterman algorithm", T. F. Smith and M. S. Waterman (1981) J. Mol. Biol. 147:195-197).

In particular embodiments, the parent phytase has an amino acid sequence which has a degree of identity to SEQ ID NO: 2 of at least about 55%, or of at least about 60%, or of at least about 65%, or of at least about 70%, or of at least about 75% or of at least about 80%, or of at least about 85%, or of at least about 90%, or of at least about 91 %, or of at least about 92%, or of at least about 93%, or of at least about 94%, or of at least about 95%, or of at least about 96%, or of at least about 97%, or of at least about 98%, or of at least about 99%.

In another particular embodiment, these homologous parent phytases have an amino acid sequence which differs by thirty, twentyfive, twentytwo, twentyone, twenty, nineteen, eighteen, seventeen, sixteen, fifteen, fourteen, thirteen, twelve, eleven, ten, nine, eight, seven, six, five, four, three, two or only one amino acid(s) from the specified amino acid sequence.

Also the resulting variant phytase is homologous to SEQ ID NO: 2. In particular embodiments, the variant phytase comprises or has an amino acid sequence which has a degree of identity to SEQ ID NO: 2 of at least about 55%, or of at least about 60%, or of at least about 65%, or of at least about 70%, or of at least about 71%, or of at least about 72%, or of at least about 73%, or of at least about 74%, or of at least about 75%, or of at least about 76%, or of at least about 77%, or of at least about 78%, or of at least about 79%, or of at least about 80%, or of at least about 82%, or of at least about 85%, or of at least about 87%, or of at least about 90%, or of at least about 92%, or of at least about 95%, or of at least about 97%.

### Nucleic Acid Hybridization

In the alternative, homologous parent phytases, as well as variant phytases, may be defined as being encoded by a nucleic acid sequence which hybridizes under very low stringency conditions, preferably low stringency conditions, more preferably medium stringency conditions, more preferably medium-high stringency conditions, even more preferably high stringency conditions, and most preferably very high stringency conditions with nucleotides 49-1320 of SEQ ID NO: 1, or a subsequence or a complementary strand thereof (J. Sambrook, E.F. Fritsch, and T. Maniatus, 1989, Molecular Cloning, A Laboratory Manual, 2d edition, Cold Spring Harbor, New York). A subsequence may be at least 100 nucleotides, or at least 200, 300, 400, 500, 600, 700, 800, 900, 1000, 1100, 1200, or at least 1300 nucleotides. Moreover, the subsequence may encode a polypeptide fragment that has the relevant enzyme activity.

For long probes of at least 100 nucleotides in length, very low to very high stringency conditions are defined as prehybridization and hybridization at 42°C in 5X SSPE, 0.3% SDS, 200 µg/ml sheared and denatured salmon sperm DNA, and either 25% formamide for very low and low stringencies, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For short probes which are about 15 nucleotides to about 70 nucleotides in length, stringency conditions are defined as prehybridization, hybridization, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCI pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to about 70 nucleotides in length, the carrier material is washed once in 6X SCC plus 0.1 % SDS for 15 minutes and twice each for 15 minutes using 6X SSC at 5°C to 10°C below the calculated Tₘ.

### Position numbering

The nomenclature used herein for defining amino acid positions is basically as described in WO 99/49022, i.e. with reference to the amino acid sequence of the phytase derived from *Peniophora lycii* CBS 686.96, except that in the present context the numbering starts at S17 of the sequence P_Iycii shown in Fig. 1 of WO 99/49022. Accordingly, the position numbers used herein differ from the position numbers of WO 99/49022 by 16 (see p. 14, line 26, and Fig. 1 of WO 99/49022). Thus, for example, S1 herein is equivalent to S17 of WO 99/49022, and F8 herein is equivalent to F24 of WO 99/49022.

Sequence SEQ ID NO: 2 comprises amino acids -14-409 of the sequence shown in Fig. 6 of WO 99/49022, corresponding to amino acids 16-439 of the P_Iycii sequence shown in Fig. 1 thereof.

Accordingly, in the present context, the basis for numbering positions is SEQ ID NO: 2 starting with S1 and ending with E423. A parent phytase, as well as a variant phytase, may comprise extensions as compared to SEQ ID NO: 2, i.e. in the N-terminal, and/or the C-terminal ends thereof. The amino acids of such extensions, if any, are to be numbered as is usual in the art, i.e. for a C-terminal extension: 424, 425, 426 and so forth, and for an N-terminal extension -1, -2, -3 and so forth.

### Alterations, such as Substitutions, Deletions, Insertions

In the present context, the following are examples of various ways in which a phytase variant - at least in theory, i.e. on paper - can be derived from a parent amino acid sequence: An amino acid can be substituted with another amino acid; an amino acid can be deleted; an amino acid can be inserted; as well as any combination of any number of such alterations.

For the present purposes, the term substitution is intended to include any number of any type of such alterations. This is a reasonable definition, because, for example, a deletion can be regarded as a substitution of an amino acid, AA, in a given position, nn, with nothing, (). Such substitution can be designated: AAnn(). Likewise, an insertion of only one amino acid, BB, downstream an amino acid, AA, in a given position, nn, can be designated: ()nnaBB. And if two amino acids, BB and CC, are inserted downstream of amino acid AA in position nn, this substitution (combination of two substitutions) can be designated: ()nnaBB+()nnbCC, the thus created gaps between amino acids nn and nn+1 in the parent sequence being assigned lower case or subscript letters a, b, c etc. to the former position number, here nn. A comma (,) between substituents, as e.g. in the substitution A327S,F,I,L means "either or", i.e. that A327 is substituted with S, or F, or I, or L. A plus-sign (+) between substitutions, as e.g. in the substitutions 29S+125D+324A means "and", i.e. that these three single substitutions are combined in one and the same phytase variant. This nomenclature defining amino acid alterations is also basically as described in WO 99/49022 (see p. 16 thereof).

In the alternative, in which the term substitution is defined differently, the present invention relates to a variant of a parent phytase, comprising an alteration in at least one position selected from the various groups of regions indicated herein, wherein the alteration(s) are independently an insertion of at least one amino acid downstream of the amino acid which occupies the position; a deletion of the amino acid which occupies the position; and/or a substitution of the amino acid which occupies the position with a different amino acid; wherein the variant has phytase activity, and each position corresponds to a position of the amino acid sequence of the enzyme having the amino acid sequence of SEQ ID NO: 2, and the variant has a percentage of identity to SEQ ID NO: 2 of at least 75%; and with proviso(s) (d) as defined herein.

In the present context, the term "a substitution" means at least one substitution. At least one means one or more, e.g. one, or two, or three, or four, or five, or six, or seven, or eight, or nine, or ten, or twelve, or fourteen, or fifteen, or sixteen, or eighteen, or twenty, or twentytwo or twentyfour, or twentyfive, or twenty eight, or thirty, and so on, to include in principle, any number of substitutions. The variants of the invention, however, still have to be, e.g., at least 75% identical to SEQ ID NO: 2, this percentage being determined by the above-mentioned program. The substitutions can be applied to any position encompassed by any region mentioned in claim 1, and variants comprising combinations of any number and type of such substitutions are also included. The term substitution as used herein also include deletions, as well as extensions, or insertions, that may add to the length of the sequence corresponding to SEQ ID NO: 2. In a particular embodiment, the number of substitutions is at most 200, or 175, or 150, or 125, or 120, or 110, or 106, or 105, or 100, or 90, or 80, or 70, or 60, or 50, or 40, or 30, or 25.

Furthermore, the term "a substitution" embraces a substitution into any one of the other nineteen natural amino acids, or into other amino acids, such as non-natural amino acids. For example, a substitution of amino acid Q in position 20 includes each of the following substitutions: 20A, 20C, 20D, 20E, 20F, 20G, 20H, 201, 20K, 20L, 20M, 20N, 20P, 20R, 20S, 20T, 20V, 20W, or 20Y. This is, by the way, equivalent to the designation 20X, wherein X designates any amino acid. These substitutions can also be designated Q20A, Q20C, Q20X, etc. The same applies by analogy to each and every position mentioned herein, to specifically include herein any one of such substitutions.

### Identifying Corresponding Position Numbers

For the parent phytase of *Peniophora lycii* CBS 686.96, the position numbering refers to the numbering of SEQ ID NO: 2. For a given position in another phytase - be it a parent or a variant phytase - a corresponding position in SEQ ID NO: 2 can always be found. Or - in the alternative - a position which corresponds to a position of the amino acid sequence of the phytase comprising (or having) the amino acid sequence of SEQ ID NO: 2, can always be found.

The amino acid sequence of a parent phytase other than the phytase of *Peniophora lycii* CBS 686.96, or, in turn, of a variant phytase amino acid sequence, is designated SEQ-X. A position corresponding to position N of SEQ ID NO: 2 is found as follows: The parent or variant phytase amino acid sequence SEQ-X is aligned with SEQ ID NO: 2 as specified above in the section entitled Amino Acid Homology. From the alignment, the position in sequence SEQ-X corresponding to position N of SEQ ID NO: 2 can be clearly and unambiguously derived, using the principles described below.

Reference is made, as an example only, to Fig. 1 of WO 99/49022, assuming for the sake of simplicity that the sequences A_pediades, A_fumigatus or T_lanuginosus are e.g. variant phytases which have been aligned to the phytase P_Iycii, which we assume corresponds to SEQ ID NO: 2 as described herein (for the present purposes they should have been aligned to SEQ ID NO: 2):
Question no. 1: Which position in A_pediades corresponds to S17 of P_lycii (said position being, for the present purposes, designated S1)? The answer is: A15.
Question no. 2: Which position in the phytase designated A_fumigatus corresponds to 55b() of P_Iycii (said position being, for the present purposes, designated 39b())? The answer is: S63.
Question no. 3: Which position in A_pediades corresponds to L31 of P_Iycii (said position being, for the present purposes, designated L15)? The answer is: ()30d.
Question no. 4: Which substitution in P_Iycii corresponds to an, imaginative, T_lanuginosa phytase variant having the substitution G3L? The answer is: ()-6L (said substitution being, for the present purposes, designated ()-22L).

SEQ-X may be a mature part of the phytase in question, or it may also include a signal peptide part, or it may be a fragment of the mature phytase which has phytase activity, e.g. a fragment of the same length as SEQ ID NO: 2, or the fragment which extends from S1 to E423 when aligned with SEQ ID NO: 2 as described herein.

### Region and Position

In the present context, the term region means at least one position of a parent phytase amino acid sequence, the term position designating an amino acid residue of such amino acid sequence. In one embodiment, region means one or more successive positions of the parent phytase amino acid sequence, e.g. one, two, three, four, five, six, seven, eight, etc., up to any number of consecutive positions of the sequence. Accordingly, a region may consist of one position only, or it may consist of any number of consecutive positions, such as, e.g., position no. 37, 38 and 39; or position no. 37, 38, 39, 40, and 41; or position no. 37, 38, 39, 40, 41, 42, 43, 44, 45, 46, 47 and 48. For the present purposes, the latter three regions are designated 37-39, 37-41, and 37-48, respectively. The boundaries of these regions or ranges are included in the region. SEQ ID NO: 2 is one example of a preferred parent phytase amino acid sequence.

A region encompasses specifically each and every position it embraces. For example, region 37-48 specifically encompasses each of the positions 37, 38, 39, 40, 41, 42, 43, 44, 45, 46, 47, and 48. The same applies by analogy for the other regions mentioned herein.

### Thermostability

For the present purposes, the term thermostable as applied in the context of a certain polypeptide, refers to the melting temperature, Tm, of such polypeptide, as determined using Differential Scanning Calorimetry (DSC) at a pH of 5.5: For a thermostable polypeptide, the Tm is at least 61 °C. In particular embodiments, the Tm is at least 62, 63, 64, 65, 66, 67, 68, 69, 70, 71, 72, 73, 74, 75, 76, 77, 77.5, 78, 79, 80, 81, 82, 83, 84, 85, 86, 87, 88, 89, 90, 91, 92, 93, 94, 95, 96, 97, 98, 99 or 100°C.

In the alternative, the term thermostable refers to a melting temperature of at least 49°C, preferably at least 50, 51, 52, 53, 54, 55, 56, 57, 58, 59, 60, 61, 62, 63, 64, 65, 66, 67, 68, 69, 70, 71, 72, 73, 74, 75, 76, 77, 77.5, 78, 79, 80, 81, 82, 83, 84, 85, 86, 87, 88, 89, or 90°C, as determined using DSC at a pH of 7.0.

In another alternative, the term thermostable refers to a melting temperature of at least 51 °C, preferably at least 52, 53, 54, 55, 56, 57, 58, 59, 60, 61, 62, 63, 64, 65, 66, 67, 68, 69, 70, 71, 72, 73, 74, 75, 76, 77, 77.5, 78, 79, 80, 81, 82, 83, 84, 85, 86, 87, 88, 89, or 90°C, as determined using DSC at a pH of 3.0.

In a still further alternative, the term thermostable refers to a melting temperature of at least 37°C, preferably at least 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 77.5, 78, 79, 80, 81, 82, 83, 84, 85, 86, 87, 88, 89, or 90°C, as determined using DSC at a pH of 2.5.

For the determination of Tm, a sample of the polypeptide with a purity of at least 90% (or 91, 92, 93, 94, 95, 96, 97, or 98%) as determined by SDS-PAGE may be used. Still further, the enzyme sample may have a concentration of between 0.5 and 2.5 mg/ml protein (or between 0.6 and 2.4, or between 0.7 and 2.2, or between 0.8 and 2.0 mg/ml protein), as determined from absorbance at 280 nm and based on an extinction coefficient calculated from the amino acid sequence of the enzyme in question.

The DSC takes place at the desired pH (e.g. pH 5.5, 7.0, 3.0, or 2.5) and with a constant heating rate, e.g. of 1, 1.5, 2, 3, 4, 5, 6, 7, 8, 9 or 10°C/min. Examples of suitable buffers are mentioned in the Experimental Part. Examples of preferred heating rates are 1.0, 1.5 or 2.0°C/min when using the equipment as described in Example 2 herein. For other types of equipment with smaller sample volumes a reliable estimate of Tm may be obtained using a heating rate of, e.g., 3, 4, 5, 6, 7, 8, 9 or even 10°C /min.

In a particular embodiment, the phytase variant of the invention is more thermostable than the parent phytase. A preferred parent phytase for this purpose is the *Peniophora lycii* CBS 686.96 phytase.

### Specific Activity

The term high, increased or improved specific activity refers to a specific activity of at least 105%, relative to the specific activity of the parent phytase as determined by the same procedure. In particular embodiments, the relative specific activity is at least 110, 115, 120, 125, 130, 140, 145, 150, 160, 170, 180, 190, 200, 220, 240, 260, 280, 300, 350 or even 400%, still relative to the specific activity of the parent phytase as determined by the same procedure.

In the alternative, the term high specific activity refers to a specific activity of at least 200 FYT/mg Enzyme Protein (EP). In particular embodiments, the specific activity is at least 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900 or 3000 FYT/mg EP.

Specific activity is measured on highly purified samples (an SDS poly acryl amide gel should show the presence of only one component). The enzyme protein concentration may be determined by amino acid analysis, and the phytase activity in the units of FYT. Specific activity is a characteristic of the specific phytase variant in question, and it is calculated as the phytase activity measured in FYT units per mg phytase variant enzyme protein.

See Example 3 for further details.

The phytase variant of the invention is thermostable and/or of a high specific activity. In a particular embodiment it is thermostable. In another particular embodiment is is of high specific activity. In a third particular embodiment it is thermostable, as well as of a high specific activity. In still further particular embodiments it is (a) more thermostable than the parent phytase; (b) of a higher specific activity as compared to the parent phytase; or (c) more thermostable than the parent phytase and of a higher specific activity as compared to the parent phytase.

### Low-allergenic Variants

In a specific embodiment, the phytase variants of the present invention are (also) low-allergenic variants, designed to invoke a reduced immunological response when exposed to animals, including man. The term immunological response is to be understood as any reaction by the immune system of an animal exposed to the phytase variant. One type of immunological response is an allergic response leading to increased levels of IgE in the exposed animal. Low-allergenic variants may be prepared using techniques known in the art. For example the phytase variant may be conjugated with polymer moieties shielding portions or epitopes of the phytase variant involved in an immunological response. Conjugation with polymers may involve in vitro chemical coupling of polymer to the phytase variant, e.g. as described in WO 96/17929, WO 98/30682, WO 98/35026, and/or WO 99/00489. Conjugation may in addition or alternatively thereto involve in vivo coupling of polymers to the phytase variant. Such conjugation may be achieved by genetic engineering of the nucleotide sequence encoding the phytase variant, inserting consensus sequences encoding additional glycosylation sites in the phytase variant and expressing the phytase variant in a host capable of glycosylating the phytase variant, see e.g. WO 00/26354. Another way of providing low-allergenic variants is genetic engineering of the nucleotide sequence encoding the phytase variant so as to cause the phytase variants to self-oligomerize, effecting that phytase variant monomers may shield the epitopes of other phytase variant monomers and thereby lowering the antigenicity of the oligomers. Such products and their preparation is described e.g. in WO 96/16177. Epitopes involved in an immunological response may be identified by various methods such as the phage display method described in WO 00/26230 and WO 01/83559, or the random approach described in EP 561907. Once an epitope has been identified, its amino acid sequence may be altered to produce altered immunological properties of the phytase variant by known gene manipulation techniques such as site directed mutagenesis (see e.g. WO 00/26230, WO 00/26354 and/or WO 00/22103) and/or conjugation of a polymer may be done in sufficient proximity to the epitope for the polymer to shield the epitope.

### Nucleic Acid Sequences and Constructs

The present invention also relates to nucleic acid sequences comprising a nucleic acid sequence which encodes a phytase variant of the invention.

The term "isolated nucleic acid sequence" refers to a nucleic acid sequence which is essentially free of other nucleic acid sequences, e.g., at least about 20% pure, preferably at least about 40% pure, more preferably at least about 60% pure, even more preferably at least about 80% pure, and most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

The nucleic acid sequences of the invention can be prepared by introducing at least one mutation into the parent phytase coding sequence or a subsequence thereof, wherein the mutant nucleic acid sequence encodes a variant phytase. The introduction of a mutation into the nucleic acid sequence to exchange one nucleotide for another nucleotide may be accomplished by site-directed mutagenesis using any of the methods known in the art, e.g. by site-directed mutagenesis, by random mutagenesis, or by doped, spiked, or localized random mutagenesis.

Random mutagenesis is suitably performed either as localized or region-specific random mutagenesis in at least three parts of the gene translating to the amino acid sequence shown in question, or within the whole gene. When the mutagenesis is performed by the use of an oligonucleotide, the oligonucleotide may be doped or spiked with the three non-parent nucleotides during the synthesis of the oligonucleotide at the positions which are to be changed. The doping or spiking may be performed so that codons for unwanted amino acids are avoided. The doped or spiked oligonucleotide can be incorporated into the DNA encoding the phytase enzyme by any technique, using, e.g., PCR, LCR or any DNA polymerase and ligase as deemed appropriate.

Preferably, the doping is carried out using "constant random doping", in which the percentage of wild-type and mutation in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% mutations in each position. An additional consideration in the choice of a doping scheme is based on genetic as well as protein-structural constraints.

The random mutagenesis may be advantageously localized to a part of the parent phytase in question. This may, e.g., be advantageous when certain regions of the enzyme have been identified to be of particular importance for a given property of the enzyme.

Alternative methods for providing variants of the invention include gene shuffling e.g. as described in WO 95/22625 or in WO 96/00343, and the consensus derivation process as described in EP 897985.

### Nucleic Acid Constructs

A nucleic acid construct comprises a nucleic acid sequence of the present invention operably linked to one or more control sequences which direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion.

### Expression vector

A nucleic acid sequence encoding a phytase variant of the invention can be expressed using an expression vector which typically includes control sequences encoding a promoter, operator, ribosome binding site, translation initiation signal, and, optionally, a repressor gene or various activator genes.

The recombinant expression vector carrying the DNA sequence encoding a glucoamylase variant of the invention may be any vector which may conveniently be subjected to recombinant DNA procedures, and the choice of vector will often depend on the host cell into which it is to be introduced. The vector may be one which, when introduced into a host cell, is integrated into the host cell genome and replicated together with the chromosome(s) into which it has been integrated.

The phytase variant may also be co-expressed together with at least one other enzyme of animal feed interest, such as a xylanase, an endoglucanase, an endo-1,3(4)-beta-glucanase, and/or a protease. The enzymes may be co-expressed from different vectors, from one vector, or using a mixture of both techniques. When using different vectors, the vectors may have different selectable markers, and different origins of replication. When using only one vector, the genes can be expressed from one or more promoters. If cloned under the regulation of one promoter (di- or multi-cistronic), the order in which the genes are cloned may affect the expression levels of the proteins. The phytase variant may also be expressed as a fusion protein, i.e. that the gene encoding the phytase variant has been fused in frame to the gene encoding another protein. This protein may be another enzyme or a functional domain from another enzyme.

### Host Cells

The present invention also relates to recombinant host cells, comprising a nucleic acid sequence of the invention, which are advantageously used in the recombinant production of the polypeptides. A vector comprising a nucleic acid sequence of the present invention is introduced into a host cell so that the vector is maintained as a chromosomal integrant or as a self-replicating extra-chromosomal vector. The term "host cell" encompasses any progeny of a parent cell that is not identical to the parent cell due to mutations that occur during replication. The choice of a host cell will to a large extent depend upon the gene encoding the polypeptide and its source.

The host cell may be a unicellular microorganism, e.g., a prokaryote, or a non-unicellular microorganism, e.g., a eukaryote cell, such as an animal, a mammalian, an insect, a plant, or a fungal cell. Preferred animal cells are non-human animal cells.

In a preferred embodiment, the host cell is a fungal cell, or a yeast cell, such as a *Candida, Hansenula, Kluyveromyces, Pichia, Saccharomyces, Schizosaccharomyces,* or *Yarrowia* cell. The fungal host cell may be a filamentous fungal cell, such as a cell of a species of, but not limited to, *Acremonium, Aspergillus, Fusarium, Humicola, Mucor, Myceliophthora, Neurospora, Penicillium, Thielavia, Tolypocladium,* or *Trichoderma.*

Useful unicellular cells are bacterial cells such as gram positive bacteria including, but not limited to, a *Bacillus* cell, or a *Streptomyces* cell, or cells of lactic acid bacteria; or gram negative bacteria such as E. *coli* and *Pseudomonas sp.* Lactic acid bacteria include, but are not limited to, species of the genera *Lactococcus, Lactobacillus, Leuconostoc, Streptococcus, Pediococcus,* and *Enterococcus.*

### Methods of Production

The present invention also relates to methods for producing a phytase of the present invention comprising (a) cultivating a host cell under conditions conducive for production of the phytase variant; and (b) recovering the phytase variant.

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the phytase is secreted into the nutrient medium, it can be recovered directly from the medium. If it is not secreted, it can be recovered from cell lysates.

The resulting phytase may be recovered by methods known in the art. For example, it be recovered from the nutrient medium by conventional procedures including, but not limited to, centrifugation, filtration, extraction, spray-drying, evaporation, or precipitation.

The phytases of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, *e.g.,* Protein Purification, J.-C. Janson and Lars Ryden, editors, VCH Publishers, New York, 1989).

### Plants

The present invention also relates to a transgenic plant, plant part, or plant cell which has been transformed with a nucleic acid sequence encoding a polypeptide having phytase activity of the present invention so as to express and produce the polypeptide in recoverable quantities. The polypeptide may be recovered from the plant or plant part. Alternatively, the plant or plant part containing the recombinant polypeptide may be used as such for improving the quality of a food or feed, e.g., improving nutritional value, palatability, and rheological properties, or to destroy an antinutritive factor.

In a particular embodiment, the polypeptide is targeted to the endosperm storage vacuoles in seeds. This can be obtained by synthesizing it as a precursor with a suitable signal peptide, see Horvath et al in PNAS, Feb. 15, 2000, vol. 97, no. 4, p. 1914-1919.

The transgenic plant can be dicotyledonous (a dicot) or monocotyledonous (a monocot) or engineered variants thereof. Examples of monocot plants are grasses, such as meadow grass (blue grass, Poa), forage grass such as festuca, lolium, temperate grass, such as Agrostis, and cereals, e.g., wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.* Low-phytate plants as described e.g. in US patent no. 5,689,054 and US patent no. 6,111,168 are examples of engineered plants.Examples of plant parts are stem, callus, leaves, root, fruits, seeds, and tubers. Also specific plant tissues, such as chloroplast, apoplast, mitochondria, vacuole, peroxisomes, and cytoplasm are considered to be a plant part. Furthermore, any plant cell, whatever the tissue origin, is considered to be a plant part.

Also included within the scope of the present invention are the progeny of such plants, plant parts and plant cells.

The transgenic plant or plant cell expressing a polypeptide of the present invention may be constructed in accordance with methods known in the art. Briefly, the plant or plant cell is constructed by incorporating one or more expression constructs encoding a polypeptide of the present invention into the plant host genome and propagating the resulting modified plant or plant cell into a transgenic plant or plant cell.

Conveniently, the expression construct is a nucleic acid construct which comprises a nucleic acid sequence encoding a polypeptide of the present invention operably linked with appropriate regulatory sequences required for expression of the nucleic acid sequence in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences is determined, for example, on the basis of when, where, and how the polypeptide is desired to be expressed. For instance, the expression of the gene encoding a polypeptide of the present invention may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression, the 35S-CaMV promoter may be used (Franck et al., 1980, Cell 21: 285-294). Organ-specific promoters may be, for example, a promoter from storage sink tissues such as seeds, potato tubers, and fruits (Edwards & Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant and Cell Physiology 39: 885-889), a *Vicia* faba promoter from the legumin B4 and the unknown seed protein gene from *Vicia* faba (Conrad et al., 1998, Journal of Plant Physiology 152: 708-711), a promoter from a seed oil body protein (Chen et al., 1998, Plant and Cell Physiology 39: 935-941), the storage protein napA promoter from *Brassica napus,* or any other seed specific promoter known in the art, e.g., as described in WO 91/14772. Furthermore, the promoter may be a leaf specific promoter such as the rbcs promoter from rice or tomato (Kyozuka et al., 1993, Plant Physiology 102: 991-1000, the chlorella virus adenine methyltransferase gene promoter (Mitra and Higgins, 1994, Plant Molecular Biology 26: 85-93), or the *aldP* gene promoter from rice (Kagaya et al., 1995, Molecular and General Genetics 248: 668-674), or a wound inducible promoter such as the potato pin2 promoter (Xu et al., 1993, Plant Molecular Biology 22: 573-588).

A promoter enhancer element may also be used to achieve higher expression of the enzyme in the plant. For instance, the promoter enhancer element may be an intron which is placed between the promoter and the nucleotide sequence encoding a polypeptide of the present invention. For instance, Xu *et al.,* 1993, *supra* disclose the use of the first intron of the rice actin 1 gene to enhance expression.

Still further, the codon usage may be optimized for the plant species in question to improve expression (see Horvath et al referred to above).

The selectable marker gene and any other parts of the expression construct may be chosen from those available in the art.

The nucleic acid construct is incorporated into the plant genome according to conventional techniques known in the art, including *Agrobacterium-mediated* transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

Presently, *Agrobacterium* tumefaciens-mediated gene transfer is the method of choice for generating transgenic dicots (for a review, see Hooykas and Schilperoort, 1992, Plant Molecular Biology 19: 15-38). However it can also be used for transforming monocots, although other transformation methods are generally preferred for these plants. Presently, the method of choice for generating transgenic monocots is particle bombardment (microscopic gold or tungsten particles coated with the transforming DNA) of embryonic calli or developing embryos (Christou, 1992, Plant Journal 2: 275-281; Shimamoto, 1994, Current Opinion Biotechnology 5: 158-162; Vasil et al., 1992, BiolTechnology 10: 667-674). An alternative method for transformation of monocots is based on protoplast transformation as described by Omirulleh et al., 1993, Plant Molecular Biology 21: 415-428.

Following transformation, the transformants having incorporated therein the expression construct are selected and regenerated into whole plants according to methods well-known in the art.

The present invention also relates to methods for producing a polypeptide of the present invention comprising (a) cultivating a transgenic plant or a plant cell comprising a nucleic acid sequence encoding a phytase variant of the present invention under conditions conducive for production of the phytase variant; and (b) recovering the phytase variant.

### Animals as Expression Hosts

The present invention also relates to a transgenic, non-human animal and products or elements thereof, examples of which are body fluids such as milk and blood, organs, flesh, and animal cells. Techniques for expressing proteins, e.g. in mammalian cells, are known in the art, see e.g. the handbook Protein Expression: A Practical Approach, Higgins and Hames (eds), Oxford University Press (1999), and the three other handbooks in this series relating to Gene Transcription, RNA processing, and Post-translational Processing. Generally speaking, to prepare a transgenic animal, selected cells of a selected animal are transformed with a nucleic acid sequence encoding a phytase variant of the present invention so as to express and produce the phytase variant. The phytase variant may be recovered from the animal, e.g. from the milk of female animals, or it may be expressed to the benefit of the animal itself, e.g. to assist the animal's digestion. Examples of animals are mentioned below in the section headed Animal Feed and Animal Feed Additives.

To produce a transgenic animal with a view to recovering the phytase variant from the milk of the animal, a gene encoding the phytase variant may be inserted into the fertilized eggs of an animal in question, e.g. by use of a transgene expression vector which comprises a suitable milk protein promoter, and the gene encoding the phytase variant. The transgene expression vector is microinjected into fertilized eggs, and preferably permanently integrated into the chromosome. Once the egg begins to grow and divide, the potential embryo is implanted into a surrogate mother, and animals carrying the transgene are identified. The resulting animal can then be multiplied by conventional breeding. The phytase variant may be purified from the animal's milk, see e.g. Meade, H.M. et al (1999): Expression of recombinant proteins in the milk of transgenic animals, Gene expression systems: Using nature for the art of expression. J. M. Fernandez and J. P. Hoeffler (eds.), Academic Press.

In the alternative, in order to produce a transgenic non-human animal that carries in the genome of its somatic and/or germ cells a nucleic acid sequence including a heterologous transgene construct including a transgene encoding the phytase variant, the transgene may be operably linked to a first regulatory sequence for salivary gland specific expression of the phytase variant, as disclosed in WO 2000064247.

### Animal Feed and Animal Feed Additives

For the present purposes, the term animal includes all animals, including human beings. In a particular embodiment, the phytase variants and compositions of the invention can be used as a feed additive for non-human animals. Examples of animals are non-ruminants, and ruminants, such as cows, sheep and horses. In a particular embodiment, the animal is a non-ruminant animal. Non-ruminant animals include mono-gastric animals, e.g. pigs or swine (including, but not limited to, piglets, growing pigs, and sows); poultry such as turkeys and chicken (including but not limited to broiler chicks, layers); young calves; and fish (including but not limited to salmon).

The term feed or feed composition means any compound, preparation, mixture, or composition suitable for, or intended for intake by an animal. The feed can be fed to the animal before, after, or simultaneously with the diet. The latter is preferred.

The composition of the invention, when intended for addition to animal feed, may be designated an animal feed additive. Such additive always comprises the phytase variant in question, preferably in the form of stabilized liquid or dry compositions. The additive may comprise other components or ingredients of animal feed. The so-called pre-mixes for animal feed are particular examples of such animal feed additives. Pre-mixes may contain the enzyme(s) in question, and in addition at least one vitamin and/or at least one mineral.

Accordingly, in a particular embodiment, in addition to the component polypeptides, the composition of the invention may comprise or contain at least one fat-soluble vitamin, and/or at least one water-soluble vitamin, and/or at least one trace mineral. Also at least one macro mineral may be included.

Examples of fat-soluble vitamins are vitamin A, vitamin D3, vitamin E, and vitamin K, e.g. vitamin K3.

Examples of water-soluble vitamins are vitamin B12, biotin and choline, vitamin B1, vitamin B2, vitamin B6, niacin, folic acid and panthothenate, e.g. Ca-D-panthothenate.

Examples of trace minerals are manganese, zinc, iron, copper, iodine, selenium, and cobalt.

Examples of macro minerals are calcium, phosphorus and sodium.

Further, optional, feed-additive ingredients are colouring agents, aroma compounds, stabilizers, additional enzymes, and antimicrobial peptides.

Additional enzyme components of the composition of the invention include at least one polypeptide having xylanase activity; and/or at least one polypeptide having endoglucanase activity; and/or at least one polypeptide having endo-1,3(4)-beta-glucanase activity; and/or at least one polypeptide having protease activity.

Xylanase activity can be measured using any assay, in which a substrate is employed, that includes 1,4-beta-D-xylosidic endo-linkages in xylans. Different types of substrates are available for the determination of xylanase activity e.g. Xylazyme cross-linked arabinoxylan tablets (from MegaZyme), or insoluble powder dispersions and solutions of azo-dyed arabinoxylan.

Endoglucanase activity can be determined using any endoglucanase assay known in the art. For example, various cellulose- or beta-glucan-containing substrates can be applied. An endoglucanase assay may use AZCL-Barley beta-Glucan, or preferably (1) AZCL-HE-Cellulose, or (2) Azo-CM-cellulose as a substrate. In both cases, the degradation of the substrate is followed spectrophotometrically at OD₅₉₅ (see the Megazyme method for AZCL-polysaccharides for the assay of endo-hydrolases at
http:www.megazyme.com/booklets/AZCLPOL.pdf .

Endo-1,3(4)-beta-glucanase activity can be determined using any endo-1,3(4)-beta-glucanase assay known in the art. A preferred substrate for endo-1,3(4)-beta-glucanase activity measurements is a cross-linked azo-coloured beta-glucan Barley substrate, wherein the measurements are based on spectrophotometric determination principles.

Protease activity can be measured using any assay, in which a substrate is employed, that includes peptide bonds relevant for the specificity of the protease in question. Examples of protease substrates are casein, and pNA-substrates, such as Suc-AAPF-pNA (available e.g. from Sigma S-7388). Another example is Protazyme AK (azurine dyed crosslinked casein prepared as tablets by Megazyme T-PRAK). Example 2 of WO 01/58276 describes suitable protease assays. A preferred assay is the Protazyme assay of Example 2D (the pH and temperature should be adjusted to the protease in question as generally described previously).

For assaying xylanase, endoglucanase, beta-1,3(4)-glucanase and protease activity the assay-pH and the assay-temperature are to be adapted to the enzyme in question (preferably a pH close to the optimum pH, and a temperature close to the optimum temperature). A preferred assay pH is in the range of 2-10, preferably 3-9, more preferably pH 3 or 4 or 5 or 6 or 7 or 8, for example pH 3 or pH 7. A preferred assay temperature is in the range of 20-80°C, preferably 30-80°C, more preferably 40-75°C, even more preferably 40-60°C, preferably 40 or 45 or 50°C. The enzyme activity is defined by reference to appropriate blinds, e.g. a buffer blind.

Examples of antimicrobial peptides (AMP's) are CAP18, Leucocin A, Tritrpticin, Protegrin-1, Thanatin, Lactoferrin, Lactoferricin, and Ovispirin such as Novispirin (Robert Lehrer, 2000), and variants or fragments thereof which retain antimicrobial activity. Other examples are anti-fungal polypeptides (AFP's) such as those derived from *Aspergillus giganteus,* and *Aspergillus niger,* as well as variants and fragments thereof which retain antifungal activity, as disclosed in WO 94/01459 and PCT/DK02/00289.

In a particular embodiment, the animal feed additive of the invention is intended for being included (or prescribed as having to be included) in animal diets or feed at levels of 0.0010-12.0%, or 0.0050-11.0%, or 0.0100-10.0%; more particularly 0.05-5.0%; or 0.2-1.0% (% meaning g additive per 100 g feed). This is so in particular for premixes.

Accordingly, the concentrations of the individual components of the animal feed additive, e.g. the premix, can be found by multiplying the final in-feed concentration of the same component by, respectively, 10-10000; 20-2000; or 100-500 (referring to the above three percentage inclusion intervals).

The final in-feed concentrations of important feed components may reflect the nutritional requirements of the animal, which are generally known by the skilled nutritionist, and presented in publications such as the following: NRC, Nutrient requirements in swine, ninth revised edition 1988, subcommittee on swine nutrition, committee on animal nutrition, board of agriculture, national research council. National Academy Press, Washington, D.C. 1988; and NRC, Nutrient requirements of poultry, ninth revised edition 1994, subcommittee on poultry nutrition, committee on animal nutrition, board of agriculture, national research council, National Academy Press, Washington, D.C., 1994.

The phytase variant should of course be applied in animal feed in an effective amount, i.e. in an amount adequate for improving the nutritional value of the feed. It is at present contemplated that it is administered in the following dosage ranges: 0.01-200; or 0.01-100; or 0.05-100; or 0.05-50; or 0.10-10 - all these ranges being in mg enzyme protein per kg feed (ppm).

For determining mg phytase protein per kg feed or feed additive, the enzyme is purified from the feed composition or the feed additive, and the specific activity of the purified enzyme is determined using a relevant assay. The phytase activity of the feed composition or the feed additive is also determined using the same assay, and on the basis of these two determinations, the dosage in mg phytase protein per kg feed is calculated.

Of course, if a sample is available of the phytase variant used for preparing the feed additive or the feed, the specific activity is determined from this sample (no need to purify the enzyme from the feed composition or the additive).

The composition of the invention can be prepared according to methods known in the art, e.g. by mixing the phytase variant with the additional ingredients, if any.

Animal feed compositions or diets have a relatively high content of protein. An animal feed composition according to the invention has a crude protein content of 50-800, or 75-700, or 100-600, or 110-500, or 120-490 g/kg, and furthermore comprises a composition of the invention.

Furthermore, or in the alternative (to the crude protein content indicated above), the animal feed composition of the invention has a content of metabolisable energy of 10-30, or 11-28, or 11-26, or 12-25 MJ/kg; and/or a content of calcium of 0.1-200, or 0.5-150, or 1-100, or 4-50 g/kg; and/or a content of available phosphorus of 0.1-200, or 0.5-150, or 1-100, or 1-50, or 1-25 g/kg; and/or a content of methionine of 0.1-100, or 0.5-75, or 1-50, or 1-30 g/kg; and/or a content of methionine plus cysteine of 0.1-150, or 0.5-125, or 1-80 g/kg; and/or a content of lysine of 0.5-50, or 0.5-40, or 1-30 g/kg.

Crude protein is calculated as nitrogen (N) multiplied by a factor 6.25, i.e. Crude protein (g/kg)= N (g/kg) x 6.25 as stated in Animal Nutrition, 4^{th} edition, Chapter 13 (Eds. P. McDonald, R. A. Edwards and J. F. D. Greenhalgh, Longman Scientific and Technical, 1988, ISBN 0-582-40903-9). The nitrogen content can be determined by the Kjeldahl method (A.O.A.C., 1984, Official Methods of Analysis 14^{th} ed., Association of Official Analytical Chemists, Washington DC). But also other methods can be used, such as the so-called Dumas method in which the sample is combusted in oxygen and the amount of nitrous gasses formed are analysed and recalculated as nitrogen.

Metabolisable energy can be calculated on the basis of the NRC publication Nutrient Requirements of Swine (1988) pp. 2-6, and the European Table of Energy Values for Poultry Feed-stuffs, Spelderholt centre for poultry research and extension, 7361 DA Beekbergen, The Netherlands. Grafisch bedrijf Ponsen & looijen bv, Wageningen. ISBN 90-71463-12-5.

In a particular embodiment, the animal feed composition of the invention contains at least one vegetable protein or protein source. Examples of vegetable proteins or protein sources are soybean, peas and rape seed from leguminosae and brassica families, and the cereals such as barley, maize (corn), oat, rice, rye, sorghum and wheat.

In still further particular embodiments, the animal feed composition of the invention contains 0-80% maize; and/or 0-80% sorghum; and/or 0-70% wheat; and/or 0-70% Barley; and/or 0-30% oats; and/or 0-40% soybean meal; and/or 0-10% fish meal; and/or 0-20% whey.

Animal diets can e.g. be manufactured as mash feed (non-pelleted) or pelleted feed. Typically, the milled feed-stuffs are mixed and sufficient amounts of essential vitamins and minerals are added according to the specifications for the species in question.

The phytase variant of the invention can be added in the form of a solid or liquid enzyme formulation, or in the form of a feed additive, such as a pre-mix. A solid composition is typically added before or during the mixing step; and a liquid composition is typically added after the pelleting step.

The phytase variant of the invention when added to animal feed leads to an improved nutritional value of the feed, e.g. the growth rate and/or the weight gain and/or the feed conversion (i.e. the weight of ingested feed relative to weight gain) of the animal is/are improved. These results may be due to, in turn, one or more of the following effects: The phosphate moieties of phytic acid chelates divalent and trivalent cations such as metal ions, i.a. the nutritionally essential ions of calcium, iron, zinc and magnesium as well as the trace minerals mangane, copper and molybdenum. Besides, the phytic acid also to a certain extent binds proteins by electrostatic interaction. At a pH below the isoelectric point, pl, of the protein, the positively charged protein binds directly with phytate. At a pH above pl, the negatively charged protein binds via metal ions to phytate. Phytic acid and its salts, phytates, are often not metabolized, since they are not absorbable from the gastro intestinal system, i.e. neither the phosphorous thereof, nor the chelated metal ions, nor the bound proteins are nutritionally available. Accordingly, since phosphorus is an essential element for the growth of all organisms, food and feed preparations need to be supplemented with inorganic phosphate. Quite often also the nutritionally essential ions such as iron and calcium, must be supplemented. And, besides, the nutritional value of a given diet decreases, because of the binding of proteins by phytic acid.

In particular embodiments the weight gain is at least 101, 102, 103, 104, 105, 106, 107, 108, 109, or at least 110% of the control (no enzyme addition).

In further particular embodiments the feed conversion is at most (or not more than) 99, 98, 97, 96, 95, 94, 93, 92, 91 or at most 90%, as compared to the control (no enzyme addition).

The composition of the invention may also be used *in vitro,* e.g. to treat vegetable proteins. The term vegetable proteins as used herein refers to any compound, composition, preparation or mixture that includes at least one protein derived from or originating from a vegetable, including modified proteins and protein-derivatives. In particular embodiments, the protein content of the vegetable proteins is at least 10, 20, 30, 40, 50, or 60% (w/w).

Examples of vegetable proteins or protein sources are cereals such as barley, wheat, rye, oat, maize (corn), rice, and sorghum. Other examples are soya bean meal, peas and rape seed meal from leguminosae and brassica families.

The vegetable protein or protein source is typically suspended in a solvent, eg an aqueous solvent such as water, and the pH and temperature values are adjusted paying due regard to the characteristics of the phytase variant and additional enzymes, if any. The enzymatic reaction is continued until the desired result is achieved, following which it may or may not be stopped by inactivating the enzyme, e.g. by a heat-treatment step.

In another particular embodiment of a treatment process of the invention, the enzyme actions are sustained, meaning e.g. that the enzyme composition is added to the vegetable proteins or protein sources, but the enzyme activity is so to speak not switched on until later when desired, once suitable reaction conditions are established, or once any enzyme inhibitors are inactivated, or whatever other means may have been applied to postpone the action of the enzymes.

### Method for Generating Phytase Variants

In a further aspect, the invention relates to a method for generating a variant of a parent phytase, wherein the variant is more thermostable and/or has a higher specific activity than the parent phytase, the method comprising:
(d) subjecting a DNA sequence encoding the parent phytase to random mutagenesis,
(e) expressing the mutated DNA sequence obtained in step (d) in a host cell, and
(f) screening for host cells expressing a phytase variant which is more thermostable and/or has a higher specific activity than the parent phytase.

Step (d) of the above method of the invention is preferably performed using doped primers, as described in the experimental part.

In a preferred embodiment, the above method includes the following additional steps preceeding above steps (d)-(f):
(a) establishing a 3D structure of a parent phytase,
(b) subjecting the 3D structure of (a) to MD simulations at increased temperatures;
(c) identifying regions in the amino acid sequence of the parent phytase of high mobility (isotropic fluctuations) and suggesting these for random mutagenesis, wherein at least one of the regions identified in step (c) is targetted in step (d).

The structure of (a) may be established by homology modelling. A preferred model is the Fig. 1 structure.

Examples of increased temperatures are one or more of the following temperatures: 300K, 350K, 400K, 450K, 500K, and 550K.

For this aspect of the present invention, the parent phytase may be a wild-type parent phytases, or allelic variants thereof, or fragments thereof that has phytase acticity, or mature parts thereof, or variants thereof, or genetically engineered or synthetic polypeptides designed on the basis thereof. The following are examples of preferred wild-type parent phytases: Fungal phytases, such as filamentous fungal phytases, or yeast phytases. An example of a yeast phytase is the *Schwanniomyces occidentalis* phytase described in US 5830732. Examples of filamentous fungal phytases are the phytases derived from the phylum *Ascomycota* or the phylum *Basidiomycota* (an ascomycete phytase, or a basidiomycete phytase, respectively). Examples of ascomycete phytases are those derived from a strain of *Aspergillus,* for example *Aspergillus awamori* PHYA (SWISSPROT P34753, Gene 133:55-62 (1993)), *Aspergillus niger (ficuum)* PHYA (SWISSPROT P34752, EP 420358, Gene 127:87-94 (1993)), *Aspergillus awamori* PHYB (SWISSPROT P34755, Gene 133:55-62 (1993)), *Aspergillus niger* PHYB (SWISSPROT P34754, Biochem. Biophys. Res. Commun. 195:53-57(1993)); or a strain of *Emericella,* for example *Emericella nidulans* PHYB (SWISSPROT 000093, Biochim. Biophys. Acta 1353:217-223 (1997)); or a strain of *Thermomyces (Humicola),* for example the *Thermomyces lanuginosus* phytase described in WO 97/35017. Other examples of ascomycete phytases are disclosed in EP 684313 (for example derived from strains of *Aspergillus fumigatus, Aspergillus terreus,* and *Myceliophthora thermophila);* JP 11000164 (a phytase derived from a strain of *Penicillium.);* US 6139902 (a phytase derived from a strain of *Aspergillus),* and WO 98/13480 *(Monascus anka* phytase). Examples of basidiomycete phytases are the phytases derived from *Paxillus involutus, Trametes pubescens, Agrocybe pediades* and *Peniophora lycii* (see WO 98/28409).

Still for the purposes of this aspect of the present invention, the basidiomycete phytases are most preferred, and a highly preferred parent phytase is the phytase derived from *Peniphora lycii* CBS 686.96 (as well as allelic variants, fragments etc. thereof as generally described above, as well as homologous phytases).

Still for the purposes of this aspect of the present invention, the following phytases and phytase sequences of WO 00/43503 are examples of preferred synthetic parent phytases: SEQ ID NO: 22 (basidio consensus), SEQ ID NO: 24 (mature part of consensus phytase-10); SEQ ID NO: 26 (consensus phytase-10); SEQ ID NO: 27 (consensus phytase-11), SEQ ID NO: 31 (consensus phytase-10-thermo(3)-Q50T-K91A), SEQ ID NO: 36 (consensus phytase-12), SEQ ID NO: 91 (consensus phytase-3-thermo(11)-Q50T), SEQ ID NO: 93 (consensus phytase-3-thermo(11)-050T-K91A), SEQ ID NO: 95 (consensus phytase-10-thermo(5)-050T); and SEQ ID NO: 97 (consensus phytase-10-thermo(5)-Q50T-K91A), consensus phytase-10-thermo(3), consensus-phytase-10-thermo(3)-050T, consensus-phytase-10-thermo(3)-K91A, amino acids 27-467 of consensus phytase-10-thermo(3), amino acids 27-467 of consensus phytase-10-thermo(3)-Q50T, amino acids 27-467 of consensus phytase-10-thermo(3)-K91A.

Other examples of preferred synthetic parent phytases are: The variants of an *Aspergillus fumigatus* phytase disclosed in EP 897010, the fungal consensus phytase and its variants disclosed in EP 897985, the *Aspergillus fumigatus* phytase variants, and the phytase designated consensus phytase-7, as well as the variants of consensus phytase-1 disclosed in WO 00/43503.

A preferred screening method for thermostable phytase variants is the primary screening described in Example 1: Cultivation of candidate colonies (e.g. a yeast library) on an appropriate solid medium (e.g. SC-glucose plates) for an appropriate time (e.g. for 3 days at 30°C), replica plate to new plates with nitrate filters, incubate for an appropriate time (e.g. at 30°C for 1 day), transfer filters to pre-heated Na-phytate plates at an appropriate pH (e.g. pH5.5 and 53°C), incubate at an elevated temperature for an appropriate time (e.g. at 53°C over night), remove filters, and pour on a solution containing Ca-ions (e.g. 0.5M CaC1₂ solution), electing colonies with larger clearing zones than the parent phytase.

Another preferred screening method is the secondary screening described in Example 1 (submerse cultivation, establishing a phytase activity vs. temperature profile on the supernatants, selecting candidates performing better than the parent phytase (the higher activity at the higher the temperature, the better)). For both of these tests, alternative temperatures are those indicated above in the thermostability section. The two methods can be used in combination/succession as described in Example 1.

Preferred random mutagenesis methods are doping, and spiked oligo shuffling.

In an even more preferred embodiment of the above method for generating phytase variants, the method is made iterative, meaning that in a first step, the parent phytase is one of the phytases mentioned above, e.g. the *Peniphora* phytase, in a second step the parent phytase is a more thermostable variant X thereof, in a third step the parent phytase is another variant Y of even higher thermostability than X, and so forth.

A preferred screening method for improved specific activity is the primary screening method referred to in Example 1. Another preferred screening method for improved specific activity is the secondary screening method referred to in Example 1.

The invention also relates to a method for producing a phytase variant obtainable or obtained by the method of generating phytase variants described above, comprising (a) cultivating the host cell to produce a supernatant comprising the variant; and (b) recovering the variant.

The present invention is further described by the following examples which should not be construed as limiting the scope of the invention.

### Particular Embodiments

An aspect of the present invention is the transfer of selected (positive, viz. e.g. improved thermostability and/or specific activity) mutations in the *Peniophora* phytase backbone via a homology alignment to other backbones, preferably homologous backbones. This concept may be described as follows: In each of the claims and additional particular embodiments such as those listed below, substitute "parent phytase" with "phytase derived from *Peniophora lycii* CBS 686.96," and add the following claim: A variant of a phytase which is homologous to the phytase derived from *Peniophora lycii* CBS 686.96, the variant comprising a substitution in at least one position corresponding to those claimed and listed in the additional embodiments, the corresponding position being deduced by aligning the homologous phytase to SEQ ID NO: 2 as described above.

Various particular embodiments of the phytase variant of the invention are claimed, and additional particular embodiments are set out below. The invention also relates to the following aspects of the below embodiments: Isolated nucleic acid sequences; nucleic acid constructs; recombinant expression vectors; recombinant host cells; methods for producing; transgenic plants, or plant parts; transgenic non-human animals, or products, or elements thereof; compositions such as feed additives, and animal feed; methods and processes of using; and uses (as claimed herein). A composition comprising at least one phytase variant of any of the claims or any of the above embodiments is specifically included herein. The animal feed preferably comprises at least one vegetable protein or protein source.

A variant of a parent phytase, comprising a substitution in at least one position selected from the group consisting of: 20; 26; 28; 29; 30; 31; 37; 38; 39; 40; 41; 42; 43; 44; 45; 46; 47; 48; 55; 56; 57; 58; 59; 60; 61; 62; 63; 64; 65; 66; 67; 68; 69; 73; 74; 75; 76; 77; 78; 79; 80; 81; 82; 83; 91; 92; 93; 94; 95; 96; 97; 98; 99; 100; 101; 102; 103; 104; 105; 106; 107; 108; 109; 110; 111; 112; 113; 114; 115; 116; 117; 118; 119; 123; 124; 125; 126; 135; 136; 137; 138; 139; 140; 141; 142; 152; 153; 154; 155; 156; 157; 158; 159; 160; 161; 162; 163; 169; 170; 171; 172; 173; 174; 175; 176; 177; 178; 179; 180; 181; 182; 183; 184; 185; 186; 187; 188; 189; 190; 191; 192; 193; 194; 195; 196; 197; 198; 199; 204; 205; 206; 207; 208; 209; 210; 211, 212; 213; 214; 215; 216; 217; 218; 219; 220; 221; 222; 229; 230; 231; 232; 233; 234; 235; 236; 237; 238; 248; 249; 250; 251; 252; 253; 254; 255; 256; 257; 258; 259; 260; 261; 262; 263; 264; 265; 266; 284; 285; 286; 287; 288; 289; 300; 301; 302; 303; 304; 305; 306; 307; 308; 321; 322; 323; 324; 325; 326; 327; 328; 329; 330; 331; 332; 333; 334; 335; 343; 344; 345; 346; 347; 348; 349; 350; 384; 385; 386; 387; 388; 389; 390; 391; 392; 393; 394; 395; 396; 397; 398; 407; 408; 409; 410; 411; 412; 419; 420; 421; 422; 423; 424;425;426;427;428;429;430;
wherein
(a) the variant has phytase activity;
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of: G26S; Y28N; D29S; F31Y; E42D,K,A; T45R; V461; W59F; S62D; A64K; R65Q,E,G,A; S66T; R67A,I,Q,K; Q68Y,V,I; K74R,A; A99N; D100S; L102V; P103E; F104L; N107T,Q; S109M; H110S,V; Q111E,N; T112A,S; M116F,I,L; A135S; D137Y; Q138D,S,G; D142A; ()154fH; S155N; G156P; E1570,S; E169S; E170A,P; G171A; ()17laS; T174P,A; N177S; N178S,T; M179T,V,L; N182A,V; V184G; D185E; ()185eT; G186S,A; D187R,K,S; ()187aV,T; E1880,S,A,V; S189E; V195L; N199A,P; L204N; A207D,H; S216T; D217E; L219Y,T; T220Y,N; L221 F; M222L; L230V; ()235eE,Q; V248R,1; S249Q,A; E251D; Y254A,L,G; D255N; D257K; Y259F; T262H; P264A; G286R,H; Q287K,S; A288P; T3210,S,G; M3221; V3231; P324A; A327S,F,I,L; F332Y; N333P; E344R; R346P; W348F; V349L,A,S; D350S,V; G388A; V393R; E395K,T; and L396R.

A variant of a parent phytase, comprising a substitution in at least one position of at least one region selected from the group of regions consisting of: 20; 26; 28-31; 37-48; 55-69; 73-83; 91-119; 123-126; 135-142; 152-163; 169-199; 204-222; 229-238; 248-266; 284-289; 300-308; 321-335; 343-350; 384-398; 407-412; and 419-430; wherein
(a) the variant has phytase activity; and
(b) each position corresponds to a position of SEQ ID NO: 2; and
(c) the variant has a percentage of identity to SEQ ID NO: 2 of at least 75%; and wherein the variant in position 26; 28; 29; 31; 42; 45; 46; 59; 62; 64; 65; 66; 67; 68; 74; 99; 100; 102; 103; 104; 107; 109; 110; 111; 112; 116; 135; 137; 138; 142; 154f; 155; 156; 157; 169; 170; 171; 171 a; 174; 177; 178; 179; 182; 184; 185; 185e; 186; 187; 187a; 188; 189; 195; 199; 204; 207; 216; 217; 219; 220; 221; 222; 230; 235e; 248; 249; 251; 254; 255; 257; 259; 262; 264; 286; 287; 288; 321 322; 323; 324; 327; 332; 333; 344; 346; 348; 349; 350; 388; 393; 395; and 396 is selected from the group consisting of:
   26A,C,D, E,F,H,I,K,L,M,N,P,Q,R,T,V,W,Y; 28A,C,D, E,F,G,H,I,K,L,M,P,Q,R,S,T,V,W; 29A,C,E,F,G,H,I,K,L,M,N,P,Q,R,T,V,W,Y; 31A,C,D, E,G,H,I,K,L,M,N,P,Q,R,S,T,V,W; 42C,F,G,H,I,L,M,N,P,Q,R,S,T,V,W,Y; 45A,C,D,E,F,G,H,I,K,L,M,N,P,Q,S,V,W,Y; 46A,C,D,E,F,G,H,K,L,M,N,P,Q,R,S,T,W,Y; 59A,C,D, E,G,H,I,K,L,M,N,P,Q,R,S,T,V,Y; 62A,C,E,F,G,H,I,K,L,M,N,P,Q,R,T,V,W,Y; 64C,D, E,F,G,H,I,L,M,N,P,Q,R,S,T,V,W,Y; 65C,D, F,H,I,K,L,M,N,P,S,T,V,W,Y; 66A,C,D, E,F,G,H,I,K,L,M,N,P,Q,R,V,W,Y; 67C,D, E,F,G,H,L,M,N,P,S,T,V,W,Y; 68A,C,D, E,F,G,H,K,L,M,N,P,R,S,T,W; 74C,D,E,F,G,H,I,L,M,N,P,Q,S,T,V,W,Y; 99C,D,E,F,G,H,I,K,L,M,P,Q,R,S,T,V,W,Y 100A,C,E,F,G,H,I,K,L,M,N,P,Q,R,T,V,W,Y; 102A,C,D,E,F,G,H,I,K,M,N,P,Q,R,S,T,W,Y; 103A,C,D,F,G,H,I,K,L,M,N,Q,R,S,T,V,W,Y; 104A,C,D,E,G,H,I,K,M,N,P,Q,R,S,T,V,W,Y; 107A,C,D,E,F,G,H,I,K,L,M,P,R,S,V,W,Y; 109A,C,D,E,F,G,H,I,K,L,N,P,Q,R,T,V,W,Y; 110A,C,D,E,F,G,I,K,L,M,N,P,Q,R,T,W,Y; 111A,C,D,F,G,H,I,K,L,M,P,R,S,T,V,W,Y; 112C,D,E,F,G,H,I,K,L,M,N,P,Q,R,V,W,Y; 116A,C,D,E,G,H,K,N,P,Q,R,S,T,V,W,Y; 135C,D, E,F,G,H,I,K,L,M,N,P,Q,R,T,V,W,Y; 137A,C,E,F,G,H,I,K,L,M,N,P,Q,R,S,T,V,W; 138A,C,E,F,H,I,K,L,M,N,P,R,T,V,W,Y; 142C,E,F,G,H,I,K,L,M,N,P,Q,R,S,T,V,W,Y; 154fA,C,D,E,F,G,I,K,L,M,N,P,Q,R,S,T,V,W,Y; 155A,C,D,E,F,G,H,I,K,L,M,P,Q,R,T,V,W,Y; 156A,C,D,E,F,H,I,K,L,M,N,Q,R,S,T,V,W,Y; 157A,C,D,F,G,H,I,K,L,M,N,P,R,T,V,W,Y; 169A,C,D, F,G,H,I,K,L,M,N,P,Q,R,T,V,W,Y; 170C,D, F,G,H,I,K,L,M,N,Q,R,S,T,V,W,Y; 171C,D, E,F,H,I,K,L,M,N,P,Q,R,S,T,V,W,Y; 171aA,C,D, E,F,G,H,I,K,L,M,N,P,Q,R,T,V,W,Y; 174C,D, E,F,G,H,I,K,L,M,N,Q,R,S,V,W,Y; 177A,C,D, E,F,G,H,I,K,L,M,P,Q,R,T,V,W,Y; 178.A,C,D, E,F,G,H,I,K,L,M,P,Q,R,V,W,Y; 179A,C,D, E,F,G,H,I,K,N,P,Q,R,S,W,Y; 182C,D, E,F,G,H,I,K,L,M,P,Q,R,S,T,W,Y; 184A,C,D,E, F,H,I,K,L,M,N,P,Q,R,S,T,W,Y; 185A,C,F,G,H,I,K,L,M,N,P,Q,R,S,T,V,W,Y; 185eA,C,D,E, F,G,H,I,K,L,M,N,P,Q,R,S,V,W,Y; 186C,D,E, F,H,I,K,L,M,N,P,Q,R,T,V,W,Y; 187A,C,E,F,G,H,I,L,M,N,P,Q,T,V,W,Y; 187aA,C,D,E,F,G,H,I,K,L,M,N,P,Q,R,S,W,Y; 188C,D,F,G,H,I,K,L,M,N,P,R,T,W,Y; 189A,C,D,F,G,H,I,K,L,M,N,P,Q,R,T,V,W,Y; 195 A,C,D,E, F,G,H,I,K,M,N,P,Q,R,S,T,W,Y; 199C,D,E,F,G,H,I,K,L,M,Q,R,S,T,V,W,Y; 204A,C,D,E,F,G,H,I,K,M,P,Q,R,S,T,V,W,Y; 207C,E,F,G,I,K,L,M,N,P,Q,R,S,T,V,W,Y; 216A,C,D,E,F,G,H,I,K,L,M,N,P,Q,R,V,W,Y; 217A,C,F,G,H,I,K,L,M,N,P,Q,R,S,T,V,W,Y; 219A,C,D, E,F,G,H,I,K,M,N,P,Q,R,S,V,W; 220A,C,D, E,F,G,H,I,K,L,M,P,Q,R,S,V,W; 221A,C,D, E,G,H,I,K,M,N,P,Q,R,S,T,V,W,Y; 222A,C,D, E,F,G,H,I,K,N,P,Q,R,S,T,V,W,Y; 230A,C,D,E,F,G,H,I,K,M,N,P,Q,R,S,T,W,Y; 235eA,C,D,F,G,H,I,K,L,M,N,P,R,S,T,V,W,Y; 248A,C,D,E,F,G,H,K,L,M,N,P,Q,S,T,W,Y; 249C,D, E,F,G,H,I,K,L,M,N,P,R,T,V,W,Y; 251A,C,F,G,H,I,K,L,M,N,P,Q,R,S,T,V,W,Y; 254C,D, E,F,H,I,K,M,N,P,Q,R,S,T,V,W; 255A,C,E,F,G,H,I,K,L,M,P,Q,R,S,T,V,W,Y; 257A,C,E,F,G,H,I,L,M,N,P,Q,R,S,T,V,W,Y; 259A,C,D, E,G,H,I,K,L,M,N,P,Q,R,S,T,V,W; 262A,C,D, E,F,G,I,K,L,M,N,P,Q,R,S,V,W,Y; 264C,D, E,F,G,H,I,K,L,M,N,Q,R,S,T,V,W,Y; 286A,C,D,E,F,I,K,L,M,N,P,Q,S,T,V,W,Y; 287A,C,D,E,F,G,H,I,L,M,N,P,R,T,V,W,Y; 288C,D,E,F,G,H,I,K,L,M,N,Q,R,S,T,V,W,Y; 321A,C,D, E,F,H,I,K,L,M,N,P,R,V,W,Y; 322A,C,D, E,F,G,H,K,L,N,P,Q,R,S,T,V,W,Y; 323A,C,D,E,F,G,H,K,L,M,N,P,Q,R,S,T,W,Y; 324C,D,E,F,G,H,I,K,L,M,N,Q,R,S,T,V,W,Y; 327C,D,E,G,H,K,M,N,P,Q,R,T,V,W,Y; 332A,C,D,E,G,H,I,K,L,M,N,P,Q,R,S,T,V,W; 333A,C,D,E,F,G,H,I,K,L,M,Q,R,S,T,V,W,Y; 344A,C,D,F,G,H,I,K,L,M,N,P,Q,S,T,V,W,Y; 346A,C,D,E,F,G,H,I,K,L,M,N,Q,S,T,V,W,Y; 348A,C,D,E,G,H,I,K,L,M,N,P,Q,R,S,T,V,Y; 349C,D,E,F,G,H,I,K,M,N,P,Q,R,T,W,Y; 350A,C,E,F,G,H,I,K,L,M,N,P,Q,R,T,W,Y; 388C,D, E,F,H,I,K,L,M,N,P,Q,R,S,T,V,W,Y; 393A,C,D, E,F,G,H,I,K,L,M,N,P,Q,S,T,W,Y; 395A,C,D,F,G,H,I,L,M,N,P,Q,R,S,V,W,Y; and 396A,C,D, E,F,G,H,I,K,M,N,P,Q,S,T,V,W,Y;

A variant of a parent phytase, comprising a substitution in at least one position of at least one region selected from the group of regions consisting of: 20; 28-31; 37-48; 55-69; 91-119; 123-126; 152-163; 169-199; 204-222; 229-238; 248-266; 284-289; 321-335; 343-350; 384-398; 407-412; and 419-430; wherein
(a) the variant has phytase activity; and
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of: Y28N; D29S; F31Y; E42D,K,A; T45R; V461; W59F; S62D; A64K; R65Q,E,G,A; S66T; R67A,I,Q,K; Q68Y,V,I; A99N; D100S; L102V; P103E; F104L; N107T,Q; S109M; H110S,V; Q111E,N; T112A,S; M116F,I,L; ()154fH; S155N; G156P; E157Q,S; E169S; E170A,P; G171A; ()17laS; T174P,A; N177S; N178S,T; M179T,V,L; N182A,V; V184G; D185E; ()185eT; G186S,A; D187R,K,S; ()187aV,T; E188Q,S,A,V; S189E; V195L; N199A,P; L204N; A207D,H; S216T; D217E; L219Y,T; T220Y,N; L221F; M222L; L230V; ()235eE,Q; V248R,1; S249Q,A; E251 D; Y254A,L,G; D255N; D257K; Y259F; T262H; P264A; G286R,H; Q287K,S; A288P; T321Q,S,G; M3221; V3231; P324A; A327S,F,I,L; F332Y; N333P; E344R; R346P; W348F; V349L,A,S; D350S,V; G388A; V393R; E395K,T; and L396R.

A variant of a parent phytase, comprising a substitution in at least one position selected from the group consisting of: 20; 29; 45; 63; 69; 92; 93; 95; 97; 98; 99; 102; 110; 114; 118; 125; 152; 156; 157; 160; 163; 183; 185; 187; 190; 191; 194; 199; 205; 210; 218; 222; 234; 248; 286; 321; 323; 324; 328; 330; 334; 343; 344; 345; 347; 349; 350; 384; 395; 398; 409; 421; 422; 423; 424; 425; 426; 427; 428; 429; 430;
wherein
(a) the variant has phytase activity;
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of: D29S; T45R; A99N; L102V; H110S,V; G156P; E157Q,S; D185E; ()185eT; D187R,K,S; ()187aV,T; N199A,P; M222L; V248R,1; G286R,H; T321Q,S,G; V3231; P324A; E344R; V349L,A,S; D350S,V; and E395K,T.

A variant of a parent phytase, comprising a substitution in at least one position selected from the group consisting of: 20; 29; 45; 63; 69; 92; 93; 95; 97; 98; 99; 102; 110; 114; 118; 125; 152; 156; 157; 160; 163; 183; 185; 187; 190; 191; 194; 199; 205; 210; 218; 222; 234; 248; 286; 321; 323; 324; 328; 330; 334; 343; 344; 345; 347; 349; 350; 384; 395; 398; 409; 421; 422; 423; 424; 425; 426; 427; 428; 429; 430;
wherein
(a) the variant has phytase activity;
(b) each position corresponds to a position of SEQ ID NO: 2; and
(c) the variant has a percentage of identity to SEQ ID NO: 2 of at least 75%; and
(d) wherein the variant in position 29; 45; 99; 102; 110; 156; 157; 185; 185e; 187; 187a; 199; 222; 248; 286; 321; 323; 324; 344; 349; 350; and 395 is selected from the group consisting of:
   29A,C,E,F,G,H,I,K,L,M,N,P,Q,R,T,V,W,Y; 45A,C,D,E,F,G,H,I,K,L,M,N,P,Q,S,V,W,Y; 99C,D,E,F,G,H,I,K,L,M,P,Q,R,S,T,V,W,Y; 102A,C,D,E,F,G,H,I,K,M,N,P,Q,R,S,T,W,Y; 110A,C,D,E,F,G,I,K,L,M,N,P,O,R,T,W,Y; 156A,C,D,E,F,H,I,K,L,M,N,Q,R,S,T,V,W,Y; 157A,C,D,F,G,H,I,K,L,M,N,P,R,T,V,W,Y; 185A,C,F,G,H,I,K,L,M,N,P,Q,R,S,T,V,W,Y; 185eA,C,D,E,F,G,H,I,K,L,M,N,P,Q,R,S,V,W,Y; 187A,C,E,F,G,H,I,L,M,N,P,Q,T,V,W,Y; 187aA,C,D,E,F,G,H,I,K,L,M,N,P,Q,R,S,W,Y; 199C,D,E,F,G,H,I,K,L,M,Q,R,S,T,V,W,Y; 222A,C,D, E,F,G,H,I,K,N,P,Q,R,S,T,V,W,Y; 248A,C,D,E,F,G,H,K,L,M,N,P,Q,S,T,W,Y; 286A,C,D,E,F,I,K,L,M,N,P,Q,S,T,V,W,Y; 321A,C,D, E,F,H,I,K,L,M,N,P,R,V,W,Y; 323A,C,D,E,F,G,H,K,L,M,N,P,Q,R,S,T,W,Y; 324C,D,E,F,G,H,I,K,L,M,N,Q,R,S,T,V,W,Y; 344A,C,D,F,G,H,I,K,L,M,N,P,Q,S,T,V,W,Y; 349C,D,E,F,G,H,I,K,M,N,P,Q,R,T,W,Y; 350A,C,E,F,G,H,I,K,L,M,N,P,Q,R,T,W,Y; and 395A,C,D,F,G,H,I,L,M,N,P,Q,R,S,V,W,Y.

The variant of the claims or any of the above embodiments, which comprises at least one of the substitutions selected from the group consisting of: 20R; 29N,C,T; 45A; 63N; 69A; 92L; 931; 95R,H,M,N,S,T; 97V; 98G; 99D; 1021; 110Y,R,W; 114A; 118A; 125D; 152A; 156C,R; 157K; 160R; 163P; 183K,A,W,G; 185A,H,N,P,K; 187G; 190S,K; 191 L,R,S; 194R; 199S; 205S; 210G; 218T; 2221; 234K,S; 248A,C,D,E,G,H,S,T; 286S; 321A; 323A; 324S,L; 328T; 330R; 334E,D,G; 343N; 344A,V,S; 345D,H; 347Q,P; 349M,K; D350T,M,I,L; 384S,A; 395G; 398T; 409S; 421 R,S; 422R; 423N; 424S; 425L; 426E; 427G; 428R; 4291; and 430M.

The variant of the claims or any of the above and below embodiments, wherein the parent phytase i) has an amino acid sequence which has a degree of identity to the amino acid sequence of SEQ ID NO: 2 of at least about 60%, preferably of at least 60%; ii) is encoded by a nucleic acid sequence which hybridizes under low stringency conditions with nucleotides 49-1320 of SEQ ID NO: 1 or the complementary strand thereof; iii) is a fungal phytase, preferably a basidiomycete phytase or a mixed ascomycete/basidiomycete phytase, such as a *Peniophora* phytase, e.g. a phytase derived from *Peniophora lycii ,* e.g. *Peniophora lycii* CBS 686.96.

The phytase variant of the claims or any of the above and below embodiments, which is thermostable and/or of a high specific activity, preferably more thermostable and/or of a higher specific activity, as compared to the parent phytase.

A variant of a parent phytase, comprising a substitution in at least one position of at least one region selected from the group of regions consisting of: 20; 29; 37-48; 63; 69; 73-83; 91-119; 123-126; 154-157; 163; 183; 187; 194; 199; 204-218; 229-238; 248; 284-289; 300-308; 323-335; 343-350; 384; 395; 398; 407-412; and 419-430; wherein (a) the variant has phytase activity; and (b) each position corresponds to a position of SEQ ID NO: 2; and (c) the variant has a percentage of identity to SEQ ID NO: 2 of at least 75%; and (d) with the proviso that the variant is not a variant of the *Peniophora lycii* CBS 686.96 phytase selected from the group consisting of: D29S; E42D,K,A; T45R; V461; K74R,A; A99N; D100S; L102V; P103E; F104L; N107T,Q; S109M; H110S,V; Q111E,N; T112A,S; M116F,I,L; S155N; ()154fH; G156P; E157Q,S; D187R,K,S; ()187aV,T; N199A,P; L204N; A207D,H; S216T; D217E; L230V; ()235eE,Q; V248R,1; G286R,H; Q287K,S; A288P; V3231; P324A; A327S,F,I,L; F332Y; N333P; E344R; R346P; W348F; V349L,A,S; D350K,A; and E395K,T.

A variant of a parent phytase, comprising a substitution in at least one position selected from the group consisting of: 20; 29; 37; 38; 39; 40; 41; 42; 43; 44; 45; 46; 47; 48; 63; 69; 73; 74; 75; 76; 77; 78; 79; 80; 81; 82; 83; 91; 92; 93; 94; 95; 96; 97; 98; 99; 100; 101; 102; 103; 104; 105; 106; 107; 108; 109; 110; 111; 112; 113; 114; 115; 116; 117; 118; 119; 123; 124; 125; 126; 154; 155; 156; 157; 163; 183; 187; 194; 199; 204; 205; 206; 207; 208; 209; 210; 211, 212; 213; 214; 215; 216; 217; 218; 229; 230; 231; 232; 233; 234; 235; 236; 237; 238; 248; 300; 301; 302; 303; 304; 305; 306; 307; 308; 323; 324; 325; 326; 327; 328; 329; 330; 331; 332; 333; 334; 335; 343; 344; 345; 346; 347; 348; 349; 350; 384; 395; 398; 407; 408; 409; 410; 411; 412; 419; 420; 421; 422; 423; 424; 425; 426; 427; 428; 429; 430;
wherein (a) the variant has phytase activity; (b) each position corresponds to a position of SEQ ID NO: 2; and (c) the variant has a percentage of identity to SEQ ID NO: 2 of at least 75%; and (d) with the proviso that the variant is not a variant of the *Peniophora lycii* CBS 686.96 phytase selected from the group consisting of: D29S; E42D,K,A; T45R; V461; K74R,A; A99N; D100S; L102V; P103E; F104L; N107T,Q; S109M; H110S,V; Q111E,N; T112A,S; M116F,I,L; S155N; ()154fH; G156P; E157Q,S; D187R,K,S; ()187aV,T; N199A,P; L204N; A207D,H; S216T; D217E; L230V; ()235eE,Q; V248R,I; G286R,H; Q287K,S; A288P; V3231; P324A; A327S,F,I,L; F332Y; N333P; E344R; R346P; W348F; V349L,A,S; D350K,A; and E395K,T.

A variant of a parent phytase, comprising a substitution in at least one position of at least one region selected from the group of regions consisting of: 20; 29; 37-48; 63; 69; 73-83; 91-119; 123-126; 154-157; 163; 183; 187; 194; 199; 204-218; 229-238; 248; 284-289; 300-308; 323-335; 343-350; 384; 395; 398; 407-412; and 419-430; wherein (a) the variant has phytase activity; and (b) each position corresponds to a position of SEQ ID NO: 2; and (c) the variant has a percentage of identity to SEQ ID NO: 2 of at least 75%; and wherein the variant in position 29, 42, 45, 46, 74, 99, 100, 102, 103, 104, 107, 109, 110, 111, 112, 116, 155, 154, 156, 157, 187, 187a, 199, 204, 207, 216, 217, 230, 235e, 248, 286, 287, 288, 323, 324, 327, 332, 333, 344, 346, 348, 349, 350, 395 is selected from the group consisting of: 29A,C,E,F,G,H,I,K,L,M,N,P,Q,R,T,V,W,Y;
42C,F,G,H,I,L,M,N,P,Q,R,S,T,V,W,Y; 45A,C,D,E,F,G,H,I,K,L,M,N,P,Q,S,V,W,Y; 46A,C,D,E,F,G,H,K,L,M,N,P,Q,R,S,T,W,Y; 74C,D,E,F,G,H,I,K,L,M,N,P,Q,S,T,V,W,Y; 99C,D,E,F,G,H,I,K,L,M,P,Q,R,S,T,V,W,Y; 100A,C,E,F,G,H,I,K,L,M,N,P,Q,R,T,V,W,Y; 102A,C,D,E,F,G,H,I,K,M,N,P,Q,R,S,T,W,Y; 103A,C,D,F,G,H,I,K,L,M,N,Q,R,S,T,V,W,Y; 104A,C,D,E,G,H,I,K,M,N,P,Q,R,S,T,V,W,Y; 107A,C,D,E,F,G,H,I,K,L,M,P,R,S,V,W,Y; 109A,C,D,E,F,G,H,I,K,L,N,P,Q,R,T,V,W,Y; 110A,C,D,E,F,G,I,K,L,M,N,P,O,R,T,W,Y; 111A,C,D,F,G,H,I,K,L,M,P,R,S,T,V,W,Y; 112C,D,E,F,G,H,I,K,L,M,N,P,Q,R,V,W,Y; 116A,C,D,E,G,H,K,N,P,Q,R,S,T,V,W,Y; 155C,D,E,F,G,H,I,K,L,M,P,Q,R,T,V,W,Y; 154fA,C,D,E,F,G,I,K,L,M,N,P,Q,R,S,T,V,W,Y; 156A,C,D,E,F,H,I,K,L,M,N,Q,R,S,T,V,W,Y; 157A,C,D,F,G,H,I,K,L,M,N,P,R,T,V,W,Y; 187A,C,E,F,G,H,I,L,M,N,P,Q,T,V,W,Y; 187aA,C,D,E,F,G,H,I,K,L,M,N,P,Q,R,S,W,Y; 199C,D,E,F,G,H,I,K,L,M,O,R,S,T,V,W,Y; 204A,C,D,E,F,G,H,I,K,M,P,Q,R,S,T,V,W,Y; 207C,E,F,G,I,K,L,M,N,P,Q,R,S,T,V,W,Y; 216A,C,D,E,F,G,H,I,K,L,M,N,P,Q,R,V,W,Y; 217A,C,F,G,H,I,K,L,M,N,P,Q,R,S,T,V,W,Y; 230A,C,D,E,F,G,H,I,K,M,N,P,Q,R,S,T,W,Y; 235eA,C,D,F,G,H,I,K,L,M,N,P,R,S,T,V,W,Y; 248A,C,D,E,F,G,H,K,L,M,N,P,Q,S,T,W,Y; 286A,C,D,E,F,I,K,L,M,N,P,Q,S,T,V,W,Y; 287A,C,D,E,F,G,H,I,L,M,N,P,R,T,V,W,Y; 288C,D,E,F,G,H,I,K,L,M,N,Q,R,S,T,V,W,Y; 323A,C,D,E,F,G,H,K,L,M,N,P,Q,R,S,T,W,Y; 324C,D,E,F,G,H,I,K,L,M,N,Q,R,S,T,V,W,Y; 327C,D,E,G,H,K,M,N,P,Q,R,T,V,W,Y; 332A,C,D,E,G,H,I,K,L,M,N,P,Q,R,S,T,V,W; 333A,C,D,E,F,G,H,I,K,L,M,Q,R,S,T,V,W,Y; 344A,C,D,F,G,H,I,K,L,M,N,P,Q,S,T,V,W,Y; 346A,C,D,E,F,G,H,I,K,L,M,N,Q,S,T,V,W,Y; 348A,C,D,E,G,H,I,K,L,M,N,P,Q,R,S,T,V,Y; 349C,D,E,F,G,H,I,K,M,N,P,Q,R,T,W,Y; 350C,E,F,G,H,I,L,M,N,P,Q,R,S,T,V,W,Y; and 395A,C,D,F,G,H,I,L,M,N,P,Q,R,S,V,W,Y.

A variant of a parent phytase, comprising a substitution in at least one position selected from the group consisting of: 20; 29; 45; 63; 69; 95; 97; 98; 99; 102; 114; 118; 125; 163; 183; 187; 194; 199; 205; 218; 234; 248; 323; 324; 328; 330; 334; 343; 344; 345; 347; 349; 350; 384; 395; 398; 421; 422; 423; 424; 425; 426; 427; 428; 429; 430; wherein (a) the variant has phytase activity; (b) each position corresponds to a position of SEQ ID NO: 2; and (c) the variant has a percentage of identity to SEQ ID NO: 2 of at least 75%; and (d) with the proviso that the variant is not a variant of the *Peniophora lycii* CBS 686.96 phytase selected from the group consisting of D29S; T45R; A99N; L102V; D187R,K,S; ()187aV,T; N199A,P; V248R,1; V3231; P324A; E344R; V349L,A,S; D350K,A; and E395K,T.

A variant of a parent phytase, comprising a substitution in at least one position of at least one region selected from the group of regions consisting of: 20; 29; 37-48; 63; 69; 73-83; 91-119; 123-126; 154-157; 163; 183; 187; 194; 199; 204-218; 229-238; 248; 284-289; 300-308; 323-335; 343-350; 384; 395; 398; 407-412; and 419-430; wherein (a) the variant has phytase activity; and (b) each position corresponds to a position of SEQ ID NO: 2; and (c) the variant has a percentage of identity to SEQ ID NO: 2 of at least 75%; and wherein the variant in position 29, 45, 99, 102, 187, 187a, 199, 248, 323, 324, 344, 349, 350, and 395 is selected from the group consisting of:
29A,C,E,F,G,H,I,K,L,M,N,P,Q,R,T,V,W,Y; 45A,C,D,E,F,G,H,I,K,L,M,N,P,Q,S,V,W,Y; 99C,D,E,F,G,H,I,K,L,M,P,Q,R,S,T,V,W,Y; 102A,C,D,E,F,G,H,I,K,M,N,P,Q,R,S,T,W,Y; 187A,C,E,F,G,H,I,L,M,N,P,Q,T,V,W,Y; 187aA,C,D,E,F,G,H,I,K,L,M,N,P,Q,R,S,W,Y; 199C,D,E,F,G,H,I,K,L,M,Q,R,S,T,V,W,Y; 248A,C,D,E,F,G,H,K,L,M,N,P,Q,S,T,W,Y; 323A,C,D,E,F,G,H,K,L,M,N,P,Q,R,S,T,W,Y; 324C,D,E,F,G,H,I,K,L,M,N,Q,R,S,T,V,W,Y; 344A,C,D,F,G,H,I,K,L,M,N,P,Q,S,T,V,W,Y; 349C,D,E,F,G,H,I,K,M,N,P,Q,R,T,W,Y; 350C,E,F,G,H,I,L,M,N,P,Q,R,S,T,V,W,Y; and 395A,C,D,F,G,H,I,L,M,N,P,Q,R,S,V,W,Y.

The variant of any of the above embodiments which comprises at least one of the substitutions selected from the group consisting of: 20R; 29N,C,T; 45A; 63N; 69A; 92L; 931; 95R; 97V; 98G; 99D; 1021; 110Y; 114A; 118A; 125D; 163P; 183K; 187G; 194R; 199S; 205S; 218T; 234K,S; 248A; 323A; 324S,L; 328T; 330R; 334E,D,G; 343N; 344A,V,S; 345D,H; 347Q,P; V349M; D350V,T; 384S,A; 395G; 398T; 409S; 421 R,S; 422R; 423N; 424S; 425L; 426E; 427G; 428R; 4291; and 430M

The variant of any of the above embodiments which variant comprises a substitution or a combination of substitutions selected from the following group of substitutions and combinations of substitutions:
20R+95R+97V+98G; 29N+1021; 29N+118A; 29N+234S; 29N+324S; 29N+163P+324S; 29N+163P+324S+395G; 29S; 29S + 205S; 29S+125D+324A; 29S+324A+350V;
29S+125D+324A+350V; 29S+125D+324A+234K+330R; 29S+125D+324A+330R+395G;
29S+99D+125D+324A+334E+350V;29S+125D+324A+218T+344S+350V;
29S+125D+324A+234K+330R+395G;
29S+324A+125D+350V+63N+218T+334E+183K+421S;
29S+324A+125D+350V+45A+69A+99D+110Y+334E+344S;29T+324L;
45A+350V+421 R+422R+423N+424S+425L+426E+427G+428R+4291+430M; 69A+334E;
99D+398T; 114A+187G+194R+324S; 114A+187G+194R+324S; 218T+334G; 323A+324S;
324S; 324S+328T; 324S+395G; 324S+345D+347Q; 330R; 334E+344A;
334D+343N+344V; 345H+347P+350V; and 350V

A variant of a parent phytase, comprising a substitution in at least one position of at least one region selected from the group of regions consisting of: 20; 29; 37-40; 45; 47-48; 63; 69; 73-83; 91-98; 99; 102; 103; 110; 113-119; 123-125; 154-157; 163; 183; 187; 194; 199; 204-218; 229-238; 248; 284-289; 300-308; 323-324; 328-335; 343-347; 349; 350; 384; 395; 398; 407-412; and 419-430; wherein
(a) the variant has phytase activity; and
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of: D29S; T45R; K74R,A; A99N; L102V; P103E; H110S,V; M116F,I,L; S155N; ()154fH; G156P; E1570,S; D187R,K,S; ()187aV,T; N199A,P; L204N; A207D,H; S216T; D217E; L230V; ()235eE,Q; V248R,1; G286R,H; Q287K,S; A288P; V3231; P324A; F332Y; N333P; E344R; R346P; V349L,A,S; D350K,A; and E395K,T.

A variant of a parent phytase, comprising a substitution in at least one position of at least one region selected from the group of regions consisting of: 20; 29; 37-48; 63; 69; 73-83; 91-119; 123-126; 152-163; 183-199; 204-218; 229-238; 248; 284-289; 300-308; 323-335; 343-350; 384; 395; 398; 407-412; and 419-430; wherein
(a) the variant has phytase activity; and
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of: D29S; E42D,K,A; T45R; V461; K74R,A; A99N; D100S; L102V; P103E; F104L; N107T,Q; S109M; H110S,V; Q111E,N; T112A,S; M116F,I,L; ()154fH; S155N; G156P; E157Q,S; V184G; D185E; ()185eT; G186S,A; D187R,K,S; ()187aV,T; E188Q,S,A,V; S189E; V195L; N199A,P; L204N; A207D,H; S216T; D217E; L230V; ()235eE,Q; V248R,1; G286R,H; Q287K,S; A288P; V3231; P324A; A327S,F,I,L; F332Y; N333P; E344R; R346P; W348F; V349L,A,S; D350S,V; and E395K,T.

A variant of a parent phytase, comprising a substitution in at least one position selected from the group consisting of:
20; 29; 37; 38; 39; 40; 41; 42; 43; 44; 45; 46; 47; 48; 63; 69; 73; 74; 75; 76; 77; 78; 79; 80; 81; 82; 83; 91; 92; 93; 94; 95; 96; 97; 98; 99; 100; 101; 102; 103; 104; 105; 106; 107; 108; 109; 110; 111; 112; 113; 114; 115; 116; 117; 118; 119; 123; 124; 125; 126; 152; 153; 154; 155; 156; 157; 158; 159; 160; 161; 162; 163; 183; 184; 185; 186; 187; 188; 189; 190; 191; 192; 193; 194; 195; 196; 197; 198; 199; 204; 205; 206; 207; 208; 209; 210; 211, 212; 213; 214; 215; 216; 217; 218; 229; 230; 231; 232; 233; 234; 235; 236; 237; 238; 248; 284; 285; 286; 287; 288; 289; 300; 301; 302; 303; 304; 305; 306; 307; 308; 323; 324; 325; 326; 327; 328; 329; 330; 331; 332; 333; 334; 335; 343; 344; 345; 346; 347; 348; 349; 350; 384; 395; 398; 407; 408; 409; 410; 411; 412; 419; 420; 421; 422; 423; 424; 425; 426; 427; 428; 429; 430; wherein
   (a) the variant has phytase activity;
   (b) each position corresponds to a position of SEQ ID NO: 2; and
   (c) the variant has a percentage of identity to SEQ ID NO: 2 of at least 75%; and
   (d) with the proviso that the variant is not a variant of the *Peniophora lycii* CBS 686.96 phytase selected from the group consisting of: D29S; E42D,K,A; T45R; V461; K74R,A; A99N; D100S; L102V; P103E; F104L; N107T,Q; S109M; H110S,V; Q111E,N; T112A,S; M116F,I,L; ()154fH; S155N; G156P; E157Q,S; V184G; D185E; ()185eT; G186S,A; D187R,K,S; ()187aV,T; E188QSAV; S189E; V195L; N199A,P; L204N; A207D,H; S216T; D217E; L230V; ()235eE,Q; V248R,1; G286R,H; Q287K,S; A288P; V3231; P324A; A327S,F,I,L; F332Y; N333P; E344R; R346P; W348F; V349L,A,S; D350S,V; and E395K,T.

A variant of a parent phytase, comprising a substitution in at least one position of at least one region selected from the group of regions consisting of: 20; 29; 37-48; 63; 69; 73-83; 91-119; 123-126; 152-163; 183-199; 204-218; 229-238; 248; 284-289; 300-308; 323-335; 343-350; 384; 395; 398; 407-412; and 419-430; wherein
(a) the variant has phytase activity; and
(b) each position corresponds to a position of SEQ ID NO: 2; and
(c) the variant has a percentage of identity to SEQ ID NO: 2 of at least 75%; and wherein the variant in position 29, 42, 45, 46, 74, 99, 100, 102, 103, 104, 107, 109, 110, 111, 112, 116, 154, 155, 156, 157, 184, 185, 185e, 186, 187, 187a, 188, 189, 195, 199, 204, 207, 216, 217, 230, 235e, 248, 286, 287, 288, 323, 324, 327, 332, 333, 344, 346, 348, 349, 350, 395 is selected from the group consisting of:
   29A,C,E,F,G,H,I,K,L,M,N,P,Q,R,T,V,W,Y; 42C,F,G,H,I,L,M,N,P,Q,R,S,T,V,W,Y; 45A,C,D,E,F,G,H,I,K,L,M,N,P,Q,S,V,W,Y; 46A,C,D,E,F,G,H,K,L,M,N,P,Q,R,S,T,W,Y; 74C,D,E,F,G,H,I,K,L,M,N,P,Q,S,T,V,W,Y; 99C,D,E,F,G,H,I,K,L,M,P,Q,R,S,T,V,W,Y; 100A,C,E,F,G,H,I,K,L,M,N,P,Q,R,T,V,W,Y; 102A,C,D,E,F,G,H,I,K,M,N,P,Q,R,S,T,W,Y; 103A,C,D,F,G,H,I,K,L,M,N,Q,R,S,T,V,W,Y; 104A,C,D,E,G,H,I,K,M,N,P,Q,R,S,T,V,W,Y; 107A,C,D,E,F,G,H,I,K,L,M,P,R,S,V,W,Y; 109A,C,D,E,F,G,H,I,K,L,N,P,Q,R,T,V,W,Y; 110A,C,D,E,F,G,I,K,L,M,N,P,Q,R,T,W,Y; 111A,C,D,F,G,H,I,K,L,M,P,R,S,T,V,W,Y; 112C,D,E,F,G,H,I,K,L,M,N,P,Q,R,V,W,Y; 116A,C,D,E,G,H,K,N,P,Q,R,S,T,V,W,Y; 154fA,C,D,E,F,G,I,K,L,M,N,P,Q,R,S,T,V,W,Y; 155A,C,D,E,F,G,H,I,K,L,M,P,Q,R,T,V,W,Y; 156A,C,D,E,F,H,I,K,L,M,N,Q,R,S,T,V,W,Y; 157A,C,D,F,G,H,I,K,L,M,N,P,R,T,V,W,Y; 184A,C,D,E, F,H,I,K,L,M,N,P,Q,R,S,T,W,Y; 185A,C,F,G,H,I,K,L,M,N,P,Q,R,S,T,V,W,Y; 185eA,C,D,E, F,G,H,I,K,L,M,N,P,Q,R,S,V,W,Y; 186C,D,E, F,H,I,K,L,M,N,P,Q,R,T,V,W,Y; 187A,C,E,F,G,H,I,L,M,N,P,Q,T,V,W,Y; 187aA,C,D,E,F,G,H,I,K,L,M,N,P,Q,R,S,W,Y; 188C,D,F,G,H,I,K,L,M,N,P,R,T,W,Y; 189A,C,D,F,G,H,I,K,L,M,N,P,Q,R,T,V,W,Y; 195 A,C,D,E, F,G,H,I,K,M,N,P,Q,R,S,T,W,Y; 199C,D,E,F,G,H,I,K,L,M,Q,R,S,T,V,W,Y; 204A,C,D,E,F,G,H,I,K,M,P,Q,R,S,T,V,W,Y; 207C,E,F,G,I,K,L,M,N,P,Q,R,S,T,V,W,Y; 216A,C,D,E,F,G,H,I,K,L,M,N,P,Q,R,V,W,Y; 217A,C,F,G,H,I,K,L,M,N,P,Q,R,S,T,V,W,Y; 230A,C,D,E,F,G,H,I,K,M,N,P,Q,R,S,T,W,Y; 235eA,C,D,F,G,H,I,K,L,M,N,P,R,S,T,V,W,Y; 248A,C,D,E,F,G,H,K,L,M,N,P,Q,S,T,W,Y; 286A,C,D,E,F,I,K,L,M,N,P,Q,S,T,V,W,Y; 287A,C,D,E,F,G,H,I,L,M,N,P,R,T,V,W,Y; 288C,D,E,F,G,H,I,K,L,M,N,Q,R,S,T,V,W,Y; 323A,C,D,E,F,G,H,K,L,M,N,P,Q,R,S,T,W,Y; 324C,D,E,F,G,H,I,K,L,M,N,Q,R,S,T,V,W,Y; 327C,D,E,G,H,K,M,N,P,Q,R,T,V,W,Y; 332A,C,D,E,G,H,I,K,L,M,N,P,Q,R,S,T,V,W; 333A,C,D,E,F,G,H,I,K,L,M,Q,R,S,T,V,W,Y; 344A,C,D,F,G,H,I,K,L,M,N,P,Q,S,T,V,W,Y; 346A,C,D,E,F,G,H,I,K,L,M,N,Q,S,T,V,W,Y; 348A,C,D,E,G,H,I,K,L,M,N,P,Q,R,S,T,V,Y; 349C,D,E,F,G,H,I,K,M,N,P,Q,R,T,W,Y; 350A,C,D,E,F,G,H,I,K,L,M,N,P,Q,R,T,W,Y; and 395A,C,D,F,G,H,I,L,M,N,P,Q,R,S,V,W,Y.

A variant of a parent phytase, comprising a substitution in at least one position selected from the group consisting of: 20; 29; 45; 63; 69; 92; 93; 95; 97; 98; 99; 102; 110; 114; 118; 125; 152; 156; 157; 160; 163; 183; 185; 187; 194; 199; 205; 218; 234; 248; 286; 323; 324; 328; 330; 334; 343; 344; 345; 347; 349; 350; 384; 395; 398; 409; 421; 422; 423; 424; 425; 426; 427; 428; 429; 430; wherein
(a) the variant has phytase activity;
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of D29S; T45R; A99N; L102V; H110S,V; G156P; E157Q,S; D185E; ()185eT; D187R,K,S; ()187aV,T; N199A,P; V248R,1; G286R,H; V3231; P324A; E344R; V349L,A,S; D350S,V; and E395K,T.

A variant of a parent phytase, comprising a substitution in at least one position selected from the group consisting of: 20; 29; 45; 63; 69; 92; 93; 95; 97; 98; 99; 102; 110; 114; 118; 125; 152; 156; 157; 160; 163; 183; 185; 187; 194; 199; 205; 218; 234; 248; 286; 323; 324; 328; 330; 334; 343; 344; 345; 347; 349; 350; 384; 395; 398; 409; 421; 422; 423; 424; 425; 426; 427; 428; 429; 430; wherein
(a) the variant has phytase activity; and
(b) each position corresponds to a position of SEQ ID NO: 2; and
(c) the variant has a percentage of identity to SEQ ID NO: 2 of at least 75%; and wherein the variant in position 29, 45, 99, 102, 110; 156; 157; 185; 185e; 187, 187a, 199, 248, 286; 323, 324, 344, 349, 350, and 395 is selected from the group consisting of:
   29A,C,E,F,G,H,I,K,L,M,N,P,Q,R,T,V,W,Y; 45A,C,D,E,F,G,H,I,K,L,M,N,P,Q,S,V,W,Y; 99C,D,E,F,G,H,I,K,L,M,P,Q,R,S,T,V,W,Y; 102A,C,D,E,F,G,H,I,K,M,N,P,Q,R,S,T,W,Y; 110A,C,D,E,F,G,I,K,L,M,N,P,Q,R,T,W,Y; 156A,C,D,E,F,H,I,K,L,M,N,Q,R,S,T,V,W,Y; 157A,C,D,F,G,H,I,K,L,M,N,P,R,T,V,W,Y; 185A,C,F,G,H,I,K,L,M,N,P,Q,R,S,T,V,W,Y; 185eA,C,D,E,F,G,H,I,K,L,M,N,P,Q,R,S,V,W,Y; 187A,C,E,F,G,H,I,L,M,N,P,Q,T,V,W,Y; 187aA,C,D,E,F,G,H,I,K,L,M,N,P,Q,R,S,W,Y; 199C,D,E,F,G,H,I,K,L,M,Q,R,S,T,V,W,Y; 248A,C,D,E,F,G,H,K,L,M,N,P,Q,S,T,W,Y; 286A,C,D,E,F,G,I,K,L,M,N,P,Q,S,T,V,W,Y; 323A,C,D,E,F,G,H,K,L,M,N,P,Q,R,S,T,W,Y; 324C,D,E,F,G,H,I,K,L,M,N,Q,R,S,T,V,W,Y; 344A,C,D,F,G,H,I,K,L,M,N,P,Q,S,T,V,W,Y; 349C,D,E,F,G,H,I,K,M,N,P,Q,R,T,W,Y; 350A,C,D,E,F,G,H,I,K,L,M,N,P,Q,R,T,W,Y; and 395A,C,D,F,G,H,I,L,M,N,P,Q,R,S,V,W,Y.

The variant of any one of the above embodiments, which comprises at least one of the substitutions selected from the group consisting of: 20R; 29N,C,T; 45A; 63N; 69A; 92L; 931; 95R,H,M,N,S,T; 97V; 98G; 99D; 1021; 110Y,R,W; 114A; 118A; 125D; 152A; 156C,R; 160R; 163P; 183K,A,W,G; 185A,H,N,P; 187G; 191L; 194R; 199S; 205S; 218T; 234K,S; 248A,C,D,E,G,H,S; 286S; 323A; 324S,L; 328T; 330R; 334E,D,G; 343N; 344A,V,S; 345D,H; 347Q,P; V349M; D350T; 384S,A; 395G; 398T; 409S; 421 R,S; 422R; 423N; 424S; 425L; 426E; 427G; 428R; 4291; and 430M.

The variant of any one of the above embodiments, which comprises a substitution or a combination of substitutions selected from the following group of substitutions and combinations of substitutions:
20R+95R+97V+98G; 29N+1021; 29N+118A; 29N+163P+324S; 29N+163P+324S+395G; 29N+234S; 29N+324S; 29S; 29S+45A+69A+99D+110Y+125D+324A+334E+344S+350V; 29S+63N +125D+183K+218T+324A+334E+350V+421S;
29S+95H+110W+183W+324A+350V; 29S+95M+110W+183W+324A+350V; 29S+95N+110R+183W+324A+350V; 29S+95R+110W+183W+324A+350V; 29S+95S+110R+183W+324A+350V; 29S+95S+110W+183W+324A+350V; 29S+95T+110R+183W+324A+350V; 29S+95T+110Y+125D+183W+286S+324A+350V; 29S+95T+110Y+152A+156C+157Q+160R+183W+324A+350V;
29S+95T+110Y+156R+157Q+183W+324A+350V;
29S+95T+110Y+183W+185A+248A+324A+350V;
29S+95T+110Y+183W+185A+248C+324A+350V;
29S+95T+110Y+183W+185A+248E+324A+350V;
29S+95T+110Y+183W+185A+248H+324A+350V;
29S+95T+110Y+183W+185H+248G+324A+350V;
29S+95T+110Y+183W+185P+248G+324A+350V;
29S+95T+110Y+183W+185P+248S+324A+350V;
29S+95T+110Y+183W+191L+324A+350V;29S+95T+110Y+183W+248D+324A+350V; 29S+95T+110Y+183W+324A;29S+95T+110Y+183W+324A+350V; 29S+99D+125D+324A+334E+350V;29S+110Y+183W+185N+324A+350V; 29S+125D+218T+324A+344S+350V;29S+125D+234K+324A+330R; 29S+125D+234K+324A+330 R+395G;29S+125D+324A;29S+125D+324A+330 R+395G; 29S+125D+324A+350V;29S+183A+324A+350V;29S+183G+324A+334E+344S+350V; 29S+183W+324A+350V; 29S+205S; 29S+324A+350V; 29T+324L;
45A+350V+421 R+422R+423N+424S+425L+426E+427G+428R+4291+430M; 69A+334E; 99D+398T; 114A+187G+194R+324S; 218T+334G; 323A+324S; 324S; 324S+328T; 324S+345D+347Q; 324S+395G; 330R; 334D+343N+344V; 334E+344A; 345H+347P+350V; and 350V.

### Examples

Chemicals used were commercial products of at least reagent grade.

### Example 1: Preparation of PE-variants, Screening and Isolation of Variants of Improved Thermostability and/or Specific Activity

### General principles

Using the phytase of *Peniophora lycii* CBS 686.96 as a parent phytase, and on the basis of the regions of interest described herein, various phytase variant libraries were constructed and expressed in yeast (dope libraries, and spiked oligo shuffling libraries, examples of which are described below).

Screening for improved thermostability: The yeast libraries were subjected to a primary screening as described below (replica, single filter) to select those colonies which show activity at 53°C.

Colonies showing activity at 53°C were transferred to the secondary screening as also described below (24 well plate cultivation) to establish a temperature profile (activity at 37°C, 60°C, 62°C, 64°C, 66°C, 68°C, 70°C, 72°C etc., as applicable) for each colony.

For colonies performing better than the wild type parent *Peniophora* phytase, the plasmids were isolated and re-transformed into yeast, and the temperature profile experiment was repeated. From the temperature profile experiments the following figures are calculated: "Relative Activity at 60°C/37°C," and/or "Relative activity 62°C/37°C," and/or "Relative activity 65°C/37°C," and/or "Relative activity 68°C/37°C," and/or "Relative activity 70°C/37°C," and/or "Relative activity 72°C/37°C," and so forth. In these ratios, the temperature first mentioned may be designated "the elevated temperature of choice." These terms are defined as the ratio of the phytase activity at the elevated temperature (e.g. 60 or 62°C), relative to the phytase activity at the lower temperature (37°C), based on the phytase activity values obtained for the supernatants in the secondary screening method (temperature profile measurements). Colonies in which one or more of these relative activites are improved as compared to the parent phytase were selected, the plasmid in question was sequenced, and the corresponding variant identified as an improved phytase variant of the invention.

In a second screening round, a thermostable phytase variant was selected as a new parent phytase (backbone) and the above was repeated (temperatures adjusted as appropriate on the basis of the performance of this new backbone).

Furthermore, combination variants were constructed, and some potential hot spots randomized, and variants tested.

In a third screening round, a further improved new thermostable phytase variant was selected as a new backbone, and the above was repeated (temperatures again adjusted in an upward direction, e.g. the first screening round temperature was now increased to 63°C. Furthermore, still some new combination variants were construed, and other apparently interesting sites were randomized to find an optimal pair of substitutions.

Further variants were prepared as generally described above.

Screening for improved specific activity: In the primary screening, the yeast library was spread on SC-glucose plate containing Ca-phytate, and cultivated for 3 days at 30°C. Clones with larger halos were selected and cultivated in 24 well plates containing YPD medium. In the secondary screening, the activity of the supernatants in wells was checked relatively and the clones with higher activity were selected. The selected clones were checked for remaining activity on plates after incubation at 80°C, pH4 for 30min to deselect variants with lower thermostability. Clones with high thermostability were selected and cultivated again in 24 well plates for semi-purification. Semi-purification was conducted by binding phytase to conA-sepharose 4B (Concanavalin A in Sepharose 4B Gel matrix from Amersham bioscience, Cat. No. 17-0440-01) and eluting it by 0.5M methyl-alpha-D-mannopyranoside in PBS + 0.1 % tween20 (PBS is 8g/l NaCI, 0.2g/l KCI, 2.68g/l Na₂HPO₄-7H₂O, 0.24g/l KH₂PO₄ (pH7.4); and tween20 is commercially available from Bio-Rad, Cat. no. 170-6531). The semi-purified supernatants were then engaged in rocket immuno electrophoresis analysis after having adjusted the activity to 30FYT/ml. For the rocket immuno electrophoresis a polyclonal antibody raised in rabbits against purified *Peniophora* phytase was used. The clones with smaller signals were selected as variants with higher specific activity (the area under the rocket being proportional to the concentration of the antigen (the phytase), ie. the smaller the area, the less phytase protein, and the higher the specific activity).

Examples of improved variants are shown in Tables 1-5 below. These variants exhibit a higher specific activity and/or are more thermostable than the *Peniophora* parent phytase. In particular, as regards thermostability, the variants exhibit higher values for "Relative Activity at 60°C / 37°C," and/or "Relative activity 62°C/37°C," and/or "Relative activity 65°C/37°C," and/or "Relative activity 68°C/37°C," and/or "Relative activity 70°C/37°C," and/or "Relative activity 72°C/37°C," and/or "Relative Activity at 74°C / 37°C," etc. (see these results shown in Tables 6-1 and 6-2). Selected variants resulting from the specific activity screening method are shown in Table 6-3 below. All of these variants are expectedly of an even higher specific activity than the variant designated "N" in Table 8.

Selected variants were expressed in *Aspergillus oryzae* IFO 4177 which is disclosed in WO 97/35956 and the transformed cells were cultivated in the medium MDU-2Bp at 25°C at 220 rpm. The phytase variants were purified from the fermentation broth and further characterized as regards thermostability and/or specific activity as described in Example 2 and shown in Tables 7-8.

**Table 1**

| **Phytase variant** | **Substitutions** |
|---|---|
| 1 | D29N+L163P+D187G+G194R+P324S |
| 2 | A99D+A398T |
| 3 | A334D+D343N+E344V |
| 4 | T114A+D187G+G194R+P324S |
| 5 | D29N |
| 6 | D29N+L1021 |
| 7 | D29N+N234S |
| 8 | P324S+A328T |
| 9 | D29S+N205S |
| 10 | D29S+P324A+D350V+E183W+K95R+H110W |
| 11 | N234K |
| 12 | V323A+P324S |
| 13 | Q20R+K95R+G97V+V98G |
| 14 | T45A+D350V+delete (P421-E423) +insert (P421 RRNSLEGRIM) |
| 15 | D29S+P324A+D350V+E183W+K95T+H110Y+D185K+V248T+V349L+ D350M |
| 16 | D29N+T118A |
| 17 | P324S+N345D+L347Q |
| 18 | N199S+V248A |
| 19 | D29T+P324L |
| 20 | D350V |
| 21 | A218T+A334G |
| 22 | L163P |
| 23 | V931+N409S+P324S |
| 24 | A334E+E344A |
| 25 | P324S+A328T |
| 26 | G330R |
| 27 | V248A |
| 28 | V69A+A334E |
| 29 | T45A+A218T |

**Table 2**

| **Phytase Variant** | **Substitutions** |
|---|---|
| 30 | D29N+ L163P+P324S+E395G |
| 31 | D29S+E125D+P324A+A334E+E344S+P421S |
| 32 | D29S+P324A+D350V+E183W+K95T+H110Y+D185K+V248T+V349L + D350I |
| 33 | D29S+K95T+H110Y+E183W+D185K+V248S+P324A+D350V |
| 34 | D29S+E125D+P324A+N234K+G330R |
| 35 | D29N+P324S |
| 36 | D29S+E125D+P324A+G330R+E395G |
| 37 | D29S+E125D+P324A+N234K+E395G |
| 38 | D29S+P324A+E125D+D350V |
| 39 | D29S+P324A+E125D+D350V+T45A+V69A+A99D+H110Y+A334E+ E344S |
| 40 | D29N+L163P+P324S |
| 41 | D29S+K95T+D350V+E183W+H110Y+D185E+V248S+P324A |
| 42 | P324S+E395G |
| 43 | D29S+P324A+E125D |

**Table 3**

| **Phytase Variant** | **Substitutions** |
|---|---|
| 44 | D29S+E125D+P324A+N234K+G330R+E395G |
| 45 | D29S+P324A+E125D |
| 46 | D29T |
| 47 | D29S+P324A+D350V+E183W+K95T+H110Y+D185K+V248T+V349A+ D350L |
| 48 | D29S+P324A+E125D+D350V+A99D+A334E |
| 49 | P324S |
| 50 | D29S+T45A+V248A+P324A+V349M+D350V+E395G |
| 51 | D29S+K95T+H110Y+E183W+D185H+D187S+T190K+T191S+S210G+ M2221+V248G+P324A+D350V |
| 52 | D29S |
| 53 | D29S+P324A+D350V+E183W+K95T+H110Y+D185P+V248G |

**Table 4**

| **Phytase Variant** | **Substitutions** |
|---|---|
| 54 | D29S+P324A+D350V+E183W+K95M+H110W |
| 55 | D29C |
| 56 | D29S+P324A+D350V+E183W+K95T+H110Y+D185K+V248G+V349K+ D350L |
| 57 | D29S+P324A+D350V+E183W+H110Y+D185N |
| 58 | D29S+P324A+E125D+D350V+A99D+A334E |
| 59 | N345H+L347P+D350V |
| 60 | D29S+P324A+E125D+D350T |
| 61 | D29S+P324A+E125D+D350V+N345H+L347P |
| 62 | P324A |
| 63 | D29S+K95R+D350V |
| 64 | D29S+K95T+H110Y+E157K+E183W+D185H+D187R+T190S+T191R+ V248G+P324A+D350V |
| 65 | D29S+P324A+E125D+D350V+E344S+A218T |
| 66 | F92L |
| 67 | D29S+P324A+D350V |

**Table 5**

| **Phytase Variant** | **Substitutions** |
|---|---|
| 68 | D29S+P324A+D350V+E183W+K95T+H110Y+T191L |
| 69 | D29S+P324A+D350V+E183W |
| 70 | D29S+P324A+E183W+K95T+H110Y |
| 71 | D29S+P324A+E125D+D350V+G63N+A218T+A334E+E183K+P421S |
| 72 | D29S+P324A+D350V+E183W+K95T+H110Y+D185P+V248S |
| 73 | D29S+P324A+D350V+E183W+K95T+H110Y+E125D+G286S |
| 74 | D29S+P324A+E125D+D350V |
| 75 | D29S+P324A+D350V+E183G+A334E+E344S |
| 76 | D29S+P324A+D350V+E183W+K95T+H110R |
| 77 | D29S+K95T+H110Y+E183W+D185H+V248G+T321A+V323A+P324A+ D350V |
| 78 | D29S+P324A+D350V+E183W+K95T+H110Y+D185H+V248G |
| 79 | D29S+P324A+D350V+E183W+K95T+H110Y+G152A+G156C+E157Q+ L160R |
| 80 | D29S+P324A+D350V+E183W+K95T+H110Y+D185A+V248A |
| 81 | D29S+P324A+D350V+E183W+K95T+H110Y |
| 82 | D29S+P324A+D350V+E183W+K95H+H110W |
| 83 | D29S+P324A+D350V+E183A |
| 84 | D29S+P324A+D350V+E183W+K95N+H110R |
| 85 | D29S+K95T+H110Y+E183W+D185K+V248T+ P324A+D350V |
| 86 | D29S+P324A+D350V+E183W+K95S+H110W |
| 87 | D29S+P324A+D350V+E183W+K95T+H110Y+D185A+V248E |
| 88 | D29S+P324A+D350V+E183W+K95T+H110Y+G156R+E157Q |
| 89 | D29S+P324A+D350V+E183W+K95T+H110Y+D185A+V248H |
| 90 | D29S+P324A+D350V+E183W+K95T+H110Y+D185A+V248C |
| 91 | D29S+P324A+D350V+E183W+K95S+H110R |
| 92 | D29S+P324A+D350V+E183W+K95T+H110Y+V248D |

**Table 6-1**

| **Phytase Variant** | **Relative Activity 60°C/37° C** | **Relative Activity 62°C/37° C** | **Relative Activity 65°C/37° C** | **Relative Activity 68°C/37° C** | **Relative Activity 70°C/37° C** | **Relative Activity 72°C/37° C** | **Relative Activity 74°C/37° C** |
|---|---|---|---|---|---|---|---|
| *Penio-phora* parent phytase | 70 | 30 | 15 | - | - | - | - |
| 1 | 104 | 55 | - | - | - | - | - |
| 2 | 95 | 50 | - | - | - | - | - |
| 3 | 120 | 70 | - | - | - | - | - |
| 4 | 120 | 70 | - | - | - | - | - |
| 5 | 195 | 175 | - | - | - | - | - |
| 6 | 105 | 40 | - | - | - | - | - |
| 7 | 94 | 47 | - | - | - | - | - |
| 8 | 115 | 65 | - | - | - | - | - |
| 9 | 118 | 69 | - | - | - | - | - |
| 10 | 106 | 60 | - | - | - | - | - |
| 11 | 140 | 107 | - | - | - | - | - |
| 12 | 150 | 130 | 80 | - | - | - | - |
| 13 | 104 | 45 | - | - | - | - | - |
| 14 | 130 | 75 | - | - | - | - | - |
| 15 | 141 | 123 | - | - | - | - | - |
| 16 | 179 | 130 | - | - | - | - | - |
| 17 | 127 | 59 | - | - | - | - | - |
| 18 | 104 | 42 | - | - | - | - | - |
| 19 | 115 | 52 | - | - | - | - | - |
| 20 | 126 | 65 | - | - | - | - | - |
| 21 | 115 | 54 | - | - | - | - | - |
| 22 | 102 | 53 | - | - | - | - | - |
| 23 | 95 | 44 | - | - | - | - | - |
| 24 | 71 | 40 | - | - | - | - | - |
| 25 | 62 | 33 | - | - | - | - | - |
| 26 | 155 | 124 | - | - | - | - | - |
| 27 | - | - | - | 170, 183 | 73, 136 | 69 | 32 |
| 28 | 147 | 126 | 61 | - | - | - | - |
| 29 | - | - | - | 176 | 105 | 69 | 32 |
| 30 | - | - | - | 109 | App. 95 | - | - |
| 31 | - | - | - | 154 | 102 | - | - |
| 32 | - | - | - | - | - | 102 | 22 |
| 33 | - | - | - | - | 186 | 125 | 49 |
| 34 | 150 | 30 | - | - | - | - | - |
| 35 | 80 | 40 | - | - | - | - | - |
| 36 | - | 137 | 63 | - | - | - | - |
| 37 | - | 156 | 81 | - | - | - | - |
| 38 | - | 125 | 46 | - | - | - | - |
| 39 | - | 130 | 52 | 24 | - | - | - |
| 40 | - | 169 | 165 | 119 | - | - | - |
| 41 | - | 145 | 126 | 72 | - | - | - |
| 42 | 115 | 84 | 35 | - | - | - | - |
| 43 | 145 | 112 | 54 | - | - | - | - |
| 44 | - | - | - | 90 | App. 70 | - | - |
| 45 | - | - | - | 105 | App. 80 | - | - |
| 46 | - | - | - | 96 | App. 70 | - | - |
| 47 | - | - | - | 104 | 42 | - | - |
| 48 | - | - | - | 120 | 57 | - | - |
| 49 | - | - | - | 147 | 102 | 53 | - |
| 50 | - | - | - | 152 | 105 | 51 | - |
| 51 | - | - | - | 184 | 101 | 48 | - |
| 52 | - | - | - | - | 115 | 88 | 31 |
| 53 | - | - | - | - | 118 | 74 | 25 |
| 54 | 118 | 69 | - | - | - | - | - |

**Table 6-2**

| **Phytase variant** | **Relative ctivity 68°C/37° C** | **Relative Activity 70°C/37° C** | **Relative Activity 72°C/37° C** | **Relative Activity 74°C/37°C** | **Relative Activity 75°C/37° C** | **Relative Activity 76°C/37° C** | **Relative Activity 78°C/37°C** | **Relative Activity 80°C/37° C** | **Relative Activity 81°C/37°** C | **Relative Activity 82°C/37°C** |
|---|---|---|---|---|---|---|---|---|---|---|
| 55 | - | - | - | - | - | - | - | 92 | 66 | - |
| 56 | - | - | - | - | - | - | - | 83 | 54 | - |
| 57 | - | - | - | - | - | - | 71 | 23 | - | - |
| 58 | 172 | 122 | - | - | - | - | - | - | - | - |
| 59 | - | - | - | - | - | 90 | 34 | - | - | - |
| 60 | - | - | - | - | - | - | - | 126 | 100 | 51 |
| 61 | - | - | - | - | 95 | 51 | - | - | - | - |
| 62 | - | - | - | - | - | - | 99 | 49 | - | - |
| 63 | - | - | 113 | 101 | 23 | - | - | - | - | - |
| 64 | - | - | - | - | - | - | - | 110 | 94 | 45 |
| 65 | - | - | - | - | - | - | - | 74 | 38 | - |
| 66 | - | - | 178 | 72 | 36 | - | - | - | - | - |
| 67 | - | - | - | - | - | - | 36 | 8 | - | - |
| 68 | - | - | - | - | - | - | - | 61 | 32 | - |
| 69 | - | - | - | - | - | 96 | 40 | - | - | - |
| 70 | - | - | - | - | - | - | - | 69 | 38 | - |
| 71 | - | - | - | - | - | 102 | 52 | - | - | - |
| 72 | - | - | - | - | - | - | 104 | 50 | - | - |
| 73 | - | - | - | - | - | - | - | 88 | 61 | - |
| 74 | - | - | - | - | - | 98 | 36 | - | - | - |
| 75 | - | - | - | - | - | 71 | 27 | - | - | - |
| 76 | - | - | 131 | 110 | 60 | 42/56 | 16 | - | - | - |
| 77 | - | - | - | - | - | - | 59 | 17 | - | - |
| 78 | - | - | - | - | - | 81 | 28 | - | - | |
| 79 | - | - | - | - | - | 91 | 32 | - | - | - |
| 80 | - | - | - | - | - | 76 | 24 | - | - | - |
| 81 | 147 | 102 | 53 | - | - | - | - | - | - | - |
| 82 | - | - | - | - | - | - | - | 85 | 45 | - |
| 83 | - | - | - | - | - | - | 91 | 31 | - | - |
| 4 | - | - | - | - | - | - | - | 85 | 67 | - |
| 85 | - | - | - | - | - | - | - | 60 | - | - |
| 86 | - | - | - | - | - | - | - | 122 | 105 | 58 |
| 87 | - | - | - | - | - | - | - | 102 | 62 | - |

**Table 6-3**

| **Phytase Variant** | **Specific Activity (%)** |
|---|---|
| N | 100 |
| Q | 144 |
| R | 122 |
| S | 189 |
| T | 200 |
| U | 167 |
| V | 167 |
| W | 156 |
| X | 167 |
| Y | 133 |
| Z | 122 |

### Strains and plasmids

*E.coli* DH12S (available from Gibco BRL) is used for yeast plasmid rescue.

pTMPP2ver2 is a *S. cerevisiae* and *E.coli* shuttle vector under the control of TPI promoter, constructed from pJC039 described in WO 00/10038. It is used for library construction, yeast expression, screening and sequencing.

*Saccharomyces cerevisiae* YNG318: MATa Dpep4[cir+] ura3-52, leu2-D2, his 4-539 is used for the construction of yeast library and the expression of the phytase variants. It is described in J. Biol. Chem. 272 (15), 9720-9727, 1997).

### Media and substrates

### 10X Basal solution

| | |
|---|---|
| 66.8 g/l | Yeast nitrogen base w/o amino acids (DIFCO) |
| 100 g/l | succinate |
| 60 g/l | NaOH |

### SC-glucose

| | |
|---|---|
| 100 ml/l | 20% glucose (i.e., a final concentration of 2% = 2 g/100ml)) |
| 4 ml/l | 5% threonine |
| 10 ml/l | 1% tryptophan |
| 25 ml/l | 20% casamino acids |
| 100 ml/l | 10 X basal solution |

The above solution is sterilized using a filter of a pore size of 0.20µm. Agar and H₂O (approx. 761ml) is autoclaved together, and the separately sterilized SC-glucose solution is added to the agar solution.

### YPD

| | |
|---|---|
| 20 g/l | Bacto pepton |
| 10 g/l | yeast extract |
| 100 ml/l | 20% glucose (sterilized separately) |

### Na-phytate plate

| | |
|---|---|
| 100 ml/l | 1M Na acetate buffer (pH 5.5) |
| 5 g/l | Na phytate |
| 30 g/l | agar |

### PEG/LiAc solution

| | |
|---|---|
| 50ml | 40% PEG4000 (sterilized by autoclaving) |
| 1ml | 5M Lithium Acetate (sterilized by autoclaving) |

### Trace Metal Solution

| | |
|---|---|
| FeS0₄ x 7H₂O | 13.90 g/l |
| MnS0₄ x 5 H₂O | 13.60 g/l |
| ZnCl₂ | 6.80 g/l |
| CuS0₄ x 5 H₂O | 2.50 g/l |
| NiCl₂ x 6 H₂O | 0.24 g/l |
| Citric acid x H₂O | 3.00 g/l |

### MDU-2Bp

| | |
|---|---|
| Maltose x H₂O | 45 g/l |
| Yeast Extract | 7 g/l |
| MgSO₄ x 7H₂O | 1 g/l |
| NaCl | 1 g/l |
| K₂SO₄ | 2 g/l |
| KH₂PO₄ | 12 g/l |
| Trace Metal Solution | 0.5 ml/l |

### Methods

### Phytase activity, primary screening method

Spread yeast library onto SC-glucose plates and incubate for 3 days at 30°C. Replica to new SC-glucose plates with nitrate filters by using velvet cloth. Incubate the plates at 30°C for 1 day (or 20°C over the weekend) Transfer the filters to pre-heated 0.5% Na-phytate plates (pH5.5). Incubate the plates at the prescribed temperature, e.g. 53°C, overnight (O/N) .Remove the filters and pour 0.5M CaCl₂ solution. Find the yeast clones with clear zones. Isolate candidate clones from the master plate and inoculate to a new SC-glucose plate and 1 ml of YPD medium in 24well plates.

### Secondary screening method

Cultivate YPD medium in 24well plates at 25°C for 3 days at 180rpm. Centrifuge the plate/ or just leave them still for an hour. Apply 5ul and 10µlof supernatants to Na-phytate plates. Incubate the plates at 37°C and 53°C overnight. Check the diameter of clear zones. Check the temperature profile of the supernatant of those clones which retain activity at 53°C, using the phytase assay described in Example 2 below (assay temperature as prescribed).

### Method for construction of yeast library

Mix 0.5µlof vector (EcoRl-Notl digested) and 1µlof PCR fragments. Thaw YNG318 competent cells on ice. Mix 100µlof the cells, the DNA mixture and 10µlof carrier DNA (Clontech) in 12ml polypropylene tubes (Falcon 2059). Add 0.6ml PEG/LiAc solution and mix gently. Incubate for 30min at 30°C, and 200 rpm. Incubate for 30 min at 42°C (heat shock). Transfer to an eppendorf tube and centrifuge for 5sec. Remove the supernatant and resolve in 3ml of YPD. Incubate the cell suspension for 45min at 200rpm at 30°C. Pour the suspension to SC-glucose plates to give approx. 300clones/plate.

### Library construction

Dope libraries are constructed by SOE method (Splicing by Overlap Extension, see "PCR: A practical approach", p. 207-209, Oxford University press, eds. McPherson, Quirke, Taylor), followed by yeast recombination.

### Other methods

*E.coli* transformation for constructing libraries and subcloning is carried out by electroporation (BIO-RAD Gene Pulser). DNA Plasmids are prepared by alkaline method (Molecular Cloning, Cold Spring Harbor) or with the Qiagen^{®} Plasmid Kit. DNA fragments are recovered from agarose gel by the Qiagen gel extraction Kit. PCR is carried out by the PTC-200 DNA Engine. The ABI PRISM^{TM} 310 Genetic Analyzer is used for determination of all DNA sequences. Yeast transformation is carried out by lithium acetate method. Yeast total DNA is extracted by the Robzyk and Kassir's method described in Nucleic acids research vol.20, No.14 (1992) 3790.

### General primers for amplification and sequencing

The below primers are used to make DNA fragments containing any mutated fragments by the SOE method, or just to amplify a whole phytase gene.

| | |
|---|---|
| SEQ lD NO: 3: | |
| 620AM34 | GAGTACTATCTTGCATTTGTAC TAGGAGTTTAGTGAACTTGC |
| SEQ ID NO: 4: | |
| 680AM35 | ATGGTTATGGATTTCGGGGATTC TTCGAGCGTCCCAAAACC |
| SEQ ID NO: 5: | |
| AM34 | TAGGAGTTTAGTGAACTTGC |
| SEQ ID NO: 6: | |
| AM35 | TTCGAGCGTCCCAAAACC |
| SEQ ID NO: 7: | |
| 620 | GAGTACTATCTTGCATTTGTAC |
| SEQ ID NO: 8: | |
| 680 | ATGGTTATGGATTTCGGGGAT |

### Examples of primers for construction of dope libraries

### Region 229-236

In accordance with established practice, in the nucleotide sequences specified below, special codes for nucleotides are used. These are apparent from, e.g., http://www.cs.pitt.edu/∼vanathi/na.html). For example, K designates G or T; Y designates C or T; R designates A or G; and N designates A or C or G or T.
T229 VNN nucleotides
L230 INMKR amino acids
S231 TVN amino acids
S232 VNN nucleotides
G233 VNN nucleotides
N234 VNN nucleotides
A235 VNN nucleotides
S236 AMK amino acids or just A (difficult to get M and K)

### Distribution of nucleotides for L230lNMKR:

| Nucleotide | Base 1 | Base 2 | Base 3 |
|---|---|---|---|
| G | 0.0 | 3.0 | 46.6 |
| A | 12.5 | 6.5 | 7.7 |
| T | 0.0 | 90.4 | 45.7 |
| C | 87.5 | 0.0 | 0.0 |

The below primers are targetted at the region T229-S236:
SEQ ID NO: 9:
   Dope1 (62mer):
      GAT ATG TGC CCG TTC GAC 12K (11 )(12)(13) (14)(15)T 42K 33K 115 32K 5CC CCC TTC TGT GAC CTA TTT AC
SEQ ID NO: 10:
   Dope1 R (18mer):
   GTCGAACGGGCACATATC

   1: A91 G3 T3 C3
   2: C91 G3 A3 T3
   3: G91 C3 A3 T3
   4: T91 G3 A3 C3
   5: T95 G5
   11: C88 A12
   12: T90 G3 A7
   13: G47 A8 T45
   14: G9 A91
   15: G83 A4 T9 C4
   Primers for construction of spiked oligo shuffling libraries
SEQ lD NO: 11:
   L102T1 (43mer):
   AGTTCGGCGTCGCCGATCTGAYCCCGTTCGGGGCTAACCAATC
SEQ lD NO: 12:
   VE248+251 X (53mer):
   TATTTACCGCGGAGGAGTATVNNTCGTACVNNTACTACTATGACCTCGACAAG
SEQ lD NO: 13:
   V271Al (45mer):
   CCGGGAACGCTCTCGGTCCTRYCCAGGGCGTCGGATACGTCAATG
SEQ ID NO: 14:
   G286X (42mer):
   ATGAGCTGCTTGCACGCTTGACCVNNCAAGCCGTTCGAGACG

### Construction of phytase variants by doping

The first PCR reaction was carried out with 2 primer pairs, 620AM34 and Dope1R, and Dope1 with nucleotide mixtures described above and 680AM35 using the following PCR reaction system and conditions:

| PCR reaction system: | | Conditions: | | |
|---|---|---|---|---|
| | | | | |
| 38.9 µl | H₂O | 1 | 98° C | 10 sec |
| 5 µl | 10 X reaction buffer | 2 | 68° C | 90 sec |
| 1 µl | *Klen Taq LA* (CLONTECH) | 1-2 | 30 cycles | |
| 4 µl | 10 mM dNTPs | 3 | 68° C | 10min |
| 0.3µl X 2 | 100 pmol/µl Primers | | | |
| 0.5 µl | pTMPP2 ver2 | | | |

The resulting fragments were gel-purified and used for the template for the second PCR reaction. The second PCR was carried out with 620 and 680 as primers using the following PCR reactions and conditions:

| PCR reaction system: | | Conditions: | | |
|---|---|---|---|---|
| 38.4 µl | H₂O | 1 | 98° C | 10 sec |
| 5 µl | 10 X reaction buffer | 2 | 66° C | 90 sec |
| 1 µl | *Klen Taq LA* (CLONTECH) | 1-2 | 30 cycles | |
| 4 µl | 10 mM dNTPs | 3 | 66° C | 10min |
| 0.3µl X 2 | 100 pmol/µl Primers | | | |
| 0.5µl X 2 | PCR fragments | | | |

### Spiked oligo shuffling library

Spiked libraries were constructed as follows: PCR reaction for preparing a gene fragment of the variant was carried out with *KlenTaq* polymerase and AM34 and AM35 as primers as described above, and the fragment was gel-purified. About 10 ug DNA/250µl was incubated with 0.8µlDNasel (Gibco BRL 18068-015) and 30µl10X buffer at 25°C for 7-10min. EDTA was added to a final concentration of 10mM which stopped the reaction. DNA fragments of correct size (50-150bp) was purified by Whatman glass filter. Then the DNase treated fragments were reassembled using the following PCR reaction system and conditions:

| PCR reaction system: | Conditions: | | |
|---|---|---|---|
| 0.2, 0.5 and 1 pmol DNase-treated template | 1 | 94°C | 2 min. |
| 3, 6, 12x molar excess of each mutagenic | 2 | 94°C | 30 sec |
| oligo | 3 | 45°C | 30 sec |
| 1 beads Ready-to-go | 4 | 72°C | 1 min. |
| 0.1µl *Pwo* polymerase | 2-4 | 30 cycles | |
| final volume 25µl | 5 | 72°C | 5 min. |

Using the above PCR mixture as a template, a second PCR reaction was carried out using the following PCR reaction system and conditions:

| PCR reaction system: | Conditions: | | |
|---|---|---|---|
| 0.2, 0.5, 1 and 2µl 1^{st} PCR reaction | 1 | 94°C | 2 min. |
| 2 beads Ready-to-go | 2 | 94°C | 30 sec |
| 0.3µl 100pmol primer 1 (AM34) | 3 | 55°C | 30 sec |
| 0.3µl 100pmol primer 2 (AM35) | 4 | 72°C | 90 sec |
| 0.1µl Pwo polymerase | 2-4 | 25cycles | |
| | 5 | 72°C | 10 min. |

### Example 2: Purification and Characterization of Phytase Variants

### Purification of phytase variants

The culture broth from fermentations of *Asperillus oryzae* expressing a number of variants selected from Tables 1-5 above (designated with upper case letters A-M), was filtered using first a series of GF-filters from Millipore and then a 0.45 µm HA-filter (Millipore). Ammonium sulphate (AMS) was then added to the filtrate to a concentration of 1.35 M, and pH was adjusted to pH 6. The sample was filtered again (0.45 µm HA-filter as above) and loaded onto a 70 ml (volume dependent on loading) Phenyl Sepharose column (XK26, Pharmacia) equilibrated in 1.35 M AMS, 20 mM succinate, pH 6. The column was washed with 1.35 M AMS, 20 mM succinate, pH 6 prior to eluting the phytase in a linear gradient of 1.35-0 M AMS in 20 mM succinate, pH 6 over 10 column volumes. Flow was set at 6 ml/min and 10 ml fractions were collected. Fractions containing activity and of sufficient purity (as evaluated by SDS-PAGE) were pooled and dialyzed over night against 50 mM sodium acetate, pH 5.5. The phytase containing pool was then added 20 mM Tris-acetic acid, pH 7.5 and subjected to anion exchange chromatography either on an 8 ml MonoQ or a larger Q-Sepharose column (Pharmacia) equilibrated in 20 mM Tris-acetic acid, pH 7.5. The phytase was eluted in a linear gradient of 0-0.5 M NaCl in 20 mM Tris-acetic acid, pH 7.5 over 10 column volumes. Flow rates and fractions sizes were adjusted according to column scale. Fractions containing phytase activity and of sufficient purity were pooled and concentrated on Amicon YM10 membranes before dialyzing against 50 mM sodium acetate, pH 5.5.

### SDS-PAGE

SDS-PAGE was conducted using Novex 4-20 Tris-Glycine according to the manufacturer's instructions. The phytase variants as purified from *A. oryzae* had a molecular weight, MW, around 65 kDa corresponding to around 25% carbohydrate.

### Phytase activity assay, pH and temperature profiles, temperature stability testing

10 µl diluted enzyme samples (diluted in 0.1 M sodium acetate, 0.01 % Tween20, pH 5.5) were added into 250 µl 5 mM sodium phytate (Sigma) in 0.1 M sodium acetate, 0.01 % Tween20, pH 5.5 (pH adjusted after dissolving the sodium phytate; the substrate was preheated) and incubated for 30 minutes at 37°C. The reaction was stopped by adding 250 µl 10 % TCA and free phosphate was measured by adding 500 µl 7.3 g FeS0₄ in 100 ml molybdate reagent (2.5 g (NH₄)₆)M0₇0₂₄.4H₂0 in 8 ml H₂S0₄ diluted to 250 ml). The absorbance at 750 nm was measured on 200 µl samples in 96 well microtiter plates. Substrate and enzyme blanks were included. A phosphate standard curve was also included (0-2 mM phosphate). 1 FYT equals the amount of enzyme that releases 1 µmol phosphate/min at the given conditions.

The pH-profiles were obtained by running above assay at various pH values in 50 mM buffers pH 3 (glycine-HCl), pH 4-5.5 (sodium acetate), pH 6 (MES), pH 7-8 (Tris-HCl).

Temperature profiles were obtained by running the assay at the various temperatures (preheating the substrate).

Temperature stability was investigated by preincubating the phytases in 0.1 M sodium phosphate, pH 5.5 at various temperatures before measuring the residual activity.

The purified variants exhibit similar pH-profiles as the P. *lycii* parent phytase (all having a pH optimum around pH 4-5).

### Differential Scanning Calorimetry (DSC)

DSC was performed at various pH-values using the VP-DSC from Micro Cal. Scans were performed at a constant scan rate of 1.5 °C/min from 20-90°C. Before running the DSC, the phytases were desalted using NAP-5 columns (Pharmacia) equilibrated in the appropriate buffers (e.g. 0.1M glycine-HCl, pH 2.5 or 3.0; 0.1 M sodium acetate, pH 5.5; 0.1M Tris-HCl, pH 7.0). Data-handling was performed using the MicroCal Origin software.

The resulting melting temperature, Tm's, are shown in Table 7 below. The thermostability of the variants is improved significantly as compared to the *Peniophora* parent phytase. The thermostability is seen to be pH-dependent.

**Table 7**

| **Phytase Variant** | **Tm (°C) at pH 2.5** | **Tm (°C) at pH 3.0** | **Tm (°C) at pH 5.5** | **Tm (°C) at pH 7.0** |
|---|---|---|---|---|
| *Penio-phora* parent phytase | 36.4 | 50.5 | 59.6 | 48.8 |
| A | - | - | 62.7 | - |
| B | - | - | 62.4 | - |
| C | - | - | 65.0 | - |
| D | - | 52.2 | 70.5 | 69.6 |
| E | 43.5 | 58.5 | 72.6 | - |
| F | 42.2 | - | 73.5 | - |
| G | 44.5 | - | 73.1 | |
| H | 48.1 | - | 78.0 | - |
| I | 49.9 | - | 79.8 | - |
| J | - | 49.0 | 75.6 | - |
| K | 52.1 | - | 80.6 | - |
| L | 59.6 | - | 82.0 | - |
| M | 53.1 | - | 80.5 | - |

### Example 3: Specific Activity

The specific activity of variants selected from Tables 1-5 (here designated N, O and P) was determined on highly purified samples dialysed against 20 mM sodium acetate, pH 5.5. The purity was checked beforehand on an SDS poly acryl amide gel showing the presence of only one component.

The protein concentration was determined by amino acid analysis as follows: An aliquot of the sample was hydrolyzed in 6N HCl, 0.1% phenol for 16 h at 110 C in an evacuated glass tube. The resulting amino acids were quantified using an Applied Biosystems 420A amino acid analysis system operated according to the manufacturer's instructions. From the amounts of the amino acids the total mass - and thus also the concentration - of protein in the hydrolyzed aliquot can be calculated.

The phytase activity was determined in the units of FYT, and the specific activity was calculated as the phytase activity measured in FYT units per mg phytase variant enzyme protein.

The results are shown in Table 8 below. The specific activity figures are relative to the specific activity of the *Peniophora* parent phytase which was set to 100%.

**Table 8**

| **Phytase Variant** | **Specific Activity (%)** |
|---|---|
| *Peniophora* parent phytase | 100 |
| N | 147 |
| O | 117 |
| P | 114 |

### Example 4: Animal Feed and Animal Feed Additives comprising a Phytase Variant

### Animal Feed Additive

10 g of Phytase Variant no. 1938 (calculated as phytase enzyme protein) is added to the following premix (per kilo of premix):

| | | |
|---|---|---|
| 5000000 | IE | Vitamin A |
| 1000000 | IE | Vitamin D3 |
| 13333 | mg | Vitamin E |
| 1000 | mg | Vitamin K3 |
| 750 | mg | Vitamin B1 |
| 2500 | mg | Vitamin B2 |
| 1500 | mg | Vitamin B6 |
| 7666 | mcg | Vitamin B12 |
| 12333 | mg | Niacin |
| 33333 | mcg | Biotin |
| 300 | mg | Folic Acid |
| 3000 | mg | Ca-D-Panthothenate |
| 1666 | mg | Cu |
| 16666 | mg | Fe |
| 16666 | mg | Zn |
| 23333 | mg | Mn |
| 133 | mg | Co |
| 66 | mg | I |
| 66 | mg | Se |
| 5.8 | % | Calcium |
| 25 | % | Sodium |

### Animal Feed

This is an example of an animal feed comprising 0.03g/kg (30ppm) of Phytase Variant E (calculated as phytase enzyme protein):
74.0% wheat
20.7% roasted soy cake
5.0% soy oil
0.3 % of the above Premix
The ingredients are mixed, and the feed is pelletized at the desired temperature, e.g. 65, 75 80, or even 85°C.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

Various references are cited herein, the disclosures of which are incorporated by reference in their entireties.

## Claims

1. A variant of a parent phytase, comprising a substitution in at least one position of at least one region selected from the group of regions consisting of: 20; 26; 28-31; 37-48; 55-69; 73-83; 91-119; 123-126; 135-142; 152-163; 169-199; 204-222; 229-238; 248-266; 284-289; 300-308; 321-335; 343-350; 384-398; 407-412; and 419-430; wherein
(a) the variant has phytase activity; and
(b) each position corresponds to a position of SEQ ID NO: 2; and
(c) the variant has a percentage of identity to SEQ ID NO: 2 of at least 75%; and
(d) with the proviso that the variant is not a variant of the *Peniophora lycii* CBS 686.96 phytase selected from the group consisting of: G26S; Y28N; D29S; F31Y; E42D,K,A; T45R; V461; W59F; S62D; A64K; R65Q,E,G,A; S66T; R67A,I,Q,K; Q68Y,V,I; K74R,A; A99N; D100S; L102V; P103E; F104L; N107T,Q; S109M; H110S,V; Q111E,N; T112A,S; M116F,I,L; A135S; D137Y; Q138D,S,G; D142A; ()154fH; S155N; G156P; E157Q,S; E169S; E170A,P; G171A; ()171as; T174P,A; N177S; N178S,T; M179T,V,L; N182A,V; V184G; D185E; ()185eT; G186S,A; D187R,K,S; ()187aV,T; E1880,S,A,V; S189E; V195L; N199A,P; L204N; A207D,H; S216T; D217E; L219Y,T; T220Y,N; L221 F; M222L; L230V; ()235eE,Q; V248R,1;S249Q,A; E251D; Y254A,L,G; D255N; D257K; Y259F; T262H; P264A; G286R,H; Q287K,S; A288P; T3210,S,G; M3221; V3231; P324A; A327S,F,I,L; F332Y; N333P; E344R; R346P; W348F; V349L,A,S; D350S,V; G388A; V393R; E395K,T; and L396R.

2. The variant of claim 1, which comprises a substitution or a combination of substitutions selected from the following group of substitutions and combinations of substitutions:
(i) 20R+95R+97V+98G;
(ii) 29N+1021;
(iii) 29N+118A;
(iv) 29N+163P+324S;
(v) 29N+163P+324S+395G;
(vi) 29N+234S;
(vii) 29N+324S;
(viii) 29S;
(ix) 29S+45A+69A+99D+110Y+125D+324A+334E+344S+350V;
(x) 29S+63N +125D+183K+218T+324A+334E+350V+421S;
(xi) 29S+95H+110W+183W+324A+350V;
(xii) 29S+95M+110W+183W+324A+350V;
(xiii) 29S+95N+110R+183W+324A+350V;
(xiv) 29S+95R+110W+183W+324A+350V;
(xv) 29S+95S+110R+183W+324A+350V;
(xvi) 29S+95S+110W+183W+324A+350V;
(xvii) 29S+95T+110R+183W+324A+350V;
(xviii) 29S+95T+110Y+125D+183W+286S+324A+350V;
(xix) 29S+95T+110Y+152A+156C+157Q+160R+183W+324A+350V;
(xx) 29S+95T+110Y+156R+157Q+183W+324A+350V;
(xxi) 29S+95T+110Y+157K+183W+185H+187R+190S+191 R+248G+324A+350V;
(xxii) 29S+95T+110Y+183W+185A+248A+324A+350V;
(xxiii) 29S+95T+110Y+183W+185A+248C+324A+350V;
(xxiv) 29S+95T+110Y+183W+185A+248E+324A+350V;
(xxv) 29S+95T+110Y+183W+185A+248H+324A+350V;
(xxvi) 29S+95T+110Y+183W+185E+248S+324A+350V;
(xxvii) 29S+95T+110Y+183W+185H+248G+321A+323A+324A+350V;
(xxviii) 29S+95T+110Y+183W+185H+248G+324A+350V;
(xxix) 29S+95T+11 OY+183W+185H+187S+190K+191 S+21 OG+2221+248G+324A+350 V;
(xxx) 29S+95T+110Y+183W+185K+248G+324A+349K+350L;
(xxxi) 29S+95T+110Y+183W+185K+248S+324A+350V;
(xxxii) 29S+95T+110Y+183W+185K+248T+324A+349A+350L;
(xxxiii) 29S+95T+110Y+183W+185K+248T+324A+349L+350l;
(xxxiv) 29S+95T+110Y+183W+185K+248T+324A+349L+350M;
(xxxv) 29S+95T+110Y+183W+185K+248T+324A+350V;
(xxxvi) 29S+95T+110Y+183W+185P+248G+324A+350V;
(xxxvii)295+95T+l10Y+183W+185P+2485+324A+350V;
(xxxviii) 29S+95T+11 OY+183W+191 L+324A+350V;
(xxxix) 29S+95T+110Y+183W+248D+324A+350V;
(xl) 29S+95T+110Y+183W+324A;
(xli) 29S+95T+110Y+183W+324A+350V;
(xlii) 29S+99D+125D+324A+334E+350V;
(xliii) 29S+110Y+183W+185N+324A+350V;
(xliv) 29S+125D+218T+324A+344S+350V;
(xlv) 29S+125D+234K+324A+330R;
(xlvi) 29S+125D+234K+324A+330R+395G;
(xlvii) 29S+125D+324A;
(xlviii) 29S+125D+324A+330R+395G;
(xlix) 29S+125D+324A+350V;
(1) 29S+183A+324A+350V;
(li) 29S+183G+324A+334E+344S+350V;
(lii) 29S+183W+324A+350V;
(liii) 29S+205S;
(liv) 29S+324A+350V;
(lv) 29T+324L;
(Ivi) 45A+350V+421 R+422R+423N+424S+425L+426E+427G+428R+4291+430M;
(Ivii) 69A+334E;
(Iviii) 99D+398T;
(lix) 114A+187G+194R+324S;
(lx) 218T+334G;
(Ixi) 323A+324S;
(Ixii) 324S;
(Ixiii) 324S+328T;
(Ixiv) 324S+345D+347Q;
(Ixv) 324S+395G;
(Ixvi) 330R;
(Ixvii) 334D+343N+344V;
(Ixviii) 334E+344A;
(Ixix) 345H+347P+350V; and
(Ixx) 350V.

3. An isolated nucleic acid sequence comprising a nucleic acid sequence which encodes the variant of any of claims 1-2.

4. A nucleic acid construct comprising the nucleic acid sequence of claim 3 operably linked to one or more control sequences that direct the production of the polypeptide in a suitable expression host.

5. A recombinant expression vector comprising the nucleic acid construct of claim 4.

6. A recombinant host cell comprising the nucleic acid construct of claim 4 and/or the expression vector of claim 5.

7. A method for producing the variant of any one of claims 1-2, comprising
(a) cultivating the host cell of claim 6 to produce a supernatant comprising the variant; and
(b) recovering the variant.

8. A transgenic plant, or plant part, capable of expressing a phytase variant of any one of claims 1-2.

9. A transgenic, non-human animal, or products, or elements thereof, being capable of expressing a phytase variant of any one of claims 1-2.

10. A composition comprising at least one phytase variant of any one of claims 1-2, and
(a) at least one fat soluble vitamin;
(b) at least one water soluble vitamin; and/or
(c) at least one trace mineral.

11. The composition of claim 10 further comprising at least one enzyme selected from the following group of enzymes: xylanases, endoglucanases, endo-1,3(4)-beta-glucanases, and proteases.

12. The composition of any one of claims 10-11 which is an animal feed additive.

13. An animal feed composition having a crude protein content of 50 to 800 g/kg and comprising the variant of any one of claims 1-2 or the composition of any one of claims 10-12.

14. A method for improving the nutritional value of an animal feed, wherein the variant of any one of claims 1-2 or the composition of any one of claims 10-12 is added to the feed.

15. A process for reducing phytate levels in animal manure comprising feeding an animal with an effective amount of the feed of claim 13.

16. A method for the treatment of vegetable proteins, comprising the step of adding the variant of any one of claims 1-2 or the composition of any one of claims 10-12 to at least one vegetable protein or protein source.

17. Use of the variant of any one of claims 1-2 or the composition of any one of claims 10-12 in animal feed; in the preparation of animal feed; for improving the nutritional value of animal feed; for reducing phytate levels in animal manure; for the treatment of vegetable proteins; or for liberating phosphorous from a phytase substrate.
